(19) **Europäisches Patentamt**
**European Patent Office**
**Office européen des brevets**

(11) **EP 4 556 486 A1**

(12) **EUROPEAN PATENT APPLICATION**
published in accordance with Art. 153(4) EPC

(43) Date of publication:
**21.05.2025 Bulletin 2025/21**

(21) Application number: **23838948.0**

(22) Date of filing: **11.07.2023**

(51) International Patent Classification (IPC):
**C07K 14/46** *(2006.01)*     **C12N 15/13** *(2006.01)*
**C12N 15/85** *(2006.01)*     **A61K 39/395** *(2006.01)*
**C07K 14/435** *(2006.01)*    **A61P 35/00** *(2006.01)*
**A61P 35/02** *(2006.01)*

(52) Cooperative Patent Classification (CPC):
**A61K 39/395; A61K 45/06; A61P 35/00;**
**A61P 35/02; C07K 14/435; C07K 14/46;**
**C07K 16/00; C07K 16/46; C12N 15/85**

(86) International application number:
**PCT/CN2023/106817**

(87) International publication number:
**WO 2024/012457 (18.01.2024 Gazette 2024/03)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **11.07.2022 CN 202210822959**

(71) Applicant: **Cytocares (Shanghai) Inc.**
**Shanghai 201203 (CN)**

(72) Inventors:
• **CHEN, Li**
**Shanghai 201203 (CN)**
• **QIAN, Wenjing**
**Shanghai 201203 (CN)**
• **ZHU, Huaxing**
**Shanghai 201203 (CN)**

(74) Representative: **Grünecker Patent- und**
**Rechtsanwälte**
**PartG mbB**
**Leopoldstraße 4**
**80802 München (DE)**

Remarks:
The complete document including Reference
Table(s) and the Sequence Listing(s) can be
downloaded from the EPO website

(54) **TRISPECIFIC ANTIBODY AND USE THEREOF**

(57)    The present invention relates to a trispecific antibody specifically binding to CD19, CD3, and CD28, which trispecific antibody contains a fragment derived from an IgD hinge region as a linker. The present invention also relates to the use of the trispecific antibody for treating tumors such as malignant hematological tumors.

EP 4 556 486 A1

**Description**

**Cross Reference to Related Applications**

The present application claims the priority of Chinese patent application no. 202210822959.8, filed on July 11, 2022, entitled "Trispecific Antibody and Use Thereof", the content of which is incorporated by reference in its entirety.

**Technical Field**

The present invention belongs to the field of antibody drugs. The present invention provides a trispecific antibody specifically binding to CD19, CD3 and CD28, comprising a fragment derived from an IgD hinge region as a linker sequence. The antibody is particularly suitable for use as a theraputic drug for tumor, in particular for the treatment of relapsed or refractory B cell malignancies.

**Background Art**

Antibody-based immunotherapeutic drugs are popular drugs in the field of tumor treatment. Tumor immunotherapy specifically removes minimal residual tumor lesions and inhibits tumor growth by activating immune cells in the body and enhancing the body's anti-tumor immune response. Because of its small side effects and obvious therapeutic effect, it is gradually becoming the future development direction of tumor treatment. Immune checkpoint blockade (ICB) can induce a durable response against a variety of solid tumor types, and is considered to be the most successful immunotherapy at present; however, there are still a large majority of cancer patients who do not respond to the treatment. In these patients, tumor immunogenicity and inherent MHC antigen presenting function are defective. Therefore, there is a need to develop other methods that is capable of promoting immune cell-mediated killing of tumor cells.

A bispecific T cell engager (BiTE) molecule consists of two single chain variable domain fragments (scFv), in which one of the scFvs targets the CD3ε chain of a T cell receptor (TCR) on T cells and is linked to another scFv that targets a tumor-associated antigen (TAA), thus the BiTE molecule is able to induce polyclonal T cell-directed lysis of cancer cells in an MHC-independent manner. The effective dose of bispecific T cell engager-based drugs is more than 10,000-fold lower than that of typical antibodies, but the bispecific T cell engager-based drugs also have some limitations, e.g., dose-limiting toxicity, poor pharmacokinetic properties, issues regarding durability of efficacy, etc.

The synergistic effect of two activation signals is required for transition from quiescence to full activation of naïve T lymphocytes. In this process, after the antigen/MHC complex is recognized by the TCR, the first signal is transduced through CD3, which is not enough to fully activate the T cell, and the signal generated by a co-stimulatory molecule is required as a supplement. Lack of the co-stimulatory signal tends to lead to T cell anergy or activation-induced T cell apoptosis (Schietinger A and Greenberg PD. Tolerance and exhaustion: defining mechanisms of T cell dysfunction. Trends in Immunology. 2014; 35: 51-60).

Among the currently found T cell co-activating molecules, CD28 and B7 families are among the most important classes of co-stimulatory molecules, CD28 can bind to ligands CD80 (B7-1) and CD86 (B7-2) and the binding amplifies the first signal transduced by TCR/CD3, thereby maintaining T cell survival, and promoting cytokine-induced T cell proliferation and differentiation. CD28, a dimeric transmembrane glycoprotein, is expressed on the surface of approximately 80% of CD4+ T cells and 50% of CD8+ T cells of humans, and thus considered a good target for T cell activation (Jeong S and Park SH. Co-stimulatory receptors in cancers and their implications for cancer immunotherapy. Immune Netw. 2020; 20(1): e3; Esensten JH, Helou YA, Chopra G, et al. CD28 costimulation: from mechanism to therapy. Immunity. 2017; 44: 973-988).

The CD28 co-stimulatory signal can prolong the survival of T cells through both exogenous and endogenous mechanisms. The exogenous mechanism is mainly promoting the release of cytokines, especially the cytokine IL-2 which plays a key role during the growth of T cells. The endogenous mechanism is manifested in the ability to enhance the expression of anti-apoptotic protein Bcl-XL (Boise LH, Minn AJ, Noel PJ, June CH, Accavitti MA, Lindsten T, Thompson CB. CD28 costimulation can promote T cell survival by enhancing the expression of Bcl-xL. Immunity. 2010; 3:87-98). Bcl-XL expression is weak in the presence of only CD3 signal or only CD28 signal, while Bcl-XL expression is significantly enhanced in the presence of the dual signals (Watts TH. Staying alive: T cell costimulation, CD28, and Bcl-xL. J Immunol. 2010; 185:3785-3787). In addition, CD28 co-stimulatory signal can promote the proliferation of effector T cells and memory T cells, in which the memory T cells can be rapidly activated when the body encounters the same tumor cells again, thus effectively eliminating tumor cells.

The importance of co-activation signals for T cell activation has also been clearly manifested in the developing course of chimeric antigen receptor T cell (CAR-T) -based drugs (Pettitt D, Arshad Z, Smith J, et al. CAR-T cells: a systematic review and mixed methods analysis of the clinical trial landscape. Molecular Therapy. 2018; 26: 342-353.; Ma S, Li XC, Wang XY, et al. Current Progress in CAR-T cell therapy for solid tumors. Int J Biol Sci. 2019; 15: 2548-2560.). The structure of the first generation of CAR molecules only consists of an extracellular scFv targeting a tumor-associated antigen and an intracellular CD3ζ chain for T cell activation, and such CAR molecules have not been successfully verified clinically due to the limited expansion capacity of T cells loaded with such CAR molecules and poor persistence of pharmacological effects. The second and third generation of CAR molecules further introduce co-stimulatory signal (such as CD28, 4-1BB, OX40, etc.) domains, which significantly enhances the activation effect and persistence of pharmacological effects of CAR-T cells loaded with such CAR molecules, and the current cell therapy designed based on the second generation of CAR has been successfully applied to the treatment of some blood tumors (Savoldo B, Ramos CA, Liu E, et al. CD28 costimulation improves expansion and persistence of chimeric antigen receptor-modified T cells in lymphoma patients. Immunology. 2011; 121: 1822-1826.; Guedan S, Posey Jr., AD, Shaw C, et al. Enhancing CAR T cell persistence through ICOS and 4-1BB costimulation. Immunology. 2018; 3(1): e96976.).

In present invention, the principle of dual-signal activation of T cells is used in the design of multifunctional antibodies on the basis of BiTE, thereby developing dual-targeting scFv domains targeting CD3 and CD28 of T cells respectively, and introducing a scFv domain targeting a tumor cell-associated antigen (TAA) to form a trispecific antibody, so as to simultaneously achieve targeting of tumor cells and dual-signal activation of T cells.

The inventors have previously disclosed in prior application CN106589129A a trispecific antibody simultaneously binding to CD19, CD3 and CD28. In the trispecific antibody of this application, an IgD hinge region sequence of up to 81 amino acids was used as a linker between the second and third domains, and the trispecific antibody can form a dimer by utilizing the cysteine contained in that linker sequence. This application verified the expression of monomers and dimers of trispecific antibodies, antigen-binding activity, cell engaging experiments (the ability of antibodies to engage CD19 positive target cells with CD3/CD28 positive effector cells thus clustering them together), and cell-killing ability (by co-incubating antibodies with lymphoma cells and CIK cells to observe the death of target cells) in in vitro experiments. However, this application did not verify the efficacy of this antibody as a therapeutic drug for tumors by any in vivo experiment.

In fact, such a trispecific antibody molecule can achieve a better effect in principle; however, due to the complexity of the molecular structure, as for how to ensure that each of its binding domains can exert the expected effect, there is a need in the art for a lot of creative work to improve the structure and function of the trispecific antibody molecule, thereby providing a safe, effective and novel anti-tumor drug.

## Summary of the Invention

The inventors further develop a new T cell engager-based antibody on the basis of previous works, which can simultaneously bind to CD19, CD3 and CD28, and design a completely new linker sequence for linking the second and third domains, thereby completing the present invention.

Therefore, in a first aspect, the present invention relates to a trispecific antibody, comprising:

    (1) a first binding domain specifically binding to CD19;
    (2) a first linker sequence;
    (3) a second binding domain specifically binding to CD3;
    (4) a second linker sequence; and
    (5) a third binding domain specifically binding to CD28.

In a preferred embodiment, the first linker sequence is a flexible linker. In a specific embodiment, the first linker sequence is $(G_4S)_n$, $(G_4S)_nG_4$, $(SG_4)_n$, $G_4(SG_4)_n$, $(G_2S)_n$, $(G_2S)_nG_2$, $(SG_2)_n$, or $G_2(SG_2)_n$ linker sequence.

In a preferred embodiment, the amino acid sequence of the second linker sequence comprises no cysteine, so that the trispecific antibody does not form a dimer. In a preferred embodiment, the amino acid sequence of the second linker sequence comprises no glycosylation site. In a specific embodiment, the second linker sequence is derived from an amino acid sequence of an IgD hinge region or is an amino acid sequence comprising $(EAAAK)_n$.

In a second aspect, the present invention provides an isolated nucleic acid molecule encoding the nucleotide sequence of the trispecific antibody of the first aspect.

In a third aspect, the present invention provides an expression vector comprising the isolated nucleic acid molecule of the second aspect.

In a fourth aspect, the present invention provides a host cell comprising the expression vector of the third aspect.

In a fifth aspect, the present invention provides a method for preparing the multispecific antibody of the first aspect.

In a sixth aspect, the present invention provides the use of a trispecific antibody of the first aspect in the preparation of a medicament for the treatment of a B cell-associated disease, in particular a B cell-associated tumor, including B cell leukemia and B cell lymphoma. In particular, the B cell-associated tumor is a refractory or relapsed B cell-associated tumor. The B cell lymphoma may be non-Hodgkin lymphoma (NHL) or Hodgkin lymphoma (HL). Examples of the B cell-associated disease include, but are not limited to, classical Hodgkin lymphoma, nodular lymphocyte predominant Hodgkin lymphoma, diffuse large B-cell lymphoma, follicular lymphoma (FL), mucosa associated lymphoid tissue lymphoma (MALT), small lymphocytic lymphoma (SLL), chronic lymphocytic leukemia (CLL), mantle cell lymphoma (MCL), Burkitt lymphoma, acute lymphoblastic leukemia (ALL), chronic lymphatic leukaemia (CLL), B cell prolymphocytic leukemia (B-PLL), precursor B-cell lymphoblastic leukemia, hairy cell leukemia (HCL), splenic B cell lymphoma/leukemia, and splenic marginal zone lymphoma (SMZL).

In a seventh aspect, the present invention relates to a method for treating a tumor, wherein the method comprises the use of the trispecific antibody of the first aspect.

In an eighth aspect, the present invention relates to a pharmaceutical composition comprising the trispecific antibody of the present invention and a pharmaceutically acceptable carrier.

In a ninth aspect, the present invention relates to the use of the trispecific antibody of the present invention in combination with an additional treatment or drug.

In a tenth aspect, the present invention relates to the use of an amino acid fragment derived from the IgD hinge region as a linker sequence in a fusion protein.

The trispecific antibody of the present invention has at least the following advantages.

(1) Long-acting T cell activation provided by the dual-signal mechanism

The trispecific antibody of the present invention can activate two T cell stimulation signals of CD3 and CD28 at the same time, and such a "dual signal" activation mechanism can better regulate the TCR/CD3 signal, so that the body produces a long-term effective immune response, that is, playing a role in the body by inducing activated T cells rather than the drug itself, and thus even in the case of a drug with a short half-life (relative to an antibody containing an Fc region), the dosing frequency can be reduced without the need of frequent continuous infusions to maintain the long-term activation of the T cells by the drug.

In comparison with CD19/CD3 bispecific antibody Blincyto® (Blinatumomab) also belonging to the T cell engager type, the trispecific antibody of the present invention may be more convenient in the dosing mode. Blinatumomab tends to lead to T cell anergy or activation-induced T cell apoptosis due to lack of co-stimulatory signals, and thus there is a need for frequent continuous infusions to maintain the durability of T cell activation by the drug.

In addition, compared with CAR-T cell therapy (such as CD19 CAR-T), the trispecific antibody of the present invention can be industrially produced by the production process and route similar to those for monoclonal antibodies and can be widely applied to different individuals. In contrast, the approved CAR-T products and the vast majority of CAR-T clinical trials use autologous CAR-T, that is, using the patient's own T cells as the starting materials, the production cost of this "privately customized" therapy is relatively high, and the exorbitant price limits the market potential of this therapy. In addition, the quality stability of CAR-T therapy is relatively poor, the patient's own T cells usually have quality and quantity defects, and the quality difference between different batches of products is large; therefore, it is difficult to achieve large-scale industrial production and standardized quality control. Individualized preparation and dosing are needed for different patients.

(2) More approximating to immune synapse formation in natural state

In a physiological state, the TCR on the T cell recognizes the antigenic peptide presented by the MHC of the tumor cell, thereby forming an immune synapse (IS), which is a key step in the T cell activation process. After the formation of the immune synapse in the physiological state, the distance between the T cell and the tumor cell is generally about 15 nm. Although some bispecific antibodies can bind T cells and tumor cells at the same time, they cannot draw the tumor cells and T cells close to a distance of about 15 nm due to the limitations of their own structures and the recognized antigen-binding epitopes. The trispecific antibody of the present application has been structurally optimized and designed, which can enable the immune synapse formed between T cells and tumor cells to be more approximate to the distance in natural state, making the trispecific antibody possess extremely high efficacy in the process of mediating the killing of tumors by polyclonal T cells (Dickopf S, Georges GJ, Brinkmann U. Format and geometries matter: Structure-based design defines the functionality of bispecific antibodies. Computational and Structural Biotechnology Journal 18 (2020) 1221-1227.).

(3) Convenience based on scFv structure

The trispecific antibody formed by scFvs also has the advantages of small molecular weight, strong tumor penetration ability, etc. Although the half-life of an antibody with the scFv structure is shorter than that of an antibody with the Fc structure, the "dual-signal" activation mechanism makes the T cells activated by the trispecific antibody persistent and less prone to apoptosis after activation. In addition, the trispecific antibody using the "dual-signal" activation mechanism can fully activate T cells, enhance the survival of T cells, and achieve long-acting killing of target cells by inducing the differentiation of a memory T cell population at a very low drug concentration.

(4) Induction of memory T cells to achieve a long-acting immune response

The existing pre-clinical research data show that the trispecific antibody of the present invention can significantly enhance immune memory, and induce the differentiation of a memory T cell population with self-renewal and replication ability, long survival time in vivo and ability of durable killing of tumor cells, which is mainly manifested by the increase in the number and proportions of effector memory T cells ($T_{EM}$) and central memory T cells ($T_{CM}$); therefore, the trispecific antibody can maintain the long-acting immune response in the body and prevent the recurrence of the tumor.

(5) Advantages brought by a novel linker sequence

In the present invention, a novel linker sequence is used to link the second and third binding domains. This linker sequence is a fragment derived from the IgD hinge region, which is obtained by modifications such as removing glycosylation sites in the IgD hinge region by modification, removing cysteines that may lead to dimer formation, and/or reducing the overall length of the hinge region. By these modifications, when the linker sequence obtained is used in the trispecific antibody, a more homogeneous antibody product can be obtained, with formation of fewer structural isomers, or reduced production of polymers, and reduced possibility of activation at a non-target site.

**Brief Description of the Drawings**

FIG. 1 shows a schematic representation of the structure of a trispecific antibody of the present invention.
FIGS. 2A-B show the results of analysis of SDS-PAGE (silver staining method) (A) and SEC-HPLC (B) of the purified LMOAME in Example 2.
FIG. 3 shows a mass spectrogram of the purified LMOAME in Example 2.
FIGS. 4A-B show the results of SEC-HPLC analysis of LMOAME (A) and a trispecific antibody (BLMOAM) as a control (B) after captured by affinity chromatography.
FIG. 5 shows the results of SDS-PAGE analysis (Coomassie brilliant blue staining) of LMOAME and a trispecific antibody (BLMOAM) as a control after captured by affinity chromatography.
FIG. 6 shows a SEC-HPLC chromatogram of the purified MLMOF in Example 2.
FIG. 7 shows curves of binding of LMOAME to hCD19 and hCD28 antigens as determined in Example 3.
FIGS. 8A-B show the results of LMOAME and Blinatumomab in T cell activation activity as determined in Example 5. (A) CD4$^+$ cell population; (B) CD8$^+$ cell population.
FIG. 9 shows the results of LMOAME and Blinatumomab in mediating effector cell proliferation as determined in Example 6.
FIG. 10 shows the results of two batches of LMOAME and Blinatumomab in mediating effector cell differentiation as determined in Example 7.
FIG. 11 shows the results of two batches of LMOAME and Blinatumomab in mediating killing activity on target cells as determined in Example 8.
FIGS. 12A-B show curves of the body weight of mice over time (A) and the tumor volume (mean $\pm$ SEM, mm$^3$) of mice over time (B) after dosing, as determined in the animal experiment in Example 10. The statistical analysis of the tumor volume of each group vs G1 was performed in Dunnett's multi-comparison, wherein *: P < 0.05, and **: P < 0.01 represent a significant difference.
FIGS. 13A-B show photographs of mice and tumors at the end of dosing (day 21) as obtained in the animal experiment in Example 10. (A): photographs of mice at the end of dosing; and (B): photographs of tumors stripped at the end of dosing.
FIG. 14 shows the results of the average tumor weight of each group of mice at the end of dosing (day 28), as determined in the animal experiment in Example 11. The tumor weight was presented as the mean $\pm$ SEM. The tumor weight among different groups was statistically analyzed by independent sample T test, wherein when compared with G1, *: P < 0.05, **: P < 0.01, and ***: P < 0.001 represent a significant difference; and when G2 is compared with G6 and G3 is compared with G7, ^: P < 0.05 represents a significant difference.
FIGS. 15A-B show curves of the body weight of mice (mean $\pm$ SEM, g) over time (A) and the number of photons in the tumor (mean $\pm$ SEM, p/sec/cm$^2$/sr) of mice over time (B) after dosing, as determined in the animal experiment in Example 12. The statistical analysis of the number of photons in the tumor in each dosing group vs G1 group was performed in Dunnett's multi-comparison, wherein *: P < 0.05, **: 0.001 $\leq$ P < 0.01, and ***: P < 0.001 represent a significant difference.
FIG. 16 shows curves of survival of mice of each group after dosing, as determined in the animal experiment in Example 12.

**Detailed Description of Embodiments**

Definitions

The "antibody" in the context of the present invention is interpreted in a broader sense, i.e. encompass not only traditional IgG antibodies, but also antibody analogs and derivatives, such as scFvs. For example, IgG antibodies are immunoglobulins with a Y-shaped structure produced by the immune system in response to foreign substances such as pathogens. An antibody with the classical structure is a homodimer, each monomer comprising a heavy chain and a light chain linked by a disulfide bond. The light chain consists of a variable region ($V_L$) and a constant region ($C_L$), while the heavy chain consists of a variable region ($V_H$) and three constant regions ($C_H1$, $C_H2$, and $C_H3$). Each variable region contains three "complementarity determining regions (CDRs)" that make up the antigen binding site responsible for complementarity with the antigen. The antigen-binding fragment consisting of $V_L$, $C_L$, $V_H$ and $C_H1$ is referred to as "Fab". The remaining portion, i.e. the "trunk" portion at the lower part of the Y-shaped structure, is referred to as the "Fc" region, and consists of the constant region $C_H2$ and $C_H3$ domains of the antibody heavy chain. The "hinge region" is the portion of immunoglobulin molecules, such as IgG, IgA, and IgG, located between $C_H1$ and $C_H2$ that links the Fab and Fc regions.

The "scFv" refers to a single chain fragment variable, sometimes also simply called a single chain antibody, which is a fusion protein consisting of a heavy chain variable region (VH) and a light chain variable region (VL) of an antibody. In scFv, the order of linking between VH and VL may be switched, i.e. VH may be placed at the N-terminus or VL may be placed at the N-terminus. The VH and VL may be linked by a "linker" sequence. The single chain fragment variable may also include a single variable domain on a heavy chain (VHH), which is also simply called a nanobody. Since there is no light chain, the nanobody has only 3 antigen recognition regions (CDR regions) belonging to the heavy chain. The molecular weight is about one-tenth of that of a traditional antibody and about one-half of that of an antigen binding fragment (scFV, VH-VL).

"IgD" is one of the isotypes of immunoglobulins, which, like IgG, has a Y-shaped structure. In the present invention, a fragment of the IgD hinge region is used as a linker sequence.

The "multispecific antibody" refers to an antibody or antibody analog capable of simultaneously and specifically binding to multiple targets.

The "trispecific antibody" refers to an antibody or antibody analog capable of simultaneously and specifically binding to three targets. In the context of the present invention, a trispecific antibody particularly refers to a single chain fusion protein formed by tandem linking of three domains having binding specificities for different antigen targets, such as by tandem linking of three different scFvs.

The "specifically binding to" or "specifically bind to" means that the antibody exhibits preferential binding to a particular target as compared to other proteins, but such specificity need not be absolute binding specificity. An antibody is considered to be "specific" for its intended target if binding of the antibody can determine the presence of a target protein in a sample, for example without producing undesirable results, for example false positives. The antibody or antigen-binding fragment thereof of the present invention will bind to a target protein with an affinity that is at least 2-fold higher, preferably at least 10-fold higher, more preferably at least 20-fold higher, and most preferably at least 100-fold higher than the affinity for a non-target protein. As an alternative or in addition, the antibody or antigen-binding fragment thereof of the present invention will have a binding affinity for its target protein, which is represented by a $K_D$ value of less than $1 \times 10^{-7}$ M, $1 \times 10^{-8}$ M, less than $1 \times 10^{-9}$ M (1 nM), less than $1 \times 10^{-10}$ M, less than $1 \times 10^{-11}$ M, or even less than $1 \times 10^{-12}$ M (1 pM). The antibody of the present application is said to specifically bind to a polypeptide comprising a given amino acid sequence (e.g., human CD19, human CD3, human CD28) if it binds to the polypeptide comprising the given amino acid sequence but does not bind to a protein lacking the sequence.

The "Binding domain" or "antigen binding domain" in the context of multispecific antibodies herein refers to a polypeptide structure, sometimes also simply called "domain", that is capable of specifically recognizing and binding to a target.

The "linker sequence" in the context of the present invention refers to a peptide segment for linking two binding domains, and also refers to a peptide segment for linking VH and VL in one binding domain. The "first linker sequence" specifically refers to a linker for linking the first binding domain and the second binding domain, and the "second linker sequence" specifically refers to a linker for linking the second binding domain and the third binding domain. Both of the first linker sequence and the second linker sequence are linkers for linking different binding domains, and thus are also collectively referred to as "inter-domain linkers". If a linker is also present between a VH and a VL comprised in a domain, it is referred to as an "intra-domain linker".

The "T-cell engager-based antibody" refers to an antibody that is capable of "engaging" two types of cells together in an expected manner by specifically binding to the two types of cells to draw them close.

The "CD19 × CD3 × CD28 antibody" refers herein to a trispecific antibody comprising a binding domain that specifically binds to CD19, a binding domain that specifically binds to CD3, and a binding domain that specifically binds to CD28.

"G$_4$S" refers to a Gly-Gly-Gly-Gly-Ser, i.e., a pentapeptide unit consisting of four glycines and one serine, and one or more such units are linker sequences commonly used in recombinant fusion proteins. Similarly, "G$_2$S" refers to Gly-Gly-Ser.

The "rigid" and "flexible" are used herein to describe the rigidity or flexibility of a molecular conformation. Specifically, when used to describe a peptide linker sequence, a "flexible linker" refers to a peptide linker that allows two segments of polypeptides or functional domains linked to change relative positions or interact with each other to a certain extent, while a "rigid linker" usually does not have a similar effect and provides a more fixed conformation.

The "percent homology" or "% homology" is used in the context of the present invention to describe the degree of similarity between two nucleotide sequences or two amino acid sequences, with the same meaning as "percent identity". The percent homology of two sequences can be calculated, after the alignment of the two sequences, by dividing the number of positions at which residues are identical by the total length of the sequences after alignment, multiplied by 100%. Methods and tools for aligning two amino acid sequences or nucleotide sequences are well known in the art, for example the BLAST suite available at the NCBI website (Altschul, S. F. et al. (1990) J. Mol. Biol. 215:403-410).

The "conservative substitution" of amino acids is well known in the art and generally refers to the change of one amino acid residue to another amino acid residue having a structurally or functionally similar side chain. For example, a list of exemplary conservative substitutions is provided in the table below.

| Original amino acid residue | Conservative substitution | Original amino acid residue | Conservative substitution |
| --- | --- | --- | --- |
| Ala | Gly; Ser | Leu | Ile; val |
| Arg | Lys; His | Lys | Arg; His |
| Asn | Gln; His | Met | Leu; Ile; Tyr |
| Asp | Glu; Asn | Phe | Tyr; Met; Leu |
| Cys | Ser; Ala | Pro | Ala |
| Gln | Asn | Ser | Thr |
| Glu | Asp; Gln | Thr | Ser |
| Gly | Ala | Trp | Tyr; Phe |
| His | Asn; Gln | Tyr | Trp; Phe |
| Ile | Leu; Val | Val | Ile; Leu |

The "cross-reactivity" refers to the ability of an antibody specifically binding to one antigen to bind to another antigen. If the two antigens are from the same species, such cross-reactivity may be referred to as "intra-species cross-reactivity" or "inter-target cross-reactivity". If the two antigens are from different species, this cross-reactivity is referred to as "inter-species cross-reactivity". Unless otherwise specified, the "cross-reactivity" of an antibody in the context of the present invention refers to inter-species cross-reactivity, i.e. the ability to react with homologous or orthologous proteins derived from other species.

The "nucleotide" in the context of the present invention includes DNAs and RNAs.

In the term "isolated nucleic acid molecule", "isolated" means that the operation of removing factors other than the desired component has been performed, and the isolated nucleic acid molecule is no longer present in its naturally occurring state, such as no longer present in the chromosomal genome.

The "expression construct" or "expression cassette" refers to a segment of nucleotide sequence comprising a gene of interest and gene expression regulatory sequences. The expression regulatory sequences include, but are not limited to, promoters, enhancers, terminators, polyadenylation sequences, and the like.

The "vector" refers to a carrier DNA molecule capable of carrying an exogenous gene into a cell. Examples of vectors include, but are not limited to, plasmids, viral vectors, cosmids, artificial chromosomes. Vectors used to introduce an exogenous transgene into a cell and facilitate expression of the transgene are referred to as "expression vectors".

The "host cell" herein refers to a cell that comprises an exogenous recombinant DNA, such as an expression vector, and also refers to a cell that comprises a polypeptide or protein obtained by expression of the recombinant DNA. Such host cells can be used to carry out the production of the trispecific antibody of the present invention. In one embodiment, the host cell is not particularly limited as long as it can be used for expression of a polypeptide sequence, for example, the trispecific antibody of the present invention.

The "tumor", understood in its broadest sense, refers to an abnormally overgrowed tissues, synonymous with "abnormal neoplasms". The "carcinoma" or "cancer" refers to malignancies, including sarcomas, cancerous tumour, lymphomas, leukemias, and gliomas.

The "B cell-associated tumor" refers to a tumor characterized by cancerous B cells, and may also be referred to as "B cell leukemia" or "B cell lymphoma".

The "subject" in the present invention refers to an animal receiving administration, preferably a vertebrate, more preferably a mammal, such as a rodent, e.g., a mouse and a rat; a primate, e.g., a monkey; most preferably a human.

The "treatment" refers to the amelioration, alleviation, or elimination (i.e., cure) of a disease or symptoms thereof. In some cases, the treatment comprises prophylactic treatment.

Antigen binding domain

The present invention provides a T-cell engager-based antibody that can simultaneously bind to CD19, CD3 and CD28, also referred to herein as a trispecific antibody or CD19 × CD3 × CD28 antibody.

Specifically, the trispecific antibody of the present invention comprises three binding domains, i.e. a first binding domain capable of specifically binding to CD19, a second binding domain capable of specifically binding to and activating a T cell surface CD3 molecule, and a third binding domain capable of specifically binding to and activating a T cell surface CD28 molecule, respectively. In addition, the trispecific antibody further comprises a first linker located between the first and second binding domains, and a second linker located between the second and third binding domains.

The human CD19 antigen is a transmembrane glycoprotein of a size of 95 kDa, and is a member of the immunoglobulin superfamily. In addition to being expressed on the surface of normal B lymphocytes, CD19 is also highly expressed in B cell malignancies, and anti-CD19 monoclonal full-length antibodies have been approved for the treatment of B cell lymphoma. Unless otherwise specified, the CD19 to which the first binding domain in the context of the present invention specifically binds is human CD19.

CD3 is a transmembrane protein, and its transmembrane region is linked with the transmembrane region of the two peptide chains of the TCR through a salt bridge to form a TCR-CD3 complex, the two components of which jointly participates in the recognition of an antigen by T cells and transduces the generated activation signal into T cells. Unless otherwise specified, the CD3 to which the first binding domain in the context of the present invention specifically binds is human CD3.

CD28, a dimeric transmembrane glycoprotein, is expressed on the surface of approximately 80% of CD4$^+$ T cells and 50% of CD8$^+$ T cells of humans. CD28 is a T cell co-stimulatory molecule, which can bind to ligands CD80 (B7-1) and CD86 (B7-2) and the binding amplifies the first signal transduced by TCR/CD3, thereby maintaining T cell survival, and promoting cytokine-induced T cell proliferation and differentiation. Unless otherwise specified, the CD28 to which the third binding domain in the context of the present invention specifically binds is human CD28. Therefore, for B cell-associated tumors, the first binding domain in the trispecific antibody of the present invention can specifically bind to the tumor cell surface associated antigen CD19, and the second and third binding domains can bind to the T cell surface receptors CD3 and CD28 respectively, thereby "engaging" tumor B cells with immune T cells together, meanwhile activating the CD3 signal pathway and CD28 co-stimulatory signal pathway of T cells, and thus the immune cells activated can exert a killing effect on the target cells.

In a preferred embodiment, the trispecific antibody of the present invention is a single chain fusion protein comprising: a scFv specifically binding to CD19 as the first binding domain; a scFv specifically binding to CD3 as the second binding domain; and a scFv that specifically binding to CD28 as the third binding domain.

The scFv sequences as the first, second and third binding domains may be derived from anti-CD19, anti-CD3 and anti-CD28 antibodies or antibody analogs or derivatives known in the art, respectively. For example, each binding domain may be a scFv comprising a VH and a VL derived from a known corresponding antibody. The antibody may be an animal-derived antibody such as a murine-derived antibody, a chimeric antibody such as a human-mouse chimeric antibody, or a human antibody or a humanized antibody.

In a preferred embodiment, the scFv specifically binding to CD19 comprises a heavy chain variable region (VH) and a light chain variable region (VL), wherein

(a) the VH comprises heavy chain CDR1 as set forth in SEQ ID NO: 11, heavy chain CDR2 as set forth in SEQ ID NO: 12, and heavy chain CDR3 as set forth in SEQ ID NO: 13, and the VL comprises light chain CDR1 as set forth in SEQ ID NO: 14, light chain CDR2 as set forth in SEQ ID NO: 15, and light chain CDR3 as set forth in SEQ ID NO: 16; or
(b) the VH comprises heavy chain CDR1 as set forth in SEQ ID NO: 46, heavy chain CDR2 as set forth in SEQ ID NO: 47, and heavy chain CDR3 as set forth in SEQ ID NO: 48, and the VL comprises light chain CDR1 as set forth in SEQ ID NO: 49, light chain CDR2 as set forth in SEQ ID NO: 50, and light chain CDR3 as set forth in SEQ ID NO: 51.

In a further preferred embodiment of the above embodiment, the scFv specifically binding to CD19 comprises a heavy chain variable region (VH) and a light chain variable region (VL), wherein

(a) the VH comprises or consists of an amino acid sequence as set forth in SEQ ID NO: 17, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 17, in which all amino acid differences are located in non-CDR regions; and the VL comprises or consists of an amino acid sequence as set forth in SEQ ID NO: 19, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 19, in which all amino acid differences are located in non-CDR regions; or

(b) the VH comprises or consists of an amino acid sequence as set forth in SEQ ID NO: 52, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 52, in which all amino acid differences are located in non-CDR regions; and the VL comprises or consists of an amino acid sequence as set forth in SEQ ID NO: 54, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 54, in which all amino acid differences are located in non-CDR regions.

In a further preferred embodiment, the scFv specifically binding to CD19 comprises or consists of an amino acid sequence as set forth in SEQ ID NO: 21 or SEQ ID NO: 56, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 21 or SEQ ID NO: 56, in which all amino acid differences are located in non-CDR regions. Preferably, the above amino acid differences are conservative substitutions of amino acids.

In one embodiment, in the scFv specifically binding to CD19, the VH is located at the C-terminus or N-terminus of the VL. In a preferred embodiment, in the scFv specifically binding to CD19, the VH is located at the C-terminus of the VL, i.e. in the linking order of VL-linker sequence-VH. In some embodiments, in the scFv specifically binding to CD19, the VH and VL are linked via an intra-domain linker or linked directly. The intra-domain linker is a linker containing (GGGGS)n or (GGS)n, or is (GGGGS)n or (GGS)n, in which n is an integer of 1-10, preferably an integer of 1-5, for example 1, 2, 3, 4 or 5. In a specific embodiment, in the scFv specifically binding to CD19, the intra-domain linker between VH and VL is selected from SEQ ID NO: 1, SEQ ID NO: 2 and SEQ ID NO: 3, preferably SEQ ID NO: 1.

In a preferred embodiment, the scFv specifically binding to CD3 comprises a heavy chain variable region (VH) and a light chain variable region (VL), wherein

(a) the VH comprises heavy chain CDR1 as set forth in SEQ ID NO: 22, heavy chain CDR2 as set forth in SEQ ID NO: 23, and heavy chain CDR3 as set forth in SEQ ID NO: 24, and the VL comprises light chain CDR1 as set forth in SEQ ID NO: 25, light chain CDR2 as set forth in SEQ ID NO: 26, and light chain CDR3 as set forth in SEQ ID NO: 27; or

(b) the VH comprises heavy chain CDR1 as set forth in SEQ ID NO: 57, heavy chain CDR2 as set forth in SEQ ID NO: 58, and heavy chain CDR3 as set forth in SEQ ID NO: 59, and the VL comprises light chain CDR1 as set forth in SEQ ID NO: 60, light chain CDR2 as set forth in SEQ ID NO: 61, and light chain CDR3 as set forth in SEQ ID NO: 62.

In a further preferred embodiment of the above embodiment, the scFv specifically binding to CD3 comprises a heavy chain variable region (VH) and a light chain variable region (VL), wherein

(a) the VH comprises or consists of an amino acid sequence as set forth in SEQ ID NO: 28, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 28, in which all amino acid differences are located in non-CDR regions; and the VL comprises or consists of an amino acid sequence as set forth in SEQ ID NO: 30, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 30, in which all amino acid differences are located in non-CDR regions; or

(b) the VH comprises or consists of an amino acid sequence as set forth in SEQ ID NO: 63, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 63, in which all amino acid differences are located in non-CDR regions; and the VL comprises or consists of an amino acid sequence as set forth in SEQ ID NO: 65, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 65, in which all amino acid differences are located in non-CDR regions.

In a further preferred embodiment, the scFv specifically binding to CD3 comprises or consists of an amino acid sequence as set forth in SEQ ID NO: 32 or SEQ ID NO: 67, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 32 or SEQ ID NO: 67, in which all amino acid differences are located in non-CDR regions. Preferably, the above amino acid differences are conservative substitutions of amino acids.

In one embodiment, in the scFv specifically binding to CD3, the VH is located at the C-terminus or N-terminus of the VL. In a preferred embodiment, in the scFv specifically binding to CD3, the VL is located at the C-terminus of the VH, i.e. in the linking order of VH-linker sequence-VL.

In some embodiments, in the scFv specifically binding to CD3, the VH and VL are linked via an intra-domain linker or linked directly. The intra-domain linker is a linker containing (GGGGS)n or (GGS)n, or is (GGGGS)n or (GGS)n, in which n is an integer of 1-10, preferably an integer of 1-5, for example 1, 2, 3, 4 or 5. In a specific embodiment, in the scFv specifically binding to CD3, the intra-domain linker between VH and VL is selected from SEQ ID NO: 1, SEQ ID NO: 2 and SEQ ID NO: 3, preferably SEQ ID NO: 1 or SEQ ID NO: 3.

In a preferred embodiment, the scFv specifically binding to CD28 comprises a heavy chain variable region (VH) and a light chain variable region (VL), wherein the VH comprises heavy chain CDR1 as set forth in SEQ ID NO: 33, heavy chain CDR2 as set forth in SEQ ID NO: 34, and heavy chain CDR3 as set forth in SEQ ID NO: 35, and the VL comprises light chain CDR1 as set forth in SEQ ID NO: 36, light chain CDR2 as set forth in SEQ ID NO: 37, and light chain CDR3 as set forth in SEQ ID NO: 38.

In a further preferred embodiment of the above embodiment, the scFv specifically binding to CD28 comprises a heavy chain variable region (VH) and a light chain variable region (VL), wherein the VH comprises or consists of an amino acid sequence as set forth in SEQ ID NO: 39, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 39, in which all amino acid differences are located in non-CDR regions; and the VL comprises or consists of an amino acid sequence as set forth in SEQ ID NO: 41, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 41, in which all amino acid differences are located in non-CDR regions.

In a further preferred embodiment, the scFv specifically binding to CD28 comprises or consists of an amino acid sequence as set forth in SEQ ID NO: 43, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 43, in which all amino acid differences are located in non-CDR regions.

Preferably, the above amino acid differences are conservative substitutions of amino acids.

In one embodiment, in the scFv specifically binding to CD28, the VH is located at the C-terminus or N-terminus of the VL. In a preferred embodiment, in the scFv specifically binding to CD28, the VL is located at the C-terminus of the VH, i.e. in the linking order of VH-linker sequence-VL.

In some embodiments, in the scFv specifically binding to CD28, the VH and VL are linked via an intra-domain linker or linked directly. The intra-domain linker is a linker containing (GGGGS)n or (GGS)n, or is (GGGGS)n or (GGS)n, in which n is an integer of 1-10, preferably an integer of 1-5, for example 1, 2, 3, 4 or 5. In a specific embodiment, in the scFv specifically binding to CD28, the intra-domain linker between VH and VL is selected from SEQ ID NO: 1, SEQ ID NO: 2 and SEQ ID NO: 3, preferably SEQ ID NO: 1.

Those skilled in the art will appreciate that the determination of the CDR regions may be determined using any one numbering manner of Kabat, IMGT and Chothia antibody numbering systems. Where different numbering manners are used, different CDR regions may be delineated for the amino acid sequence of the same variable region. The CDR regions determined, regardless of which numbering manner is used, fall within the scope of protection of the present invention. Unless otherwise stated, the amino acid sequences of particular CDR regions documented in the present application are determined according to the Kabat antibody numbering system.

In some embodiments, one or more of the first binding domain, the second binding domain, and the third binding domain of the present application have cross-reactivity with corresponding antigens in other species. The other species may be a laboratory animal, for example a monkey, such as a *Macaca fascicularis.* For example, in addition to specifically binding to human CD19, the first binding domain of the present application also has cross-reactivity with monkey CD19, i.e., can also specifically bind to monkey CD19. For example, in addition to specifically binding to human CD3, the second binding domain of the present application also has cross-reactivity with monkey CD3, i.e., can also specifically bind to monkey CD3. For example, in addition to specifically binding to human CD28, the third binding domain of the present application also has cross-reactivity with monkey CD28, i.e., can also specifically bind to monkey CD28. The cross-reactivity with the corresponding monkey antigen would facilitate antibody development.

Linker sequence

On the basis of the determination of the binding domains, how to design the linkers for linking the individual binding domains and the linkers for linking the VH and VL in each binding domain is also crucial for the structure and function of the trispecific antibody that, especially the former is crucial. In one aspect, the linker sequences, together with the functional domains, form the entirety of the trispecific antibody molecule, and the linker sequences will affect the structural stability of the entire trispecific antibody molecule. On the other hand, the length and flexibility of the linker sequences will affect the spatial position relationship between individual binding domains, and thus affect the binding of each domain to its target. In addition, whether certain amino acids in the linker sequences are easy to form further modifications, for example dimerization and glycosylation, will also have an impact on the overall properties of the trispecific antibody molecule.

The linker sequences of the present invention may be derived from naturally occurring sequences or modified variants thereof, or may be de novo designed artificial sequences. The linker sequence may be from a naturally occurring multidomain protein, for example, may be a linker sequence contained in a naturally occurring multidomain protein or a variant thereof. An example of a linker sequence from a naturally occurring sequence is a linker sequence from the IgD hinge region, which may be used in the present application as the second linker sequence.

Examples of artificial linker sequences are for example linker sequences based on $(GGGGS)_n$, $(GGGGS)_nGGGG$, $(SGGGG)_n$, $GGGG(SGGGG)_n$, $(GGS)_n$, $(GGS)_nGG$, $(SGG)_n$ or $GG(SGG)_n$, which can be used in the present invention as the first inter-domain linker, or as an intra-domain linker. Another example of an artificial linker sequence is a linker sequence comprising $(EAAAK)_n$, such as $A(EAAAK)_nA$, $A(EAAAK)_nALEA(EAAAK)_nA$, which can be used in the present invention as the second linker sequence.

The rigidity/flexibility of the linker is a factor which needs to be considered. A linker having flexibility allows the polypeptide sequences linked at both ends to be capable of moving within a certain range. Flexible linkers are usually composed of small-sized amino acids such as glycine (G), serine (S) and threonine (T), and it is these small-sized amino acids that endow the linker with flexibility. The use of flexible linkers is preferred where it is desirable to retain the possibility of some relative movement for the domains at both ends of the linker. However, in some cases, flexible linkers do not allow for proper expression or functioning of a multidomain molecule due to the inability to fix the domains linked at both ends. Considering from this aspect, for a multifunctional molecule, for example a trispecific antibody that needs to bind to three targets, rigid linkers may be able to provide better structural and functional stability than flexible linkers.

Specifically for the trispecific antibody of the present invention, the first domain targets tumor cells, while the second and third domains target immune cells, i.e., adjacent first and second domains will necessarily bind to different cells. In this case, it is desirable for the ability of certain relative movement between the first domain and the second domain, and the binding can be made easier by adjusting the position when binding to different cells. Thus, in a preferred embodiment, the first linker sequence of the present invention is a flexible linker, e.g. a linker consisting of one or more of glycine (G), serine (S), threonine (T) and lysine (K), such as a GS-rich linker, such as a linker containing $(GGGGS)_n$, $(SGGGG)_n$, $(GGS)_n$, $(SGG)_n$, for example $(GGGGS)_n$, $(GGGGS)_nGGGG$, $(SGGGG)_n$, $GGGG(SGGGG)_n$, $(GGS)_n$, $(GGS)_nGG$, $(SGG)_n$ or $GG(SGG)_n$, wherein n is a positive integer, preferably 1-10. The linker used to link VH and VL in each binding domain is also preferably such a flexible linker.

On the other hand, it is desirable for an engager-based antibody to draw the two types of cells engaged as close as possible, so that the distance of the immune synapse formed between the cells will be closer to the distance of the immune synapse naturally formed under the physiological state, which is more favourable for the killing effect exerted by immune cells. The distance at which the two types of cells are drawn closely is influenced by the length of the first linker sequence. For example, the length of the first linker sequence is no more than 30 amino acids, preferably no more than 25 amino acids, more preferably no more than 20 amino acids, still more preferably no more than 15 amino acids. For example, if the first linker sequence is any one of $(GGGGS)_n$, $(SGGGG)_n$, and $GGGG(SGGGG)_n$, then preferably n is a positive integer of no more than 6, preferably a positive integer of no more than 5, for example 1, 2, 3, 4 or 5; if the first linker sequence is any one of $(GGS)_n$, $(SGG)_n$ or $(GGS)_nGG$, then preferably n is a positive integer of no more than 10, preferably a positive integer of no more than 7, for example 1, 2, 3, 4, 5, 6 or 7. In a specific embodiment, the first linker sequence is $(GGGGS)_3$ (SEQ ID NO: 1), GGGGS (SEQ ID NO: 2), or $(GGS)_4GG$ (SEQ ID NO: 3), preferably the amino acid sequence as set forth in SEQ ID NO: 1.

The second linker sequence links the second and third domains, both of which will bind to receptors on the surface of T cells. In order to prevent the steric hindrance effect generated after binding of one of the domains from affecting the binding of the other domain to the same T cell, a slightly longer second linker sequence is more desirable. Thus, in a preferred embodiment, the second linker sequence is longer than the first linker sequence. For example, the length of the second linker sequence is at least 1.25-fold, at least 1.5-fold, at least 2-fold, at least 2.25-fold, at least 2.5-fold or longer than the length of the first linker sequence. For example, the length of the second linker sequence is at least 15 amino acids, at least 20 amino acids, at least 25 amino acids, at least 30 amino acids, at least 35 amino acids, at least 40 amino acids, or longer.

In addition, a linker that is biased towards rigidity may also be preferred as the second linker sequence. A more rigid linker is also advantageous to the avoid steric hindrance effect. For example, one example of the second linker is a linker sequence comprising $(EAAAK)_n$. The Glu-Ala-Ala-Ala-Lys structure in such a linker is capable of forming a rigid alpha helical structure. The "linker sequence comprising $(EAAAK)_n$" means that such linkers usually also contain other amino acid residues, for example, one or more of alanine (A), leucine (L), and glutamic acid (E) on both sides of the EAAAK repeat, and specific examples include $A(EAAAK)_nA$, $LEA(EAAAK)_nALE$, $LEA(EAAAK)_nALEA(EAAAK)_nALE$, etc., wherein n can be any integer of 1-10, preferably 2-6. The linker type can be selected and/or the value of n can be adjusted according to the required length of the linker sequence.

In one specific embodiment, one of the linker sequences of the present invention, e.g. the second linker sequence, is a fragment from the hinge region of immunoglobulin IgD or a variant thereof.

Potential glycosylation sites present in the linker sequence may also influence the properties and performance of the trispecific antibody molecule. For example, if the linker sequence contains a glycosylation site, the sugar chain formed by glycosylation may mask the active site in an unexpected manner, reducing the activity of the antibody. When antibodies are produced in cells, sugar chains of different compositions and lengths may be formed, resulting in a decrease in the uniformity of the antibody product. Common types of glycosylation include N-linked glycosylation and O-linked glycosylation. N-linked glycosylation is a type of glycosylation in which a sugar chain is linked to the nitrogen atom of the side chain of an amino acid such as asparagine and arginine, and O-linked glycosylation is a type of glycosylation in which a sugar chain is linked to the oxygen atom of the side chain hydroxyl of an amino acid such as serine, threonine, and tyrosine. When the linker sequence is derived from a natural sequence, for example from an immunoglobulin hinge region, potential glycosylation sites in the sequence, such as threonine, can be removed by introducing mutations to the natural sequence, including deletions, substitutions, truncations, and the like.

In a specific embodiment, a fragment of the IgD hinge region is used as a linker sequence, e.g., a second linker sequence. The IgD hinge region is known to include multiple O-linked glycosylation sites, for example serine at position 109 (S109) and four threonines (T126, T127, T131 and T132). These sites are included in the 81-amino acid long linker provided in the present application, and the amino acid sequence of the linker sequence is set forth in SEQ ID NO: 4 or SEQ ID NO: 8, where one serine and four threonines exemplified above are located at positions 20, 37, 38, 42 and 43, respectively. In a preferred embodiment, the IgD hinge region fragment of the present invention comprises no glycosylation site, for example does not comprise the five glycosylation sites described above. In one embodiment, the IgD hinge region fragment that comprises no glycosylation site may be obtained by truncation, for example by removing at least the five glycosylation sites described above. For example, the entire part before the last threonine (inclusive) may be removed, leaving only all or part of the C-terminal sequence. As another alternative, the amino acids at these five glycosylation sites can also be substituted with other amino acids by modification.

The single chain antibody may be formed as a dimer through the cysteine contained in the linker region. A dimer of a trispecific antibody comprises doubled antigen binding domains. Studies have shown that since CD28 exists as a dimer in natural state, bivalent antibodies comprising two domains that specifically bind to CD28 (e.g., antibodies of the IgG type) are more potent than monovalent antibodies (e.g., single chain antibodies). Similarly, the CD28 antibody will have higher potency when cross-linking occurs or when the degree of aggregation is higher. However, the trispecific antibody needs to bind to three targets correctly at the same time to exert their intended effect. If a trispecific antibody only binds to one or two targets (CD3 and/or CD28) on the T cell, but fails to bind to CD19 on the B cell tumor, it may result in T cell activation far away from the tumor target cell, i.e., causing "T cell activation at a non-target site". In this case, the trispecific antibody present as a dimer will be more dangerous, because of its greater ability and higher potency of binding of the T cell receptor CD28, and high susceptibility to cross-linking reactions, which will produce activation effect at a non-target site which is more difficult to deal with and increase the risk of occurrence of cytokine storm. Thus, it is not intended in the present invention that trispecific antibodies form dimers upon preparation and/or use, and for this purpose the linker sequence, in particular the second linker sequence based on the IgD hinge region, has been engineered to remove amino acid residues therein, e.g. cysteine (Cys; C) residues, which may cause dimer formation.

In one preferred embodiment, the linker sequence of the present invention comprises no amino acid site capable of forming dimers, for example cysteine (C). In a specific embodiment, the second linker sequence is derived from the IgD hinge region and has the cysteine (C161) removed therefrom. For example, the cysteine in the linker sequence is removed by substituting the cysteine at position 161 with another amino acid. For example, the cysteine in the linker sequence is removed by substituting the cysteine at position 161 with serine or glycine (C161S or C161G substitution).

In a preferred embodiment, the first linker sequence of the trispecific antibody of the present invention is a linker containing (GGGGS)n or (GGS)n, or is (GGGGS)n or (GGS)n, in which n is an integer of no more than 6, preferably an integer of no more than 5, for example 1, 2, 3, 4 or 5. In a specific embodiment, the first linker sequence is the amino acid sequence as set forth in SEQ ID NO: 1, SEQ ID NO: 2, or SEQ ID NO: 3. In a more specific embodiment, the first linker sequence is the amino acid sequence as set forth in SEQ ID NO: 1.

In a preferred embodiment, the second linker sequence of the trispecific antibody of the present invention is derived from the IgD hinge region and has one or more of the following characteristics:

(1) lack of cysteine, compared with the amino acid sequence as set forth in SEQ ID NO: 10;
(2) lack of glycosylation sites, compared with the amino acid sequence as set forth in SEQ ID NO: 10; and
(3) a shorter length, compared with the amino acid sequence as set forth in SEQ ID NO: 10.

In a preferred embodiment, the cysteine corresponding to position 72 in the second linker sequence is mutated to another amino acid, preferably serine or glycine, compared with SEQ ID NO: 10. In a specific embodiment, the second linker sequence is an amino acid sequence of SEQ ID NO: 4, SEQ ID NO: 6, SEQ ID NO: 8, or SEQ ID NO: 9.

In a preferred embodiment, the second linker sequence is a partial fragment of SEQ ID NO: 4 that contains no glycosylation site, for example, an N-terminal partial fragment or a C-terminal partial fragment of SEQ ID NO: 4 that contains no glycosylation site, preferably a partial fragment of the amino acid sequence of SEQ ID NO: 4 that consists of 38 amino acids at the C-terminal; the glycosylation sites comprise at least a serine corresponding to position 20 and threonines corresponding to positions 37, 38, 42 and 43. In a specific embodiment, the second linker sequence is the amino acid sequence as set forth in SEQ ID NO: 5, SEQ ID NO: 6, or SEQ ID NO: 9.

In the most preferred embodiment, the second linker sequence of the trispecific antibody of the present invention is an amino acid sequence as set forth in SEQ ID NO: 6 or SEQ ID NO: 9. In another preferred embodiment, the second linker sequence of the trispecific antibody of the present invention is a linker sequence comprising $(EAAAK)_n$, such as $A(EAAAK)_nA$, $LEA(EAAAK)_nALE$, $LEA(EAAAK)_nALEA(EAAAK)_nALE$, wherein n is any integer of 1 to 10, preferably 2-6, more preferably 2-4. Specifically, the second linker sequence is a linker sequence comprising the amino acid sequence as set forth in SEQ ID NO: 7, for example, a linker sequence comprising SEQ ID NO: 7 as a repeating unit, wherein the repeating unit is repeated 1-10 times.

In a preferred embodiment, the linker sequence used to link VH and VL in each binding domain in the trispecific antibody of the present invention, i.e. the intra-domain linker, is a linker containing (GGGGS)n or (GGS)n, or is (GGGGS)n or (GGS)n, in which n is a positive integer of no more than 10, preferably a positive integer of no more than 5, for example 1, 2, 3, 4 or 5. In some embodiments, the linker sequence used to link VH and VL in each binding domain is the same. In some embodiments, the linker sequence used to link VH and VL in each binding domain is different . For example, the intra-domain linker sequence is any one of $(GGGGS)_n$, $(SGGGG)_n$ and $GGGG(SGGGG)_n$, wherein preferably n is a positive integer of no more than 6, preferably a positive integer of no more than 5, for example 1, 2, 3, 4 or 5. For example, the intra-domain linker is any one of $(GGS)_n$, $(SGG)_n$ and $(GGS)_nGG$, wherein preferably n is a positive integer of no more than 10, preferably a positive integer of no more than 7, for example 1, 2, 3, 4, 5, 6 or 7.

In a specific embodiment, the intra-domain linker is selected from a sequence as set forth in SEQ ID NO: 1, SEQ ID NO: 2, or SEQ ID NO: 3. In a more specific embodiment, the linker used to link VH and VL in each binding domain is $(GGGGS)_3$ as set forth in SEQ ID NO: 1. In a more specific embodiment, the three intra-domain linkers are $(GGGGS)_3$ as set forth in SEQ ID NO: 1, $(GGS)_4GG$ as set forth in SEQ ID NO: 3, and $(GGGGS)_3$ as set forth in SEQ ID NO: 1, respectively.

Trispecific antibody

In the case that the antigen specificity and arrangement order of each antigen binding domain are determined, the specific sequence of each binding domain is not particularly limited. The three binding domains may be derived from corresponding antibodies or antibody analogs known in the art.

In a preferred embodiment, the trispecific antibody of the present invention is capable of specifically binding to human CD19, human CD3 and human CD28, and comprises a sequence such as SEQ ID NO: 4, SEQ ID NO: 5, SEQ ID NO: 6, SEQ ID NO: 8 or SEQ ID NO: 9 as a linker sequence. Preferably, the amino acid sequence of SEQ ID NO: 4, SEQ ID NO: 5, SEQ ID NO: 6, SEQ ID NO: 8 or SEQ ID NO: 9 is a linker sequence that links a binding domain specifically binding to CD3 and a binding domain specifically binding to CD28.

In a specific embodiment, the trispecific antibody of the present invention is a trispecific antibody with an amino acid sequence as set forth in SEQ ID NO: 44 or SEQ ID NO: 68; or its variant which has at least 80% homology with the amino acid sequence as set forth in SEQ ID NO: 44 or SEQ ID NO: 68, can specifically bind to CD19, CD3, and CD28, and comprises a sequence such as SEQ ID NO: 4, SEQ ID NO: 5, SEQ ID NO: 6, SEQ ID NO: 8 or SEQ ID NO: 9 as the linker sequence. Preferably, the amino acid sequence of the variant has at least 85%, preferably at least 90%, more preferably at least 95%, such as at least 96%, at least 97%, at least 98%, at least 99% homology with the amino acid sequence as set forth in SEQ ID NO: 44 or SEQ ID NO: 68.

Preparation method

The present invention also relates to a method for preparing the trispecific antibody. The method may be a molecular biological method. For example, the method comprises preparing one or more nucleotide sequences encoding the trispecific antibody as a single chain fusion protein, constructing the one or more coding nucleotide sequences into one or more expression vectors, and expressing the expression vector(s) in appropriate cells.

Those skilled in the art can understand that on the premise that the amino acid sequence of the trispecific antibody has been determined, due to the existence of codon degeneracy, different coding nucleotide sequences can be used, and the coding nucleotide sequences can also be optimized. Methods for performing codon optimization on a coding sequence are known to those skilled in the art, including adjusting codons to host-preferred codons for the host type, reducing GC content and/or reducing GC-rich regions, and increasing mRNA stability, so that the efficiency of expression of a nucleotide sequence of interest in a particular host is increased.

In a specific embodiment, the coding nucleotide sequence comprises a nucleotide sequence encoding the first binding domain that specifically binds to CD19 as described herein.

In a specific embodiment, the coding nucleotide sequence of the first binding domain may comprise: (1) a nucleotide sequence as set forth in SEQ ID NO: 18 encoding VH, or a nucleotide sequence having at least 85%, preferably at least 90%, more preferably at least 95%, such as at least 96%, at least 97%, at least 98%, at least 99% homology therewith and encoding the amino acid sequence of VH as set forth in SEQ ID NO: 17; and/or (2) a nucleotide sequence as set forth in SEQ ID NO: 20 encoding VL, or a nucleotide sequence having at least 85%, preferably at least 90%, more preferably at least 95%, such as at least 96%, at least 97%, at least 98%, at least 99% homology therewith and encoding the amino acid sequence of VL as set forth in SEQ ID NO: 19. In a specific embodiment, the coding nucleotide sequence of the first binding domain may comprise: (1) a nucleotide sequence as set forth in SEQ ID NO: 53 encoding VH, or a nucleotide sequence having at least 85%, preferably at least 90%, more preferably at least 95%, such as at least 96%, at least 97%, at least 98%, at least 99% homology therewith and encoding the amino acid sequence of VH as set forth in SEQ ID NO: 52; and/or (2) a nucleotide sequence as set forth in SEQ ID NO: 55 encoding VL, or a nucleotide sequence having at least 85%, preferably at least 90%, more preferably at least 95%, such as at least 96%, at least 97%, at least 98%, at least 99% homology therewith and encoding the amino acid sequence of VL as set forth in SEQ ID NO: 54. In a specific embodiment, the coding nucleotide sequence of the first binding domain further comprises a nucleotide sequence encoding the linker sequence as set forth in SEQ ID NO: 1, SEQ ID NO: 2 or SEQ ID NO: 3.

In a specific embodiment, the coding nucleotide sequence comprises a nucleotide sequence encoding the second binding domain that specifically binds to CD3 as described herein. The coding nucleotide sequence of the second binding domain may comprise: (1) a nucleotide sequence as set forth in SEQ ID NO: 29 encoding VH, or a nucleotide sequence having at least 85%, preferably at least 90%, more preferably at least 95%, such as at least 96%, at least 97%, at least 98%, at least 99% homology therewith and encoding the amino acid sequence of VH as set forth in SEQ ID NO: 28; and/or (2) a nucleotide sequence as set forth in SEQ ID NO: 31 encoding VL, or a nucleotide sequence having at least 85%, preferably at least 90%, more preferably at least 95%, such as at least 96%, at least 97%, at least 98%, at least 99% homology therewith and encoding the amino acid sequence of VL as set forth in SEQ ID NO: 30. In a specific embodiment, the coding nucleotide sequence of the first binding domain may comprise: (1) a nucleotide sequence as set forth in SEQ ID NO: 64 encoding VH, or a nucleotide sequence having at least 85%, preferably at least 90%, more preferably at least 95%, such as at least 96%, at least 97%, at least 98%, at least 99% homology therewith and encoding the amino acid sequence of VH as set forth in SEQ ID NO: 63; and/or (2) a nucleotide sequence as set forth in SEQ ID NO: 66 encoding VL, or a nucleotide sequence having at least 85%, preferably at least 90%, more preferably at least 95%, such as at least 96%, at least 97%, at least 98%, at least 99% homology therewith and encoding the amino acid sequence of VL as set forth in SEQ ID NO: 65. In a specific embodiment, the coding nucleotide sequence of the second binding domain further comprises a nucleotide sequence encoding the linker sequence as set forth in SEQ ID NO: 1, SEQ ID NO: 2 or SEQ ID NO: 3.

In a specific embodiment, the coding nucleotide sequence comprises a nucleotide sequence encoding the third binding domain that specifically binds to CD28 as described herein. The coding nucleotide sequence of the third binding domain may comprise: (1) a nucleotide sequence as set forth in SEQ ID NO: 40 encoding VH, or a nucleotide sequence having at least 85%, preferably at least 90%, more preferably at least 95%, such as at least 96%, at least 97%, at least 98%, at least 99% homology therewith and encoding the amino acid sequence of VH as set forth in SEQ ID NO: 39; and/or (2) a nucleotide sequence as set forth in SEQ ID NO: 42 encoding VL, or a nucleotide sequence having at least 85%, preferably at least 90%, more preferably at least 95%, such as at least 96%, at least 97%, at least 98%, at least 99% homology therewith and encoding the amino acid sequence of VL as set forth in SEQ ID NO: 41. In a specific embodiment, the coding nucleotide sequence of the third binding domain further comprises a nucleotide sequence encoding the linker sequence as set forth in SEQ ID NO: 1, SEQ ID NO: 2 or SEQ ID NO: 3.

In a specific embodiment, the nucleotide sequence encoding the trispecific antibody of the present invention comprises a nucleotide sequence as set forth in SEQ ID NO: 45, or a nucleotide sequence having at least 85%, preferably at least 90%, more preferably at least 95%, such as at least 96%, at least 97%, at least 98%, at least 99% homology therewith and encoding an amino acid sequence as set forth in SEQ ID NO: 44. In a specific embodiment, the nucleotide sequence encoding the trispecific antibody of the present invention comprises a nucleotide sequence as set forth in SEQ ID NO: 69, or a nucleotide sequence having at least 85%, preferably at least 90%, more preferably at least 95%, such as at least 96%, at least 97%, at least 98%, at least 99% homology therewith and encoding an amino acid sequence as set forth in SEQ ID NO: 68.

Suitable producer cells are known to those skilled in the art. In some embodiments, mammalian cells, e.g., 293T, CHO, or a derived cell line thereof, are used as producer cells. In some embodiments, microbial cells, e.g., bacterial cells or fungal cells, e.g., *E. coli* or yeast, are used as producer cells,. In some embodiments, insect cells e.g., Sf9, are used as producer cells. The nucleotide sequence encoding the trispecific antibody of the present invention may be codon optimized for a specific producer cell line.

The produced trispecific antibody is preferably purified. Purification of the trispecific antibody can be performed by a conventional method in the field of antibody production, which may include steps of filtration, chromatography, and the like. The filtration step may be selected from one or more of deep filtration, ultrafiltration, diafiltration, and nanofiltration. The chromatography step may be selected from one or more of affinity chromatography, cation chromatography, anion chromatography, size exclusion chromatography, hydrophobic chromatography, and hydroxyapatite chromatography.

The antibody of the present invention can be prepared into a liquid or solid preparation, for example an injection, a suspension, powder, and granules. For example, it can be prepared into a lyophilized powder injection by a conventional method for example freeze-drying, and then formulated into an injection for administration by reconstitution immediately before administration.

Tumor treatment

The trispecific antibody of the present invention may be used in therapeutic or diagnostic applications, for example the treatment of cancer.

As an engager-based antibody, the first binding domain of the trispecific antibody of the present invention is responsible for targeting and binding to a tumor-associated antigen. When the first binding domain specifically binds to CD19, the trispecific antibody of the present invention is particularly suitable for targeting target cells expressing CD19, for example CD19$^+$ B cells associated with a disease.

Thus, the trispecific antibody of the present invention is particularly suitable for the treatment of B cell-associated diseases, such as B cell-associated tumors, including B cell lymphoma and B cell leukemia. In particular, the B cell-associated tumors are relapsed or refractory B cell malignancies, that is, the subject has relapsed after having undergone a prior relevant treatment and having achieved therapeutic benefit, or has not responded expectedly to the prior relevant treatment.

The B cell lymphoma may be non-Hodgkin lymphoma (NHL), for example aggressive or indolent non-Hodgkin lymphoma, or Hodgkin lymphoma. Examples of the B cell-associated disease include, but are not limited to, classical Hodgkin lymphoma, nodular lymphocyte predominant Hodgkin lymphoma, diffuse large B-cell lymphoma, follicular lymphoma, mucosa associated lymphoid tissue lymphoma, small lymphocytic lymphoma, chronic lymphocytic leukemia, mantle cell lymphoma, Burkitt lymphoma, acute lymphoblastic leukemia, chronic lymphatic leukemia, B cell prolymphocytic leukemia, precursor B-cell lymphoblastic leukemia, hairy cell leukemia, splenic B cell lymphoma/leukemia, and splenic marginal zone lymphoma. Non-Hodgkin lymphoma (NHL), one of the most common cancers worldwide, represents a class of heterogeneous malignant lymphoid neoplasms. The major subtypes are B cell-derived, including diffuse large B-cell lymphoma (DLBCL) and follicular lymphoma (FL). Current treatment strategies for B cell-derived non-Hodgkin lymphoma include anti-CD20 rituximab and R-CHOP regimens of rituximab in combination with chemotherapy (rituximab, cyclophosphamide, doxorubicin, vincristine, prednisone). Although rituximab as a single agent or in combination with chemotherapy has improved therapeutic effect in patients with certain subtypes of NHL, there are still some patients who experience failure of R-CHOP treatment, causing their condition to progress into the relapsed/refractory stage. It is difficult for patients to obtain satisfactory effects after using anti-CD20 monoclonal antibodies again. The therapeutic effect of rituximab on B cell lymphoma depends on the expression level of CD20 in tumor cells, and the expression of CD20 in tumor cells is heterogeneous, patients with low expression of CD20 are insensitive to rituximab and have congenital resistance; in addition, patients with high expression of CD20 who are sensitive to rituximab have decreased CD20 expression or even negative for CD20 expression after treatment, resulting in acquired resistance. Blinatumomab, a bispecific T cell activator (BiTE) antibody, can selectively binds to the B cell surface antigen CD19 and the invariant CD3 portion of the T cell receptor (TCR), and is reported to have anti-lymphoma activity in early proof-of-concept clinical trials for relapsed or refractory B-NHL. Blinatumomab requires continuous 28-day intravenous infusion, and such difficult dosing mode is a significant obstacle to the application of Blinatumomab in the treatment of NHL. Unlike Blinatumomab, the trispecific antibody of the present invention can be administered less frequently and provide comparable or better tumor inhibition effect. In one specific embodiment, the trispecific antibody of the present invention is used in the treatment of non-Hodgkin lymphoma.

Precursor B-cell acute lymphoblastic leukemia (B-ALL) is an tumor of immature B cell precursors that usually affects children under 6 years of age, but also occurs in adolescent and adult populations. The main current treatment strategies for B-ALL are chemotherapy and hematopoietic stem cell transplantation (HSCT), the overall survival rate (OS) of pediatric B-ALL achieves 80-90%, and the OS rate of adult B-ALL is 30-50%. However, the prognosis for relapsed or refractory ALL is very poor in both age groups. The trispecific antibody of the present invention provides a new option of treatment regime for relapsed or refractory ALL. In one specific embodiment, the trispecific antibody of the present invention is used for the treatment of precursor B-cell acute lymphoblastic leukemia, in particular relapsed or refractory precursor B-cell acute lymphoblastic leukemia.

Dosing method

The trispecific antibody of the present invention may be delivered by a conventional method, preferably by systemic administration, for example administrated by intravenous infusion.

The antibody of the present invention may be administered in a therapeutically effective amount. The "therapeutically effective amount" refers to an amount of an antibody that, when administered to a subject to treat a disease or at least one clinical symptom of a disease or condition, is sufficient to be effective in such treatments for the disease, condition or symptom. The "therapeutically effective amount" may vary with the antibody, the disease, the condition and/or symptoms of the disease or condition, the severity of the disease, the condition and/or symptoms of the disease or condition, the age of the subject to be treated, and/or the body weight of the subject to be treated.

In one preferred embodiment of the present application, the dosing amount of the antibody (mg/kg) is based on the body weight of the subject. For example, a single administered dose of the trispecific antibody of the present invention may be in the range of about 1 ng/kg body weight to about 5 mcg/kg body weight, preferably in the range of 5 ng/kg body weight to about 2.5 mcg/kg body weight, more preferably in the range of 0.1 mcg/kg body weight to about 1 mcg/kg body weight. For example, a dose of about 0.1, 0.2, 0.3, 0.4, 0.5, 0.6, 0.7, 0.8, 0.9 or 1 mcg/kg body weight can be dosed.

An advantage of the trispecific antibody of the present invention is that it can activate T cells efficiently and completely with a low propensity for post-activation apoptosis to occur, and in addition, it can induce memory T cells to provide a persistent immune response, and thus can be dosed at relatively short dosing intervals. In a preferred embodiment, when the trispecific antibody of the present invention is administered by intravenous infusion, the interval between two dosing thereof may be not less than 1 day, not less than 3 days, not less than 5 days, or even one week. For example, the trispecific antibody of the present invention may be dosed 7 times per week, 5 times per week, 3 times per week, twice per week, or once per week. The trispecific antibody of the present invention may be dosed in a cycle of 2-4 weeks, e.g., 3 weeks, and for one or more cycles. The trispecific antibody of the present invention, when dosed at a frequency of once per week, can produce a better tumor inhibition effect than other antibodies, such as CD19xCD3 bispecific antibodies, such as Blinatumomab, at an equal dose (such as the same molar dose).

Drug combination

The trispecific antibody of the present invention may be used in combination with an additional anti-tumor treatment or drug.

In some embodiments, the trispecific antibody of the present invention may be used in combination with an additional antibody-based drug. The additional antibody may be an antibody-based drug that targets a different antigen on a target cell, such as a B cell. The additional antibody-based drug may be an antibody-based drug that specifically binds to CD20, CD22, CD79a, CD38, or the like. Specific examples include, but are not limited to, rituximab, inotuzumab ozogamicin.

In some embodiments, the trispecific antibody of the present invention may be used in combination with a targeting therapy. The targeting therapy is for example a tyrosine kinase inhibitor (TKI), which includes, but is not limited to, imatinib, gefitinib, erlotinib, sunitinib, lapatinib, neratinib, and pyrotinib.

In some embodiments, the trispecific antibody of the present invention may be used in combination with a small molecule chemotherapeutic agent, such as cyclophosphamide, vincristine sulfate, doxorubicin hydrochloride, dexamethasone, and the like.

The present invention also relates to the following items:

1. A trispecific antibody specifically binding to CD19, CD3, and CD28, wherein the trispecific antibody comprises:

(1) a first binding domain specifically binding to CD19;

(2) a first linker sequence;

(3) a second binding domain specifically binding to CD3;

(4) a second linker sequence, which is an amino acid sequence selected from SEQ ID NO: 4, SEQ ID NO: 5, SEQ ID NO: 6, SEQ ID NO: 8 or SEQ ID NO: 9; and

(5) a third binding domain specifically binding to CD28.

2. The trispecific antibody of item 1, wherein the trispecific antibody is a single chain fusion protein.

3. The trispecific antibody of item 1 or 2, wherein the first linker sequence comprises $(G_4S)n$ or $(G_2S)n$, or the first linker sequence is $(G_4S)n$ or $(G_2S)n$, wherein n is an integer of 1-6, preferably 1-3.

4. The trispecific antibody of item 3, wherein the first linker sequence is selected from an amino acid sequence of SEQ ID NO: 1, SEQ ID NO: 2, or SEQ ID NO: 3.

5. The trispecific antibody of any one of items 1-4, wherein the first binding domain is a single chain antibody specifically binding to CD19, comprising a heavy chain variable region (VH) and a light chain variable region (VL), wherein

(a) the VH comprises heavy chain CDR1 as set forth in SEQ ID NO: 11, heavy chain CDR2 as set forth in SEQ ID NO: 12, and heavy chain CDR3 as set forth in SEQ ID NO: 13, and the VL comprises light chain CDR1 as set forth in SEQ ID NO: 14, light chain CDR2 as set forth in SEQ ID NO: 15, and light chain CDR3 as set forth in SEQ ID NO: 16; or

(b) the VH comprises heavy chain CDR1 as set forth in SEQ ID NO: 46, heavy chain CDR2 as set forth in SEQ ID NO: 47, and heavy chain CDR3 as set forth in SEQ ID NO: 48, and the VL comprises light chain CDR1 as set forth in SEQ ID NO: 49, light chain CDR2 as set forth in SEQ ID NO: 50, and light chain CDR3 as set forth in SEQ ID NO: 51.

6. The trispecific antibody of item 5, wherein:

(a) the VH comprises or consists of an amino acid sequence as set forth in SEQ ID NO: 17, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 17, in which all amino acid differences are located in non-CDR regions; and the VL comprises or consists of an amino acid sequence as set forth in SEQ ID NO: 19, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 19, in which all amino acid differences are located in non-CDR regions; or

(b) the VH comprises or consists of an amino acid sequence as set forth in SEQ ID NO: 52, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 52, in which all amino acid differences are located in non-CDR regions; and the VL comprises or consists of an amino acid sequence as set forth in SEQ ID NO: 54, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 54, in which all amino acid differences are located in non-CDR regions.

7. The trispecific antibody of item 6, wherein the first binding domain comprises or consists of an amino acid sequence as set forth in SEQ ID NO: 21 or SEQ ID NO: 56, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 21 or SEQ ID NO: 56, in which all amino acid differences are located in non-CDR regions.

8. The trispecific antibody of any one of items 1-7, wherein the second binding domain is a single chain antibody specifically binding to CD3, comprising a heavy chain variable region (VH) and a light chain variable region (VL), wherein

(a) the VH comprises heavy chain CDR1 as set forth in SEQ ID NO: 22, heavy chain CDR2 as set forth in SEQ ID NO: 23, and heavy chain CDR3 as set forth in SEQ ID NO: 24, and the VL comprises light chain CDR1 as set forth in SEQ ID NO: 25, light chain CDR2 as set forth in SEQ ID NO: 26, and light chain CDR3 as set forth in SEQ ID NO: 27; or

(b) the VH comprises heavy chain CDR1 as set forth in SEQ ID NO: 57, heavy chain CDR2 as set forth in SEQ ID NO: 58, and heavy chain CDR3 as set forth in SEQ ID NO: 59, and the VL comprises light chain CDR1 as set forth in SEQ ID NO: 60, light chain CDR2 as set forth in SEQ ID NO: 61, and light chain CDR3 as set forth in SEQ ID NO: 62.

9. The trispecific antibody of item 8, wherein

(a) the VH comprises or consists of an amino acid sequence as set forth in SEQ ID NO: 28, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 28, in which all amino acid differences are located in non-CDR regions; and the VL comprises or consists of an amino acid sequence as set forth in SEQ ID NO: 30, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 30, in which all amino acid differences are located in non-CDR regions; or
(b) the VH comprises or consists of an amino acid sequence as set forth in SEQ ID NO: 63, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 63, in which all amino acid differences are located in non-CDR regions; and the VL comprises or consists of an amino acid sequence as set forth in SEQ ID NO: 65, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 65, in which all amino acid differences are located in non-CDR regions.

10. The trispecific antibody of item 9, wherein the scFv specifically binding to CD3 comprises or consists of an amino acid sequence as set forth in SEQ ID NO: 32 or SEQ ID NO: 67, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 32 or SEQ ID NO: 67, in which all amino acid differences are located in non-CDR regions.

11. The trispecific antibody of any one of items 1-10, wherein the third binding domain is a single chain antibody specifically binding to CD28, comprising a heavy chain variable region (VH) and a light chain variable region (VL), wherein the VH comprises heavy chain CDR1 as set forth in SEQ ID NO: 33, heavy chain CDR2 as set forth in SEQ ID NO: 34, and heavy chain CDR3 as set forth in SEQ ID NO: 35, and the VL comprises light chain CDR1 as set forth in SEQ ID NO: 36, light chain CDR2 as set forth in SEQ ID NO: 37, and light chain CDR3 as set forth in SEQ ID NO: 38.

12. The trispecific antibody of item 11, wherein

the VH comprises or consists of an amino acid sequence as set forth in SEQ ID NO: 39, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 39, in which all amino acid differences are located in non-CDR regions; and
the VL comprises or consists of an amino acid sequence as set forth in SEQ ID NO: 41, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 41, in which all amino acid differences are located in non-CDR regions.

13. The trispecific antibody of item 12, wherein the third binding domain comprises or consists of an amino acid sequence as set forth in SEQ ID NO: 43, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 43, in which all amino acid differences are located in non-CDR regions.

14. The trispecific antibody of any one of items 1-13, wherein each binding domain comprises a linker sequence for linking the heavy chain variable region and the light chain variable region, and the linker sequence comprises $(G_4S)n$ or $(G_2S)n$, wherein n is an integer of 1-10, preferably 1-5, more preferably 1-3.

15. The trispecific antibody of item 14, wherein the amino acid sequence of the linker sequence for linking the heavy chain variable region and the light chain variable region is selected from an amino acid sequence as set forth in SEQ ID NO: 1, SEQ ID NO: 2 or SEQ ID NO: 3.

16. The trispecific antibody of any one of items 1-15, comprising or consisting of an amino acid sequence as set forth in SEQ ID NO: 44 or SEQ ID NO: 68; or comprising or consisting of an amino acid sequence having at least 80%, at least 90%, at least 95%, at least 96%, at least 97%, at least 98%, or at least 99% homology with the amino acid sequence as set forth in SEQ ID NO: 44 or SEQ ID NO: 68.

17. An isolated nucleic acid molecule encoding the trispecific antibody of any one of items 1-16.

18. The isolated nucleic acid molecule of item 17, wherein the nucleotide sequence encoding the first binding domain comprises the following nucleotide sequences:

(1) a nucleotide sequence as set forth in SEQ ID NO: 18 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 17; and/or
(2) a nucleotide sequence as set forth in SEQ ID NO: 20 encoding VL, or a nucleotide sequence having at least 85%, preferably at least 90%, more preferably at least 95%, such as at least 96%, at least 97%, at least 98%, at least 99% homology therewith encoding VL as set forth in SEQ ID NO: 19.

19. The isolated nucleic acid molecule of item 17, wherein the nucleotide sequence encoding the first binding domain comprises the following nucleotide sequences:

(1) a nucleotide sequence as set forth in SEQ ID NO: 53 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 52; and/or
(2) a nucleotide sequence as set forth in SEQ ID NO: 55 encoding VL, or a nucleotide sequence having at least 85%, preferably at least 90%, more preferably at least 95%, such as at least 96%, at least 97%, at least 98%, at least 99% homology therewith encoding VL as set forth in SEQ ID NO: 54.

20. The isolated nucleic acid molecule of items 17-19, wherein the nucleotide sequence encoding the second binding domain comprises the following nucleotide sequences:

(1) a nucleotide sequence as set forth in SEQ ID NO: 29 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 28; and/or
(2) a nucleotide sequence as set forth in SEQ ID NO: 31 encoding VL, or a nucleotide sequence having at least 85%, preferably at least 90%, more preferably at least 95%, such as at least 96%, at least 97%, at least 98%, at least 99% homology therewith encoding VL as set forth in SEQ ID NO: 30.

21. The isolated nucleic acid molecule of items 17-19, wherein the nucleotide sequence encoding the second binding domain comprises the following nucleotide sequences:

(1) a nucleotide sequence as set forth in SEQ ID NO: 64 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 63; and/or
(2) a nucleotide sequence as set forth in SEQ ID NO: 66 encoding VL, or a nucleotide sequence having at least 85%, preferably at least 90%, more preferably at least 95%, such as at least 96%, at least 97%, at least 98%, at least 99% homology therewith encoding VL as set forth in SEQ ID NO: 65.

22. The isolated nucleic acid molecule of any one of items 17-21, wherein the nucleotide sequence encoding the third binding domain comprises the following nucleotide sequences:

(1) a nucleotide sequence as set forth in SEQ ID NO: 40 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 39; and/or
(2) a nucleotide sequence as set forth in SEQ ID NO: 42 encoding VL, or a nucleotide sequence having at least 85%, preferably at least 90%, more preferably at least 95%, such as at least 96%, at least 97%, at least 98%, at least 99% homology therewith encoding VL as set forth in SEQ ID NO: 41.

23. The isolated nucleic acid molecule of any one of items 17-22, comprising:

(1) a nucleotide sequence encoding the first binding domain, comprising a nucleotide sequence as set forth in SEQ ID NO: 18 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 17; and a nucleotide sequence as set forth in SEQ ID NO: 20 encoding VL, or a nucleotide sequence having at least 85%, preferably at least 90%, more preferably at least 95%, such as at least 96%, at least 97%, at least 98%, at least 99% homology therewith and encoding VL as set forth in SEQ ID NO: 19;

(2) a nucleotide sequence encoding the second binding domain, comprising a nucleotide sequence as set forth in SEQ ID NO: 29 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 28; and a nucleotide sequence as set forth in SEQ ID NO: 31 encoding VL, or a nucleotide sequence having at least 85%, preferably at least 90%, more preferably at least 95%, such as at least 96%, at least 97%, at least 98%, at least 99% homology therewith and encoding VL as set forth in SEQ ID NO: 30; and

(3) a nucleotide sequence encoding the third binding domain, comprising a nucleotide sequence as set forth in SEQ ID NO: 40 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 39; and a nucleotide sequence as set forth in SEQ ID NO: 42 encoding VL, or a nucleotide sequence having at least 85%, preferably at least 90%, more preferably at least 95%, such as at least 96%, at least 97%, at least 98%, at least 99% homology therewith and encoding VL as set forth in SEQ ID NO: 41.

24. The isolated nucleic acid molecule of any one of items 17-22, comprising:

(1) a nucleotide sequence encoding the first binding domain, comprising a nucleotide sequence as set forth in SEQ ID NO: 53 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 52; and a nucleotide sequence as set forth in SEQ ID NO: 55 encoding VL, or a nucleotide sequence having at least 85%, preferably at least 90%, more preferably at least 95%, such as at least 96%, at least 97%, at least 98%, at least 99% homology therewith and encoding VL as set forth in SEQ ID NO: 54;

(2) a nucleotide sequence encoding the second binding domain, comprising a nucleotide sequence as set forth in SEQ ID NO: 64 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 63; and a nucleotide sequence as set forth in SEQ ID NO: 66 encoding VL, or a nucleotide sequence having at least 85%, preferably at least 90%, more preferably at least 95%, such as at least 96%, at least 97%, at least 98%, at least 99% homology therewith and encoding VL as set forth in SEQ ID NO: 65; and

(3) a nucleotide sequence encoding the third binding domain, comprising a nucleotide sequence as set forth in SEQ ID NO: 40 encoding VH, or a nucleotide sequence having at least 85%, preferably at least 90%, more preferably at least 95%, such as at least 96%, at least 97%, at least 98%, at least 99% homology therewith and encoding VH as set forth in SEQ ID NO: 39; and a nucleotide sequence as set forth in SEQ ID NO: 42 encoding VL, or a nucleotide sequence having at least 85%, preferably at least 90%, more preferably at least 95%, such as at least 96%, at least 97%, at least 98%, at least 99% homology therewith and encoding VL as set forth in SEQ ID NO: 41.

25. The isolated nucleic acid molecule of any one of items 17-24, comprising a nucleotide sequence encoding a linker sequence.

26. The isolated nucleic acid molecule of any one of items 17-25, comprising or consisting of a nucleotide sequence as set forth in SEQ ID NO: 45 or SEQ ID NO: 69.

27. An expression vector comprising the isolated nucleic acid molecule of any one of items 17-26.

28. A host cell comprising the isolated nucleic acid molecule of any one of items 17-26 or the expression vector of item 27.

29. The host cell of item 28, wherein the host cell is selected from a bacterial cell, a fungal cell, an insect cell, or an animal cell.

30. The host cell of item 29, wherein the animal cell is a mammalian cell and is selected from a 293T cell, a CHO cell, or a derived cell line thereof.

31. A method for preparing a trispecific antibody, comprising:

(a) providing the isolated nucleic acid molecule of any one of items 17-26 or the expression vector of item 27,
(b) introducing the isolated nucleic acid molecule or the expression vector in (a) into a host cell,
(c) culturing the host cell obtained in (b) to express the trispecific antibody, and
(d) recovering the trispecific antibody from the culture of the cell.

32. A method for preparing a trispecific antibody, comprising:

(a) providing the host cell of item 28 or 29,
(b) culturing the host cell to express the trispecific antibody, and
(c) recovering the trispecific antibody from the culture of the cell.

33. The method of item 31 or 32, further comprising purifying the recovered trispecific antibody.
34. A trispecific antibody obtained by the method of any one of items 31-33.
35. Use of the trispecific antibody of any one of items 1-16 or 34 in the preparation of a medicament.
36. The use of item 35, wherein the medicament is used for treating a B cell-associated tumor.
37. The use of item 36, wherein the B cell-associated tumor is B cell lymphoma.
38. The use of item 36, wherein the B cell-associated tumor is B cell leukemia.
39. The use of item 37, wherein the B cell-associated tumor is a refractory or relapsed B cell tumor.
40. The use of item 37 or 39, wherein the B cell lymphoma is aggressive or indolent non-Hodgkin lymphoma.
41. The use of item 38 or 39, wherein the B cell leukemia is B cell acute lymphoblastic leukemia.
42. A method for treating a B cell-associated tumor in a subject in need thereof, comprising administering to the subject a therapeutically effective amount of the trispecific antibody of any one of items 1-16 or 34.
43. The method of item 42, wherein the B cell-associated tumor is a refractory or relapsed B cell tumor.
44. The method of item 42 or 43, wherein the B cell-associated tumor is B cell lymphoma or B cell leukemia.
45. The method of any one of items 42-44, wherein the B cell-associated tumor is aggressive or indolent non-Hodgkin lymphoma.
46. The method of any one of items 42-44, wherein the B cell-associated tumor is B cell acute lymphoblastic leukemia.
47. The method of any one of items 42 to 46, wherein the trispecific antibody is dosed at a frequency of 3 times per week, 2 times per week, or once per week.
48. The method of any one of items 42 to 47, wherein the trispecific antibody is dosed at a dose of about 1 ng/kg body weight to about 5 mcg/kg body weight, preferably 10 ng/kg body weight to about 2.5 mcg/kg body weight, more preferably 0.1 mcg/kg body weight to about 1 mcg/kg body weight.
49. A pharmaceutical composition comprising the trispecific antibody of any one of items 1-16 or 34, and a pharmaceutically acceptable carrier.
50. A pharmaceutical combination comprising the trispecific antibody of any one of items 1-16 or 34, and an additional therapeutic agent.
51. The pharmaceutical combination of item 50, wherein the additional therapeutic agent is a therapeutic agent for treating a B cell-associated tumor.
52. A linker sequence, wherein the linker sequence is derived from the amino acid sequence of an IgD hinge region, has a length of 20-85 amino acids, and has at least one or both of the following characteristics:

(1) comprising no cysteine; and
(2) comprising no glycosylation site.

53. The linker sequence of item 52, wherein the cysteine is substituted with another amino acid relative to the amino acid sequence of the IgD hinge region.
54. The linker sequence of item 53, wherein the cysteine is substituted with serine or glycine.
55. The linker sequence of item 53 or 54, wherein the substituted cysteine is a cysteine at position 161.
56. The linker sequence of any one of items 52 to 55, wherein the glycosylation sites are serine at position 109 and threonines corresponding to positions 126, 127, 131 and 132.
57. The linker sequence of any one of items 52 to 56, wherein the glycosylation sites are removed by an amino acid substitution or truncation relative to the amino acid sequence of the IgD hinge region.
58. The linker sequence of any one of items 52 to 57, wherein the linker sequence has one or more of the following characteristics:

(1) lack of cysteine, compared with the amino acid sequence as set forth in SEQ ID NO: 10;
(2) lack of glycosylation sites, compared with the amino acid sequence as set forth in SEQ ID NO: 10; and
(3) a shorter length, compared with the amino acid sequence as set forth in SEQ ID NO: 10.

59. The linker sequence of any one of items 52 to 58, wherein the linker sequence has an amino acid sequence as set forth in SEQ ID NO: 4, SEQ ID NO: 5, SEQ ID NO: 6, SEQ ID NO: 8, or SEQ ID NO: 9.

60. A fusion protein comprising the linker sequence of any one of items 52 to 59.

61. The fusion protein of item 60, wherein the fusion protein is a multispecific antibody.

62. The fusion protein of item 61, wherein the linker sequence is used for linking domains having different antigen specificities.

64. Use of the linker sequence of any one of items 52 to 59 for linking different domains in a fusion protein.

**Examples**

For a more complete understanding and application of the present invention, the present invention will be described in detail below with reference to the examples and the drawings, and the examples are only intended to illustrate the present invention and are not intended to limit the scope of the present invention. The scope of the present invention is specifically defined by the appended claims.

Example 1. Construction of eukaryotic expression vectors expressing trispecific antibodies LMOAME and MLMOF

This example exemplarily describes the method for the preparation of two trispecific antibodies LMOAME and MLMOF, in particular for the construction of expression vectors for expressing the trispecific antibodies.

The amino acid sequence of LMOAME or MLMOF is as set forth in SEQ ID NO:44 or SEQ ID NO:68 below, respectively, wherein the respective VH and VL sequences are represented in normal font; the three linker sequences for linking the respective VH and VL within the three binding domains are underlined; and the two linker sequences for linking different binding domains between the individual binding domains are boxed.

The complete amino acid sequence of LMOAME (SEQ ID NO: 44)

```
DIQLTQSPAS LAVSLGQRAT ISCKASQSVD YDGDSYLNWY QQIPGQPPKL LIYDASNLVS 61
GIPPRFSGSG SGTDFTLNIH PVEKVDAATY HCQQSTEDPW TFGGGTKLEI KGGGGSGGGG 121
SGGGGSQVQL QQSGAELVRP GSSVKISCKA SGYAFSSYWM NWVKQRPGQG LEWIGQIWPG 181
DGDTNYNGKF KGKATLTADE SSSTAYMQLS SLASEDSAVY FCARRETTTV GRYYYAMDYW 241
GQGTTVTVSS GGGGSDIKLQ QSGAELARPG ASVKMSCKTS GYTFTRYTMH WVKQRPGQGL 301
EWIGYINPSR GYTNYNQKFK DKATLTTDKS SSTAYMQLSS LTSEDSAVYY CARYYDDHYC 361
LDYWGQGTTL TVSSVEGGSG GSGGSGGSGG VDDIQLTQSP AIMSASPGEK VTMTCRASSS 421
VSYMNWYQQK SGTSPKRWIY DTSKVASGVP YRFSGSGSGT SYSLTISSME AEDAATYYCQ 481
QWSSNPLTFG AGTKLELKRN TGRGGEEKKK EKEKEEQEER ETKTPESPSH TQPLGVQVQL 541
VQSGAEVKKP GASVKVSCKA SGYTFTSYYI HWVRQAPGQG LEWIGCIYPG NVNTNYNEKF 601
KDRATLTVDT SISTAYMELS RLRSDDTAVY FCTRSHYGLD WNFDVWGQGT TVTVSSGGGG 661
SGGGGSGGGGSDIQMTQSPS SLSASVGDRV TITCHASQNI YVWLNWYQQK PGKAPKLLIY 721
KASNLHTGVPSRFSGSGSGT DFTLTISSLQ PEDFATYYCQ QGQTYPYTFG GGTKVEIKR
```

The complete amino acid sequence of MLMOF (SEQ ID NO: 68)

```
DIVMTQSPAT LSLSPGERAT LSCRSSKSLQ NVNGNTYLYW FQQKPGQSPQ LLIYRMSNLN 61
SGVPDRFSGS GSGTEFTLTI SSLEPEDFAV YYCMQHLEYP ITFGAGTKLE IKGGGGSGGG 121
GSGGGGSEVQ LVESGGGLVK PGGSLKLSCA ASGYTFTSYV MHWVRQAPGK GLEWIGYINP 181
YNDGTKYNEK FQGRVTISSD KSISTAYMEL SSLRSEDTAM YYCARGTYYY GTRVFDYWGQ 241
GTLVTVSSGG GGSEVQLVES GGGLVQPGGS LKLSCAASGF TFNKYAMNWV RQAPGKGLEW 301
VARIRSKYNN YATYYADSVK DRFTISRDDS KNTAYLQMNN LKTEDTAVYY CVRHGNFGNS 361
YISYWAYWGQ GTLVTVSSGG GGSGGGGSGG GGSQTVVTQE PSLTVSPGGT VTLTCGSSTG 421
AVTSGNYPNW VQQKPGQAPR GLIGGTKFLA PGTPARFSGS LLGGKAALTL SGVQPEDEAE 481
YYCVLWYSNR WVFGGGTKLT VLRNTGRGGE EKKKEKEKEE QEERETKTPE SPSHTQPLGV 541
QVQLVQSGAE VKKPGASVKV SCKASGYTFT SYYIHWVRQA PGQGLEWIGC IYPGNVNTNY 601
NEKFKDRATL TVDTSISTAY MELSRLRSDD TAVYFCTRSH YGLDWNFDVW GQGTTVTVSS 661
GGGGSGGGGS GGGGSDIQMT QSPSSLSASV GDRVTITCHA SQNIYVWLNW YQQKPGKAPK 721
LLIYKASNLH TGVPSRFSGS GSGTDFTLTI SSLQPEDFAT YYCQQGQTYP YTFGGGTKVE 781
IKR
```

As shown above, different single chain antibody sequences are used in the CD19 binding domains and the CD3 binding domains in LMOAME and MLMOF, wherein the CD19 binding domain and the CD3 binding domain of MLMOF are cross-reactive with the corresponding monkey antigen. The CD28 binding domains in LMOAME and MLMOF are the same, and this CD28 binding domain is cross-reactive with the corresponding monkey antigen. In LMOAME and MLMOF, five of the six intra-domain linkers for linking VH and VL are $(G_4S)_3$ (SEQ ID NO: 1), and only the linker for linking VH and VL in the second binding domain (CD3 binding domain) of LMOAME is $(G_2S)_3G_2$ (SEQ ID NO: 3). LMOAME and MLMOF use completely identical interdomain linkers, including the first linker sequence $G_4S$ (SEQ ID NO: 2) for linking the CD19 binding domain and the CD3 binding domain, and the second linker sequence RNTGRGGE EKKKEKEKEE QEERETKTPE SPSHTQPLGV (SEQ ID NO: 6) for linking the CD3 binding domain and the CD28 binding domain.

In order to express the above two trispecific antibodies in mammalian cells, the codon optimization was first performed on the nucleotide sequences encoding the trispecific molecules comprising the CD19 binding domain, CD3 binding domain and CD28 binding domain, wherein the optimized nucleotides encoding the full trispecific antibodies are set forth in SEQ ID NO: 45 (LMOAME) and SEQ ID NO: 69 (MLMOF), respectively. These two nucleotide sequences encoding the full-length amino acid sequences of the antibodies were separately synthesized, and then the synthesized sequences were cloned as inserts into the pWNP15 (Novoprotein) expression vectors to form antibody expression constructs pWNP15-LMOAME and pWNP15-MLMOF. In the process, both ends of the insert were provided with EcoRI and SalI restriction sites by a chemical synthesis method, which facilitated the cloning of the synthesized inserts into the expression vector pWNP15 linearized by EcoRI and SalI. The obtained recombinant plasmids were identified by sequencing. The recombinant plasmid verified to be correct by sequencing will be used for construction of CHO-DG44 (Thermo Fisher/Life Technologies, Lot no. 55463WCB2) strains with stable expression. The constructed recombinant plasmid was linearized with PvuI, and transfected into the CHO-DG44 using a PEI-mediated method, and the monoclonal cell strains stably transfected with the two constructs were screened out by a method involving cell expansion, methotrexate (MTX) pressure screening and limiting dilution of cells, called CHO-DG44-LMOAME and CHO-DG44-MLMOF, respectively.

Example 2. Expression and purification of trispecific antibodies LMOAME and MLMOF

In this example, the description continues with the method for preparing two exemplary trispecific antibodies.

2.1 Expression and purification of LMOAME

The stably transfected CHO-DG44 strain cells comprising the recombinant plasmid encoding LMOAME which were obtained in Example 1 were taken to perform scale-up culture by a series of shake-flask culture, followed by culturing the cells in a reactor, allowing proteins to be expressed and secreted into the culture supernatant, harvesting the supernatant, clarifying and filtering, and then purifying the antibody product obtained in the culture supernatant according to the following method.

Capto L packing (purchased from Cytiva, Inc.) was used to capture the target protein, and the chromatography column was first treated with an affinity washing solution for 3-4 column volumes. The column was equilibrated with an affinity equilibration solution for at least 4 column volumes. The clarified cell harvest solution was loaded in one batch. After loading was complete, the column was equilibrated with an affinity equilibration solution for 3-4 column volumes. The column was then rinsed with an affinity rinsing solution for at least 4 column volumes. The protein was eluted with an affinity eluent after rinsing was complete, the protein was collected when the ultraviolet absorption value increased to 10-50 mAU/2mm, and the collection was ended when the ultraviolet absorption value reduced to 10-50 mAU/2mm. The eluted protein was mixed evenly by stirring and weighed, and the protein concentration was determined.

The target protein captured by affinity chromatography was then treated by a series of purification steps such as low pH virus inactivation, hydrophobic chromatography, anion chromatography, UF/DF, and nanofiltration, thus obtaining the purified protein.

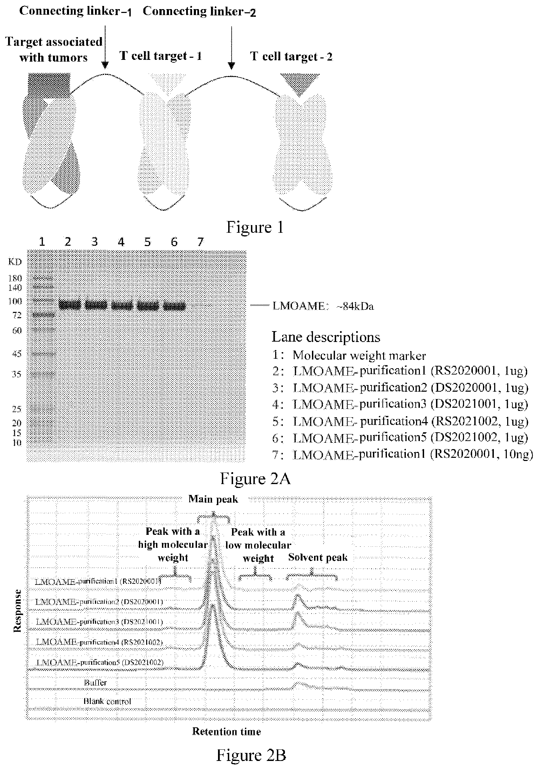

The LMOAME protein purified in the multiple steps was analyzed by SDS-PAGE and SEC-HPLC. The analysis results of the SDS-PAGE and SEC-HPLC are shown in FIGS. 2A-B, where bands of the correct molecular weight were obtained for 5 different batches of samples.

As shown in FIG. 2A, under the non-reducing SDS-PAGE condition, the LMOAME protein prepared in this example showed a single electrophoresis band. The theoretical molecular weight of the LMOAME recombinant protein was 83.8 kDa, and the results showed that the molecular weight of the purified protein was consistent with the theoretical molecular weight of the monomer. In addition, it can be seen from the SEC-HPLC plot of FIG. 2B that after purification by the method and steps of this example, the purity of LMOAME was > 95%, and no obvious protein formed by misfolding or polymerization was observed in the cell culture solution.

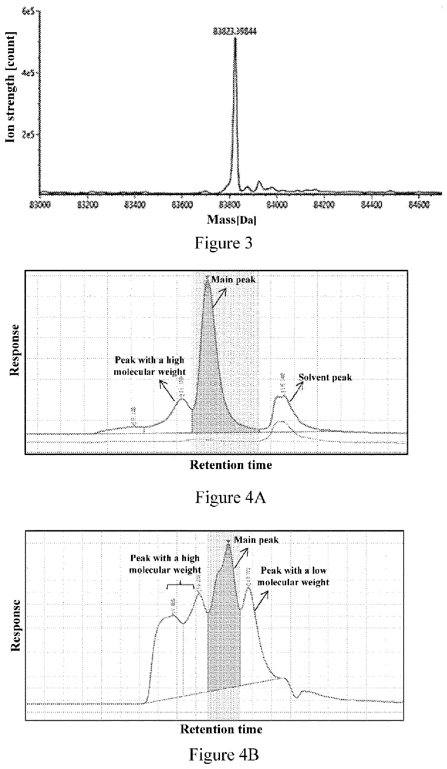

Mass spectrometry analysis was performed on the purified LMOAME by reverse phase ultra performance liquid chromatography-quadrupole-time of flight mass spectrometry (XEVO G2-XS-QToF, Waters), and the results are shown in FIG. 3. As shown in FIG 3, the purified LMOAME has only one major component, with its molecular weight consistent with the theoretical molecular weight of the monomer, without glycosylation modification.

Further N-terminal sequence analysis of the purified LMOAME samples was performed, and the result showed that the reading frame of the expressed recombinant protein was correct.

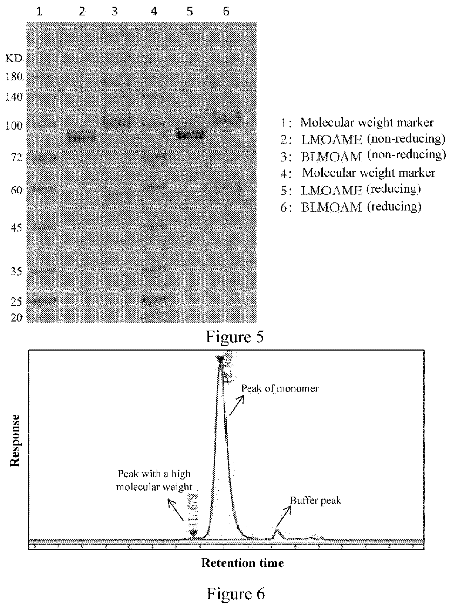

In order to facilitate the comparison of the effects of different linker designs on the physicochemical properties of the trispecific antibody, another trispecific antibody BLMOAM previously developed by the inventors (see Chinese Patent Application Publication No. CN106589129A) was used as a control and subjected to one-step affinity chromatography for capture and purification along with LMOAME, and the purification results are shown in FIGS. 4A-B and FIG. 5. BLMOAM is also a trispecific antibody specifically binding to CD19, CD3, and CD28, but using G4S as a linker between the first and second binding domains (linking fragment 1), and a linker of up to 81 amino acids containing cysteine between the second and third binding domains (linking fragment 2, SEQ ID NO: 29 in this application). The results showed that compared with BLMOAM, LMOAME had a uniform composition, and contained less components of dimers or polymers, indicating that LMOAME was not prone to form dimers or polymers.

<u>2.2 Purification of MLMOF</u>

The antibody product in the culture supernatant of cells transfected with the recombinant plasmid encoding MLMOF was purified employing a method and steps similar to those in the experiment in section 2.1, and the results were shown in FIG. 6 demonstrating a purity similar to that of LMOAME; and the results of N-terminal sequence analysis indicated that the reading frame of the expressed recombinant protein was correct.

Example 3. ELISA detection of antigen-binding activity of the trispecific antibody LMOAME This example employs an ELISA assay to assess the binding activity of LMOAME to recombinant human CD19 antigen (rhCD19) and recombinant human CD28 antigen (rhCD28).

Recombinant human CD19 antigen (CD19 (C-Fc), Novoprotein, Cat#: C572), and CD28 antigen (CD28 (C-Fc), Novoprotein, Cat#: C81) were diluted to 2 μg/mL with a protein coating solution, and plates were coated with the dilutions of 100 μL per well overnight at 2-8°C. The plates were washed the next day and blocked with a blocking solution of 1% Casein + 3% BSA (PBS + 2% BSA (V/W)).

The LMOAME purified in Example 2 was serially diluted with PBS to specified concentration ranges (rhCD19 binding assay: 0.0016-30 μg/mL; rhCD28 binding assay: 0.0006-10 μg/mL). The diluted antibodies were added to the 96-well flat bottom plates coated with antigens respectively and incubated for 1 h at 37°C. After washing the plates with PBST (PBS + 0.05% Tween-20 (V/V)), the detection was performed with HRP-protein L (GenScript, Cat#: M00098) and TMB (KPL SureBlue™, Cat#: 5120-0075).

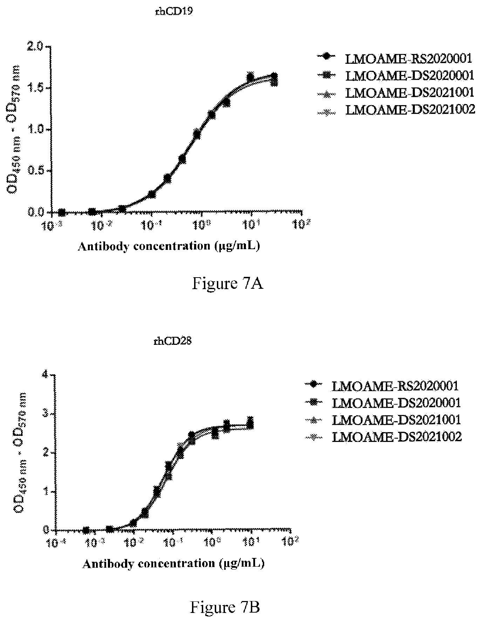

Data were analyzed using GraphPad Prism software, and the half-maximum effective concentration $EC_{50}$ value was used to represent the binding capacity of LMOAME to rhCD19 and rhCD28 antigens. The results are shown in FIG. 7.

The results showed that the binding capacity of LMOAME to rhCD19 and rhCD28 antigens was strong, with $EC_{50}$ values ranging from 0.3607-0.6853 and 0.0571-0.0758 μg/mL, respectively. Example 4. In vitro cell binding method to assess the binding activity of the trispecific antibody LMOAME to membrane-bound antigens

This example employed in vitro cell binding method to assess the binding activity of LMOAME to membrane-bound antigens hCD19, hCD3e, and hCD28.

Test cells (A549-CD19, CHO-CD28 or H9) expressing the target antigens were added to a 96-well plate at a density of $1 \times 10^5$ cells per well, with negative cells not expressing the target proteins as controls. Specifically, the cells and negative controls used for detection for each antigen were as follows.

For detection of the binding activity to hCD19, A549-CD19 cell line, which was a stably transfected A549 strain expressing CD19 with a phenotype of CD19$^+$CD3$^-$CD28$^-$, was used; and the A549 cell line (purchased from CCTCC, GDC0063) not expressing CD19 was used as a negative control.

For detection of the binding activity to hCD3, the H9 cell line (purchased from CCTCC, GDC0031), which was a human T lymphoma cell expressing CD3 with a phenotype of CD19$^-$CD3$^+$CD28$^-$, was used; and the CHO cell line not expressing CD19, CD3 and CD28 was used as a negative control.

For detection of the binding activity to hCD28, the CHO-CD28 cell line, which was a stably transfected CHO cell strain expressing CD28 with a phenotype of CD19$^-$CD3$^-$CD28$^+$, was used; and the CHO cell line not expressing CD19, CD3 and CD28 was used as a negative control.

The LMOAME purified in Example 2 was diluted with staining buffer STB (PBS containing 0.5% vol FBS) and then added to the above 96-well plate at final concentrations of 30, 10, 3, 1, 0.3, 0.1, 0.03 and 0.01 µg/mL respectively, and incubated with the test cells at 4°C for 1 h. Unbound antibodies were removed by centrifugation. Cells bound to LMOAME antibodies were detected by Biotin-protein L(GenScript, Cat#: M00097) and APC-Streptavidin (Biolegend, Cat#: 405207), and the mean fluorescence intensity (MFI) was recorded by Thermo Fisher Attune NxT flow cytometer.

Data were analyzed using GraphPad Prism software, and the half-maximum effective concentration EC$_{50}$ value was used to represent the binding capacity of LMOAME to membrane-bound antigens hCD19, hCD3e and hCD28.

The results showed that EC$_{50}$ values for the binding of LMOAME to membrane-bound antigens hCD19, hCD28 and hCD3e were about 0.15-0.17 µg/mL, about 0.27-0.32 µg/mL and about 5.1-7.5 µg/mL, respectively. The results also showed that there was no detectable binding activity between LMOAME and negative cell lines not expressing the target proteins.

Example 5. In vitro assessment of trispecific antibody-mediated effector cell activation

5.1 Assessment of the effect of LMOAME in inducing T cell activation

In this example, the human PBMC was used as an assessment system to test the ability of LMOAME antibody to mediate the activation of effector cells in an in vitro experiment. After different concentrations of LMOAME were co-incubated with PBMCs, the expression of T cell activation markers CD25, CD69 and GrB in PBMCs was detected, and thus the activation effect of LMOAME on T cells was observed.

Specifically, PBMCs from three healthy volunteers were extracted with Ficoll-Paque Plus (GE, Cat#: 17-1440-02) and added to a 96-well U-bottom cell culture plate at a density of $2 \times 10^5$ cells/well. The LMOAME purified in Example 2 and commercially available CD19xCD3 bispecific antibody (BiTE) Blinatumomab (Taizhou Biointron Biotechnology Co., Ltd., #20210330T001) were serially diluted with RPMI 1640 complete medium (Gibco, Cat#: C11875500CP): the final concentrations of LMOAME were set at 0.001, 0.01, 0.03, 0.1, 0.3, 1, 3, 10, and 100 ng/mL; and the final concentrations of Blinatumomab were set at 0.00065, 0.0065, 0.0195, 0.065, 0.195, 0.65, 1.95, 6.5, and 65 ng/mL. A vehicle control group of RPMI 1640 complete medium was used as a negative control. LMOAME and Blinatumomab were added to a 96-well U-bottom cell culture plate, and incubated with PBMCs at 37°C for 48 h or 120 h, respectively, and the cells were stained using detection antibodies after washing the plate with STB. Specifically, the following detection antibodies were used:

PerCP/Cyanine5.5 anti-human CD4 antibody: BioLegend, Cat#: 317428
FITC anti-human CD8 antibody: BioLegend, Cat#: 301006
PE anti-human CD25 antibody: BioLegend, Cat#: 356104
BV605 anti-human CD69 antibody: BioLegend, Cat#: 310938
APC anti-human/mouse Granzyme B antibody: BioLegend, Cat#: 372204.

Thermo Fisher Attune NxT flow cytometer was then used to detect the proportion and number of the following target cell subpopulations in each well of the 96-well plate: CD4$^+$, CD4$^+$CD25$^+$, CD4$^+$CD69$^+$, CD4$^+$Granzyme B$^+$, CD8$^+$, CD8$^+$CD25$^+$, CD8$^+$CD69$^+$, and CD8$^+$Granzyme B$^+$ cells. GraphPad Prism software was used for data statistics. If the one-way ANOVA analysis was statistically significant ($P < 0.05$), further comparative analysis was performed. Dunnett's Multiple Test was used to analyze the difference between LMOAME or Blinatumomab and the vehicle control; Tukey's Multiple Test was used to analyze the difference between LMOAME and Blinatumomab at the same molar concentration.

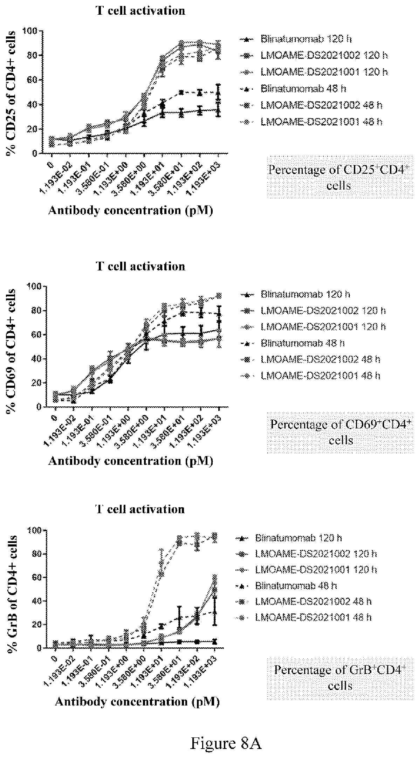

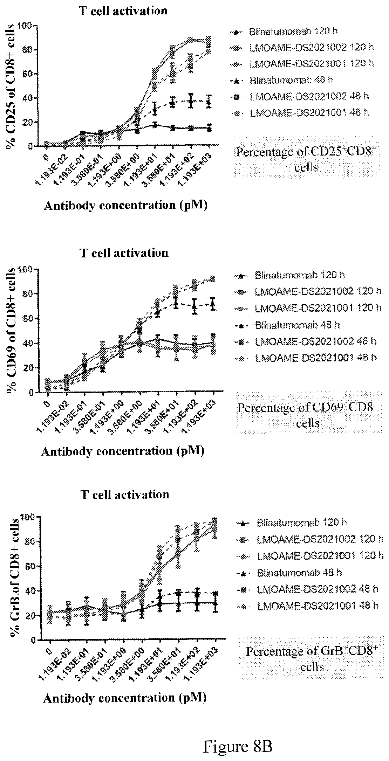

The results are shown in FIGS. 8A and 8B. Compared with the vehicle control, after LMOAME was co-incubated with PBMCs for 48 h or 120 h, the proportions of $CD4^+$ and $CD8^+$ cells with the expression of $CD25^+$, $CD69^+$ and $GrB^+$ were significantly increased, and presented a dose correlation with the concentration of LMOAME.

In addition, the results showed that after 48 h or 120 h of co-incubation with PBMCs, the proportions of $CD4^+$ and $CD8^+$ cell populations with the expression of $CD25^+$, $CD69^+$ and $GrB^+$ were higher in the case of LMOAME, compared with Blinatumomab.

In summary, after co-incubation with PBMCs, the trispecific antibody LMOAME provided by the present invention can significantly increase the expression of CD25 and CD69 on the surface of $CD4^+$ or $CD8^+$ effector cells, and significantly increase the expression of intracellular GrB in the effector cells, thus fully activating the effector cells in PBMCs. Meanwhile, the results also showed that the activation effect of the trispecific antibody LMOAME provided by the present invention on the effector cells in PBMCs was significantly higher than that of the Blinatumomab.

<u>5.2 Assessment of the effect of LMOAME, MLMOF and BLMOAM in inducing T cell activation (Jurkat cell system)</u>

The effector cell activation mediated by different trispecific antibodies were detected in an in vitro cell biology experiment. The trispecific antibodies to be detected include the <u>LMOAME and MLMOF</u> prepared in Examples 1 and 2 of the present invention, <u>as well as BLMOAM in the applicant's prior application. A vehicle control group of RPMI 1640 complete medium was used as a negative control.</u>

The specific process includes: (1) Jurkat cells (ATCC, TIB-152) were taken to be formulated into a cell suspension of $2.5 \times 10^6$ cells/mL with an experimental medium, and then inoculated into a 96-well cell culture plate in 40 $\mu$L per well; (2) the Nalm-6 cell strain stably transfected with GFP (Novoprotein) was taken to be formulated into a cell suspension of $2.5 \times 10^5$ cells/mL with an experimental medium, and then inoculated into the 96-well cell culture plate in 40 $\mu$L per well; (3) gradient-diluted test sample solutions were added in 20 $\mu$L per well respectively; (4) after mixing evenly, they were cultured at 37°C and 5% $CO_2$ for 24 h; (5) after washing the plate with 1% FBS-PBS, fluorescent dyes 7AAD (Biolegend, Cat# 420404) and Anti-CD69-APC (Biolegend, Cat #310910) were diluted, then added to the 96-well plate, mixed evenly, and incubated statically at 2°C - 8°C in the dark for 30 min; (6) after washing the plate with 1% FBS-PBS, 200 $\mu$L/well of 1% FBS-PBS was added and mixed evenly, and then the ratio of the APC positive cell population was detected using a flow cytometer. Data analysis was performed using GraphPad Prism software, and the 50% maximum effective concentration $EC_{50}$ value was used to represent the T cell activation activity of the different trispecific antibodies. The results showed that the $EC_{50}$ values of LMOAME, MLMOF and BLMOAM in inducing T cell activation were 0.624, 0.871 and 1.505 ng/mL, respectively. This result indicates that the two trispecific antibodies of the present invention can induce T cell activation at a lower dosage, which means a stronger T cell activation ability, than the previous trispecific antibody BLMOAM with different linker sequences. In addition, the T cell activation ability of LMOAME was slightly higher than that of MLMOF.

Example 6. In vitro assessment of trispecific antibody LMOAME-mediated effector cell proliferation

This example assessed the effect of LMOAME-mediated effector cell proliferation, in which the human PBMC was used as an assessment system, and after different concentrations of LMOAME were co-incubated with PBMCs, the proportion and number of $CD4^+$, $CD4^+CFSE_{dim}$, $CD8^+$ and $CD8^+CFSE_{dim}$ cells in PBMCs were detected, thereby investigating the effect of LMOAME in inducing T cell proliferation.

Specifically, PBMCs from three healthy volunteers were extracted with Ficoll-Paque Plus, incubated with 2 $\mu$M carboxyfluorescein diacetate succinimidyl ester (CFSE) at 37°C for 30 min, and then added to a 96-well U-bottom cell culture plate at a density of $2 \times 10^5$ cells/well. The LMOAME purified in Example 2 and the commercially available Blinatumomab were serially diluted with RPIM 1640 complete medium, respectively: the final concentrations of LMOAME were set at 0.001, 0.01, 0.03, 0.1, 0.3, 1, 3, 10, and 100 ng/mL; and the final concentration of Blinatumomab were set at 0.00065, 0.0065, 0.0195, 0.065, 0.195, 0.65, 1.95, 6.5, and 65 ng/mL. A vehicle control group of RPMI 1640 complete medium was used as a negative control. LMOAME and Blinatumomab were added to a 96-well U-bottom cell culture plate respectively, and incubated with PBMC at 37°C for 120 h, and then the cells were stained with Pacific Blue anti-human CD4 antibody (BioLegend, Cat#: 317429) and APC anti-human CD8 antibody (BioLegend, Cat#: 344722); and the proportion and number of $CD4^+$, $CD4^+CFSE_{dim}$, $CD8^+$ and $CD8^+CFSE_{dim}$ cells in PBMCs in each well were detected by Thermo Fisher Attune NxT flow cytometer.

The formulas for calculating the proliferation fold of each cell subpopulation are as follows:

$CD4^+$ cell proliferation fold = the number of $CD4^+$ cells in sample wells/the number of $CD4^+$ cells in vehicle control wells;

$CD4^+CFSE_{dim}$ cell proliferation fold = the number of $CD4^+CFSE_{dim}$ cells in sample wells/the number of $CD4^+CFSE_{dim}$ cells in vehicle control wells;

CD8$^+$ cell proliferation fold = the number of CD8$^+$ cells in sample wells/the number of CD8$^+$ cells in vehicle control wells;

CD8$^+$CFSE$_{dim}$ cell proliferation fold = the number of CD8$^+$CFSE$_{dim}$ cells in sample wells/the number of CD8$^+$CFSE$_{dim}$ cells in vehicle control wells.

In the formulas, the sample wells refer to wells in which LMOAME or Blinatumomab was co-incubated with PBMCs; and the vehicle control wells refer to wells in which vehicle control was co-incubated with PBMCs.

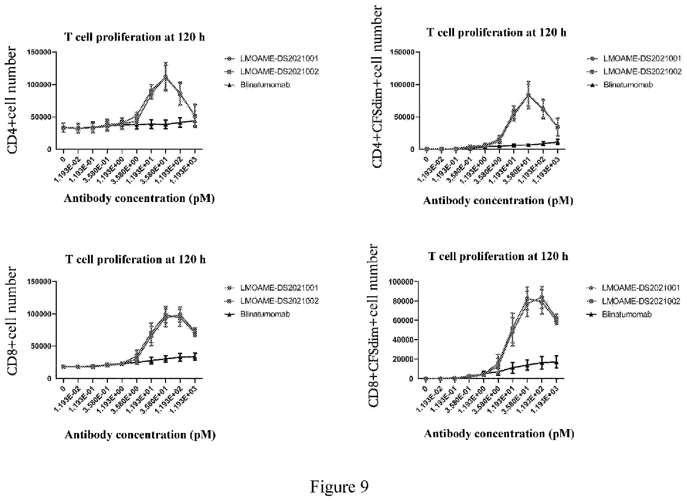

The measurement data was presented as the mean ± standard error, and the data statistics were carried out by GraphPad Prism software. If the one-way ANOVA analysis was statistically significant (P < 0.05), further comparative analysis was performed. The difference between LMOAME or Blinatumomab and vehicle control was analyzed using Dunnett's Multiple Comparison Test. The difference between LMOAME and Blinatumomab was analyzed using Tukey's Multiple Comparison Test. The results are shown in FIG. 9.

As shown in the figure, LMOAME can induce significant T cell proliferation, and the proliferation fold of T cells was dose-dependent with the concentration of added LMOAME.

The results also showed that under the same test conditions, the increased number of CD4$^+$ and CD8$^+$ T cells in the LMOAME group was higher than that in the Blinatumomab group at equivalent molar concentrations. The maximum proliferation folds of CD4$^+$, CD4$^+$CFSE$_{dim}$, CD8$^+$ and CD8$^+$CFSE$_{dim}$ cells by LMOAME were 3.58, 798.13, 5.37 and 8639.48, respectively, and the maximum proliferation folds of the above cells by equivalent molar concentrations of Blinatumomab were 1.35, 104.00, 1.84 and 1975.62, respectively. These results indicate that the trispecific antibody LMOAME of the present invention has a stronger ability to promote the proliferation of effector cells than the bispecific antibody Blinatumomab.

Example 7. In vitro assessment of the trispecific antibody LMOAME-mediated effector cell differentiation

This example assessed the ability of LMOAME to induce the differentiation of effector cells, in which the PBMC from three healthy volunteers was used as an assessment system, and after different concentrations of LMOAME were co-incubated with PBMCs, the proportion and number of T$_{CM}$, T$_{EM}$, T$_{Effector}$ and T$_{Naive}$ cell proportions in CD4$^+$ and CD8$^+$ T cells were detected, thereby investigating the function of LMOAME in inducing T cell differentiation.

Specifically, PBMCs from three healthy volunteers were extracted with Ficoll-Paque Plus and added to a 96-well U-bottom cell culture plate at a density of $2 \times 10^5$ cells/well. The LMOAME or Blinatumomab were serially diluted with RPIM 1640 complete medium: the final concentrations of LMOAME were set at 0.001, 0.01, 0.03, 0.1, 0.3, 1, 3, 10, and 100 ng/mL; the final concentrations of Blinatumomab were set at 0.00065, 0.0065, 0.0195, 0.065, 0.195, 0.65, 1.95, 6.5, and 65 ng/mL, and then these dilutions were added to the above 96-well U-bottom cell culture plate respectively, and incubated with PBMCs at 37°C for 48 h or 120 h. The cells were stained using a detecting antibody.

The antibodies for detecting cell phenotype used therein were as follows:

Pacific Blue anti-human CD4 antibody: BioLegend, Cat#: 317429
FITC anti-human CD8a antibody: BioLegend, Cat#: 301006
PE anti-human CD45RO antibody: BioLegend, Cat#: 304206
APC anti-human CCR7 antibody: BioLegend, Cat#: 353214

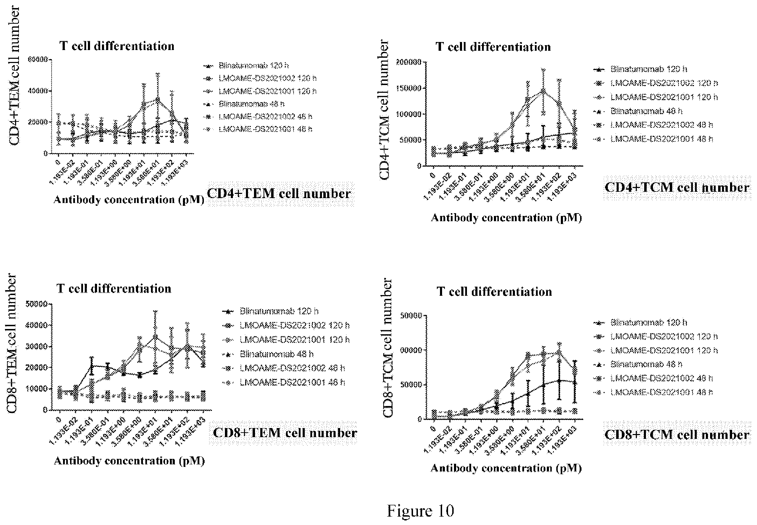

The proportion and number of target cell subpopulations were detected by Thermo Fisher Attune NxT flow cytometer. The results are shown in FIG. 10.

The results showed that after LMOAME was co-incubated with hPBMCs for 48 h, the number of CD4$^+$T$_{CM}$ significantly increased; the number of CD4$^+$T$_{CM}$ significantly increased in the LMOAME group compared with the Blinatumomab group (P < 0.05). After 120 h of co-incubation, LMOAME induced a significant increase in the number of CD4$^+$ and CD8$^+$ memory T cell T$_{CM}$ and T$_{EM}$ cell populations, and there was a relationship of "bell-shaped curve" between the number and the LMOAME added. Compared with Blinatumomab group, the ability of LMOAME to induce a differentiation into CD4$^+$ and CD8$^+$ memory T cell populations was much higher than that of Blinatumomab.

Example 8. In vitro assessment of the trispecific antibody LMOAME-mediated killing activity on target cells

This example assessed LMOAME-mediated killing activity on target cells, in which human PBMCs were used as effector cells, B cell lymphoma cell strains Raji-GFP, Ramos and Daudi, and precursor B-cell leukemia cell line Nalm-6 were used as target cells, and the proportion and number of GFP$^+$ or CD20$^+$ cells in the system were detected after co-incubating different concentrations of LMOAME with PBMCs and target cells, thereby assessing the LMOAME-mediated killing activity of effector cells in PBMCs on different target cells.

Specifically, in this example, PBMCs were extracted from 3 healthy volunteers with Ficoll-Paque Plus, and effector cells and target cells were added to a 96-well U-bottom cell culture plate at a ratio of 10 : 1 (PBMCs: $2 \times 10^5$ cells/well, target cells: $2 \times 10^4$ cells/well). LMOAME was serially diluted with RPIM 1640 complete medium: the final concentrations of LMOAME were set at 0.001, 0.01, 0.03, 0.1, 0.3, 1, 3, 10, and 100 ng/mL, and then added to the above 96-well U-bottom cell culture plate. After incubation at 37°C for 72 h, the plate was washed and stained with 7-AAD or FITC anti-human CD20 antibody (BD Pharmingen, Cat#: 555622), the proportion and number of GFP$^+$ or CD20$^+$ cells in each well were detected using Thermo Fisher Attune NxT flow cytometer, and the killing efficiency was calculated according to the formula below:

$$\text{Killing efficiency (\%)} = 100\% \times \left[1 - \frac{\text{Number of GFP}^+ \text{ or CD20}^+ \text{ cells in sample wells}}{\text{Number of GFP}^+ \text{ or CD20}^+ \text{cells in vehicle control wells}}\right]$$

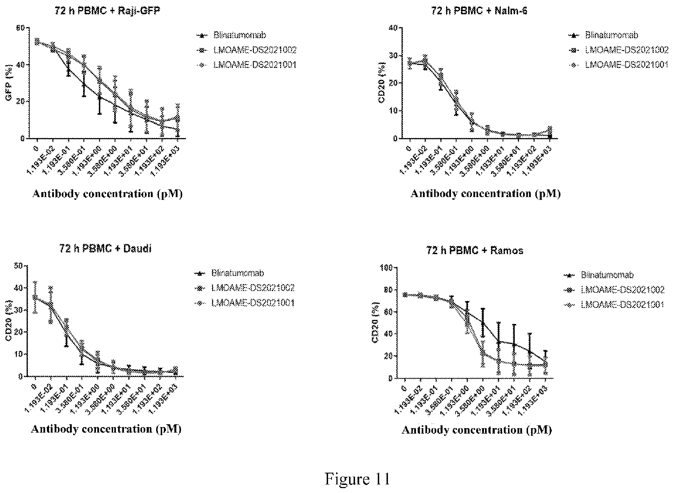

Data was analyzed by GraphPad Prism software, LMOAME-mediated PBMCs' killing activity on target cells was represented by the 50% maximum effective concentration EC$_{50}$ value. The results are shown in FIG. 11.

The results showed that LMOAME can dose-dependently mediate the killing of the different B cell lymphomas or B cell leukemia cell line by PBMCs (at an effector-to-target ratio of 10 : 1). Among them, LMOAME mediated the killing of the different target cells with EC$_{50}$ values of 1.738 - 2.071pM (Raji-GFP), about 0.265 - 0.286 pM (Nalm-6), 0.690 - 0.864 pM (Ramos) and 0.172 -0.173 pM (Daudi), respectively.

Example 9. In vitro detection of the trispecific antibody LMOAME-mediated hemolysis or coagulation reactions

In this example, different concentrations of LMOAME were incubated with 2% New Zealand rabbit red blood cell suspension by an in vitro test tube method, and the presence or absence of hemolysis or coagulation reaction was assessed by a naked-eye observation method. The 0.9% sodium chloride injection group was established in the test as a negative control, and the deionized water group as a positive control, and the observation results of LMOAME were compared with the control group.

Specifically, about 5 mL of blood was collected from the jugular vein or the ear central artery of male New Zealand rabbits, and red blood cells were extracted, and prepared into a 2% (v/v) suspension with 0.9% sodium chloride injection. 2.5 mL of red blood cell suspension and 0.1-0.5 mL of LMOAME purified in Example 2 (0.14 mg/mL) were added to each test group respectively, and the reaction system was adjusted to 5 mL with 0.9% sodium chloride injection. The test group and the control group were incubated at 37°C ± 1°C for 3 h, observed once every 15 min for the first 1 h, and then observed once every 1 h, and the colour of the upper-layer solution and the change of erythrocyte sedimentation in the test tube, as well as whether flocculent precipitate was produced, were photographed and recorded.

The results showed that LMOAME with a final concentration of 0.0028-0.014 mg/mL did not cause hemolysis reaction of rabbit red blood cells, manifested in that different degrees of erythrocyte sedimentation could be seen in the test tube and the upper-layer solution was colorless and transparent over the incubation time. At the same time, the results showed that no red-brown or brown-red flocculent precipitate was produced in the test tube, indicating that there was no agglutination reaction caused by the drug.

Example 10. Cancer suppressive effect of LMOAME in a subcutaneous tumor-bearing model having co-transplantation of Raji-luc/human PBMCs in NPG mice

In this example, the cancer suppressive effect of the trispecific antibody LMOAME was detected in a subcutaneous tumor-bearing model having co-transplantation of Raji-luc/human PBMCs in NPG mice.

The right flank of each female immunodeficient NPG mouse of about 21 grams (Beijing Vitalstar Animal Breeding Co., Ltd.) was co-inoculated subcutaneously with $5 \times 10^6$ Raji-luc cells and $2.5 \times 10^6$human PBMC cells. The Raji-luc cell lines used in this test were cultured in an incubator with 5% CO$_2$ at 37°C in RPMI-1640 medium with 10% FBS added. $5 \times 10^6$ cells were suspended in 50 μL PBS, which was mixed evenly with an equal volume of $2.5 \times 10^6$ PBMC cells, then mixed evenly with an equal volume of Matrigel, and the mixture was inoculated subcutaneously on the right dorsum of each mouse in an inoculation volume of 200 μL. Animal handling procedures were approved by the Institutional Animal Care and Use Committee (IACUC) of Pharmalegacy.

On day 6 after inoculation, 32 mice were selected and randomly divided into 4 groups (8 mice/group) according to the body weight and tumor volume, including vehicle control group (G1) and 20, 80, and 150 μg/kg dose groups (G2-G4) of LMOAME purified in Example 2.

Mice in each group were dosed intravenously twice a week (on the 1st and 4th days) for 3 weeks (defined as the dosing period from D1 to D21), with a total of 6 administrations. The body weight and tumor volume of mice were measured and recorded twice a week, and the following indices were calculated: tumor volume (V), tumor growth inhibition rate (TGI), relative tumor volume (RTV) and tumor inhibition rate by tumor weight (IR$_{TW}$%). The calculation method of each index is as follows:

$$V = (\text{length} \times \text{width}^2)/2$$

RTV = $V_t/V_0$, wherein Vt is the tumor volume measured at time point t, and $V_0$ is the tumor volume at the start of treatment.

TGI = (1-T/C) $\times$ 100%, wherein T and C are the RTVs at a given time point for the test group and vehicle control group, respectively.

$IR_{TW}$% = (average tumor weight of vehicle control group-average tumor weight of test group)/average tumor weight of vehicle control group $\times$ 100%.

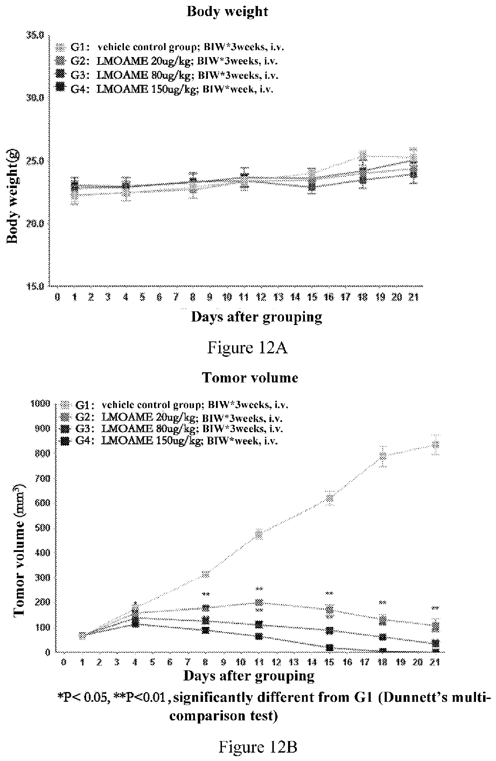

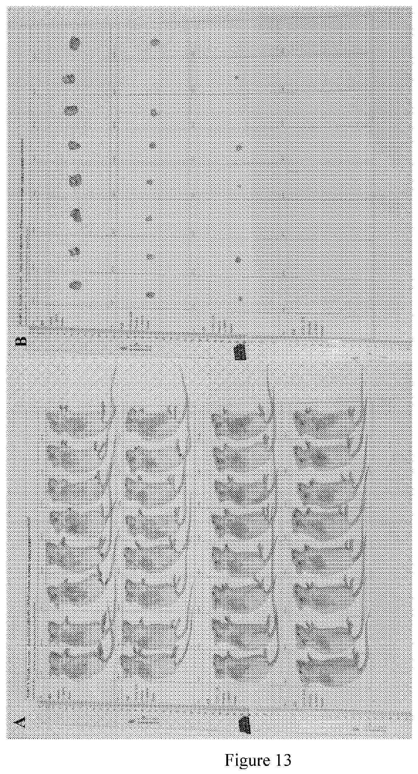

The results are shown in FIGS. 12A, 12B, and 13.

Body weight of mice

Compared with the vehicle control (G1), the weight of mice in the 20, 80 and 150 µg/kg dose groups (G2-G4) of LMOAME showed no significant difference from D1 to D21 (P > 0.05).

Tumor volume (V), relative tumor volume (RTV) and tumor growth inhibition rate (TGI)

On D8-D21, compared with the vehicle control (G1), the 20, 80, and 150 µg/kg dose groups (G2-G4) of LMOAME had significant tumor inhibition effect (P < 0.001), and the decrease in tumor volume was dose-dependent with the increase of LMOAME concentration. At the end of dosing, complete clearance of tumors was detected in all 8 mice in the 150 µg/kg dose group of LMOAME (G4).

At the endpoint of the study, the tumor was stripped, the tumor was weighed and the tumor inhibition rate by tumor weight (IRTW%) was calculated, and the results showed that the IRTW% values at the endpoint of the dosing of the G2-G4 groups were 87.21, 95.35 and 100, respectively.

Example 11. Cancer suppressive effect and toxicity evaluation of the trispecific antibody LMOAME in an Raji-luc subcutaneous tumor-bearing model of huHSC-NCG mice

In this example, the cancer suppressive effect and toxicity of the trispecific antibody LMOAME were detected in an Raji-luc subcutaneous tumor-bearing model of huHSC-NCG mice.

An huHSC-NCG mouse was obtained in a way of transplanting human hematopoietic stem cells (HSCs) by which a human immune system was re-established in a severely immunodeficient NCG mouse. Female huHSC-NCG mice were inoculated subcutaneously on the right flank with $5 \times 10^6$ Raji-luc cells. When the mean tumor volume reached 59.99 mm$^3$, 54 mice were selected and randomly divided into the following 9 groups (6/group) according to the flow cytometry data of hCD45% $\times$ hCD3% in peripheral blood of mice, body weight and tumor volume of mice: vehicle control group G1; Blinatumomab control groups G2-G3, the administrated dose of which was 100 µg/kg; and LMOAME dosing groups G4-G9, in which the administrated dose of G4 and G5 groups was 50 µg/kg, the administrated dose of G6 and G7 groups was 150 µg/kg, and the administrated dose of G8 and G9 groups was 500 µg/kg. Among them, the molar concentration of the Blinatumomab group with the administrated dose of 100 µg/kg was the same as that of the LMOAME group with the administrated dose of 150 µg/kg.

Mice in each group of G1, G2, G4, G6, and G8 groups were intravenously dosed twice a week (on the 1st and 4th days) for 4 weeks, with a total of 8 administrations. Mice in each group of G3, G5, G7, and G9 groups were intravenously dosed once a week for 4 weeks, with a total of 4 administrations.

The date of grouping was defined as D1 and the dosing period was D1-D28. The body weight and tumor volume of mice were measured and recorded twice a week, and the tumor growth inhibition rate by tumor volume ($TGI_{TV}$) was calculated according to the tumor volume determined. At the endpoint of the study, the tumor was stripped, the tumor was weighed and the tumor growth inhibition rate by tumor weight ($TGI_{TW}$) was calculated. The drug effect was represented by a tumor growth inhibition rate (TGI), including a tumor growth inhibition rate by tumor volume ($TGI_{TV}$) and a tumor growth inhibition rate by tumor weight ($TGI_{TW}$).

The calculation formula of $TGI_{TV}$ is as follows:

$$RTV_n = \frac{V_{nt}}{V_{n0}}$$

$$TGI_{TV} = \left(1 - \frac{meanRTV_{treat}}{meanRTV_{vehicle}}\right) \times 100\%$$

Vnt: the tumor volume of mice numbered n on day T;

Vn0: the tumor volume of mice numbered n on day 0;

RTVn: the relative tumor volume of mice numbered n on day T;

meanRTVtreat: the mean value of RTV of the dosing group;

meanRTVvehicle: the mean value of RTV of the vehicle control group.

The calculation formula of $TGI_{TW}$ is as follows:

$$TGI_{TW} = \left(1 - \frac{meanTW_{treat}}{meanTW_{vehicle}}\right) \times 100\%$$

meanTWtreat: the mean value of the tumor weight of the dosing group at the endpoint of the study;

meanTWvehicle: mean value of the tumor weight of the vehicle control group at the endpoint of the study.

The pharmacodynamics and toxicology of LMOAME were evaluated according to the clinical observation indices such as tumor growth inhibition rate, mouse body weight, 24 h food intake/water intake, and the like, as well as results of HE staining of main tissues and organs, human CD3 antigen IHC staining (for tumor tissues, spleen and brain tissues), and peripheral blood flow data analysis of mice. The animal handling procedures were approved by the Institutional Animal Care and Use Committee (IACUC) of GemPharmatech.

The changes in the tumor growth inhibition rate by tumor volume ($TGI_{TV}$) of each group during the dosing period are shown in Table 1. As can be seen from the data in Table 1, LMOAME can dose-dependently inhibited tumor growth, and mice dosed twice weekly exhibited higher tumor inhibition rates than mice dosed once weekly. In addition, the detection of the number of hCD19[+]and hCD20[+] cells in peripheral blood of mice by the flow cytometer indicated that different doses of LMOAME (50 μg/kg, 150 μg/kg and 500 μg/kg) and 100 μg/kg Blinatumomab were both able to exert the tumor inhibition effect by inhibiting hCD19[+]and hCD20[+]cell proliferation in this mouse tumor model.

Table 1. Changes in tumor growth inhibition rates by tumor volume ($TGI_{TV}$) in different groups during the dosing period (D1-D28)

| Group | Changes in tumor growth inhibition rates by tumor volume ($TGI_{TV}$) in different groups during the dosing period | | | | | | | | |
| --- | --- | --- | --- | --- | --- | --- | --- | --- | --- |
| | D1 | D4 | D8 | D11 | D15 | D18 | D22 | D25 | D28 |
| G1 | - | - | - | - | - | - | - | - | - |
| G2 | 0.00% | -4.05% | 32.07% | 44.59% | 53.71% | 56.23% | 54.90% | 53.64% | 52.68% |
| G3 | 0.00% | -7.10% | 8.11% | 28.77% | 24.48% | 37.09% | 33.70% | 25.42% | 17.60% |
| G4 | 0.00% | -23.85% | 8.89% | 42.53% | 51.29% | 57.56% | 59.33% | 64.09% | 61.60% |
| G5 | 0.00% | 1.41% | 12.17% | 20.05% | 17.66% | 27.39% | 25.63% | 24.56% | 11.69% |
| G6 | 0.00% | -8.65% | 26.35% | 49.60% | 58.15% | 62.17% | 62.90% | 64.44% | 63.48% |
| G7 | 0.00% | -43.50% | -29.10% | 12.54% | 3.18% | 34.96% | 39.18% | 42.85% | 42.39% |
| G8 | 0.00% | -6.49% | 41.56% | 71.17% | 87.43% | 94.57% | 96.86% | 97.74% | 98.43% |
| G9 | 0.00% | 2.11% | 9.09% | 42.37% | 55.46% | 66.78% | 74.67% | 77.76% | 77.86% |

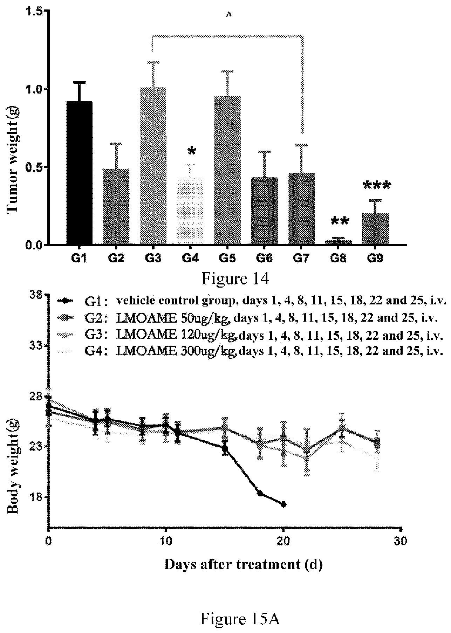

At the endpoint of the study, the tumor was stripped, the tumor was weighed and the tumor growth inhibition rate by tumor weight ($TGI_{TW}$) was calculated, and the results are shown in Table 2 and FIG. 14. In the case that the dosing frequency was changed from twice a week (BIW) to once a week (QW), the tumor inhibition rate of LMOAME in G7 group was higher than that in Blinatumomab (G3) group with the same molar concentration and dosing frequency.

Table 2. Endpoint tumor weight and tumor growth inhibition rate by tumor weight (TGI$_{TW}$) of each dosing group

| Group | Test samples | Endpoint tumor weight (g) | Tumor growth inhibition rate by tumor weight TGI$_{TW}$ | P value Vs G1 | P value Vs G2 | P value Vs G3 |
|---|---|---|---|---|---|---|
| G1 | Vehicle | 0.9434±0.1480 | - | - | - | - |
| G2 | Blinatumomab, 100 μg/kg, BIW | 0.4832±0.1639 | 48.78% | 0.071 | - | - |
| G3 | Blinatumomab, 100 μg/kg, QW | 1.0068±0.1611 | -6.72% | 0.782 | - | - |
| G4 | LMOAME DP, 50 μg/kg, BIW | 0.4212±0.0930 | 55.35% | 0.013* | - | - |
| G5 | LMOAME DP, 50 μg/kg, QW | 0.9460±0.1652 | -0.27% | 0.991 | - | - |
| G6 | LMOAME DP, 150 μg/kg, BIW | 0.4275±0.1709 | 54.69% | 0.053 | 0.819 | - |
| G7 | LMOAME DP, 150 μg/kg, QW | 0.4587±0.1806 | 51.38% | 0.074 | - | 0.047^ |
| G8 | LMOAME DP, 500 μg/kg, BIW | 0.0234±0.0195 | 97.52% | 0.003** | - | - |
| G9 | LMOAME DP, 500 μg/kg, QW | 0.1656±0.0780 | 82.45% | <0.001* ** | - | - |

Notes: the tumor weight was presented as the mean±SEM; and the tumor inhibition rates between different groups were statistically analyzed by independent sample T-test, vs G1 (*: P < 0.05, **: P < 0.01, ***: P < 0.001); G2 vs G6, and G3 vs G7 (^: P < 0.05).

During the dosing period, no abnormal finding was found in the ear temperature, food and water intake of mice in each dosing group. Compared with the vehicle control group, there was no significant difference in the body weight of mice in each dosing group. The spleen weights of mice in G2, G4, and G8 groups were significantly reduced, and since the spleen was rich in lymphocytes, and Blinatumomab and LMOAME had a significant B lymphocyte clearance effect, therefore, the reduction of spleen weight of mice in the dosing group was considered to be related to the pharmacological effect of the test sample. No drug-related pathological reaction was found in the histopathological study. IHC staining of hCD3 showed that hCD3 positive staining was mainly concentrated in spleen and tumor tissues, and no hCD3 positive staining was found in brain tissues.

Example 12. Cancer suppressive effect of the trispecific antibody LMOAME in an Nalm6-luc tail vein tumor-bearing model of huHSC-NCG mice

Female huHSC-NCG mice were inoculated with $1 \times 10^6$ Nalm6-luc cells through the tail vein. When the average number of photons reached $5.25 \times 10^4$ p/sec/cm$^2$/sr, 32 mice were randomly divided into 4 groups (8/group) according to the flow data of hCD45%×hCD3% cells in peripheral blood, the body weight of mice and the number of photons in tumors, including the vehicle control group (G1) and 50, 120 and 300 μg/kg dose groups (G2-G4) of LMOAME purified in Example 2.

Mice in each group were dosed intravenously twice a week (on the 1st and 4th days) for 4 weeks (defined as the dosing period from D1 to D28), with a total of 8 administrations. The body weight of mice and the number of photons in tumors were measured and recorded twice a week; the tumor growth inhibition rate (TGI) was calculated according to the measured number of photons, and the calculation formula of TGI is as follows:

$$RR_n = \frac{R_{nt}}{R_{n0}}$$

$$TGI = \left(1 - \frac{mean\ RR_{treat}}{mean\ RR_{vehicle}}\right) \times 100\%$$

Rnt: the number of photons in tumors of mice numbered n on day t;
Rn0: the number of photons in tumors of mice numbered n on day 0;
RRn: the relative number of photons in tumors of mice numbered n on day t;
meanRRtreat: the mean value of RR in the dosing group;

meanRTVvehicle: the mean value of RR in the vehicle control group.

The animal handling procedures were approved by the Institutional Animal Care and Use Committee (IACUC) of GemPharmatech.

12.1 Body weight of mice

As shown in FIG. 15A, the body weight of mice in each group was decreased, and tumor growth was considered to be the main cause of the weight loss.

12.2 Survival curve and death statistics

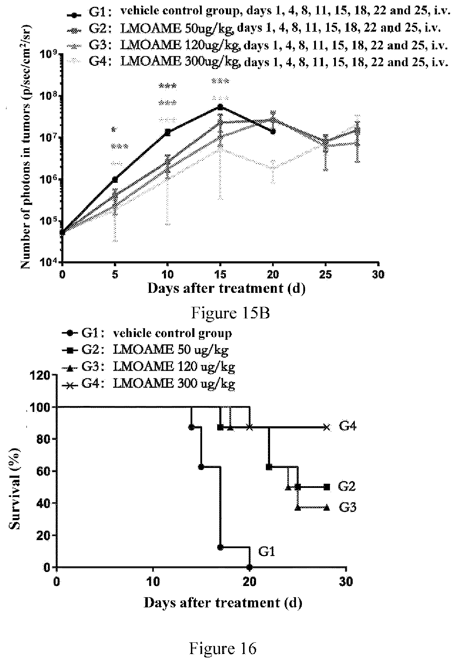

No mice were euthanized during the study. The survival of mice in the different groups is shown in Table 3 and FIG. 16. Mouse deaths began to occur in G1 group from D15, only one mouse survived on D20, and all the mice in G1 group died by D21. For G2-G4 groups, mouse deaths began to occur from D20, and by the endpoint of the study, the number of surviving mice in G2-G4 groups was 4, 3 and 7, respectively. Combined with the tumor fluorescence imaging data, the deaths of mice were considered to be caused by tumor burden. It can be seen that different doses of LMOAME can significantly prolong the average survival of mice, compared with the vehicle control (P < 0.001 ***).

Table3. Survival statistics (days) of mice in different groups

| Group | Median survival (days) | Mean survival (days) | *P* value | | |
|-------|------------------------|----------------------|-----------|-------|-------|
| | | | Vs G1 | Vs G2 | Vs G3 |
| G1 | 17.0 | 16.5±0.7 | - | - | - |
| G2 | 26.5 | 24.8±1.4 | < 0.001*** | - | - |
| G3 | 24.5 | 24.4±1.3 | < 0.001*** | 0.076 | - |
| G4 | N/A | 27.0±1.0 | < 0.001*** | 0.141 | 0.060 |
| Note: Data are presented as mean ± SEM. The test method was Log-rank (Mantel-Cox) test, ***: P < 0.001. N/A: more than half of the mice survived during the observation period, and median survival could not be confirmed. | | | | | |

12.3 The number of photons in tumors and tumor growth inhibition rate (TGI)

The average number of photons in tumors of mice in each group is shown in Table 4 and FIG. 15B, and the tumor growth inhibition rate TGI value is shown in Table 5.

Table4. Average number of photons in tumors (p/sec/cm2/sr) of mice in different groups during the study

| Group | Number of photons in tumors (p/sec/cm$^2$/sr) | | | | | | |
|-------|------|------|------|------|------|------|------|
| | D0 | D5 | D10 | D15 | D20 | D25 | D28 |
| G1 | 5.29E+04 ±5.05E+03 | 1.02E+06± 1.36E+05 | 1.34E+07± 2.09E+06 | 5.55E+07± 8.25E+06 | 1.39E+07 | - | - |
| G2 | 5.21E+04± 5.60E+03 | 4.07E+05± 1.64E+05 | 2.61E+06± 1.12E+06 | 2.30E+07± 1.26E+07 | 2.62E+07± 1.16E+07 | 8.14E+06± 3.53E+06 | 1.52E+07± 8.63E+06 |
| G3 | 5.20E+04± 5.25E+03 | 2.28E+05± 8.61E+04 | 1.77E+06± 7.21E+05 | 1.03E+07± 3.80E+06 | 2.90E+07± 1.29E+07 | 6.17E+06± 4.51E+06 | 7.47E+06± 4.87E+06 |
| G4 | 5.29E+04± 6.15E+03 | 1.85E+05± 1.53E+05 | 9.92E+05± 9.11E+05 | 5.29E+06± 4.95E+06 | 1.79E+06± 9.91E+05 | 7.33E+06± 3.22E+06 | 2.14E+07± 1.18E+07 |
| Note: Data are presented as mean ± SEM. "-" indicates no statistical data due to the death of mice. | | | | | | | |

Table 5. Tumor growth inhibition rates (TGI) of mice in different groups

| Group | D0 | D5 | D10 | D15 |
|-------|------|--------|--------|--------|
| G2 | 0.00% | 67.12% | 83.86% | 66.77% |

(continued)

| Group | D0 | D5 | D10 | D15 |
|---|---|---|---|---|
| G3 | 0.00% | 76.18% | 86.11% | 79.12% |
| G4 | 0.00% | 86.08% | 94.38% | 92.86% |

It can be seen from Table 4 and Table 5 that the tumor inhibition effect was observed on D5, D10, and D15, and the survival of mice was significantly prolonged in 50, 120, and 300 $\mu$g/kg dose groups (G2-G4) of LMOAME. The TGI values of G2-G4 groups were 67.12%, 76.18%, and 86.08%, respectively, on D5; 83.86%, 86.11%, and 94.38%, respectively, on D10; and 66.77%, 79.12%, and 92.86%, respectively, on D15.

12.4 Flow data analysis of human immune cells in peripheral blood of mice

The peripheral blood of mice was collected on D3 and D28 for flow analysis, and the proportion and number of mCD45, hCD45, hCD19, hCD20, hCD3, hCD4 and hCD8 cells therein were detected. The data showed that in 50, 120, 300 $\mu$g/kg dose groups (G2-G4) of LMOAME, the number of hCD19$^+$and hCD20$^+$ cells in the peripheral blood of mice was all significantly lower than the level on D3, demonstrating that LMOAME can target and eliminate human B cells.

Example 13. Phase I clinical study of LMOAME

This example describes a phase I clinical study involving the trispecific antibody of the present invention, in which the LMOAME trispecific antibody was prepared as a lyophilized powder injection, and tested for its safety, pharmacokinetics (PK) and immunogenicity in subjects with relapsed/refractory (r/r) B cell-associated tumors. The B cell-associated tumors specifically include B cell non-Hodgkin lymphoma (NHL) and B cell acute lymphoblastic leukemia (B-ALL) that express CD19. Patients must be those who have experienced disease progression after receiving approved or established therapies that are currently available and known to produce clinical benefits.

13.1 Enrolled population

Relapsed/refractory B-ALL and B cell lymphoma.

13.2 Objectives of the study

Primary objectives of the study:

To assess the safety of LMOAME and determine the recommended Phase-II dose (RP2D) of LMOAME in adult patients based on dose-limiting toxicity (DLT) assessment.

Secondary objectives of the study:

- To assess LMOAME drug-related toxicity, and the correlation between the toxicity and the dose and PK of LMOAME.
- To assess the pharmacokinetic (PK) parameters and immunogenicity of LMOAME in patients with CD19-expressing B cell malignancies.
- To determine safe, biologically active administration doses and treatment regimens for Phase 2 clinical trials.
- To assess any objective tumor response rate of LMOAME in B cell-associated tumors.

Exploratory objectives of the study:

- To investigate the relationship between PK parameters and B cell depletion, serum cytokine levels and CRS, and the correlation between PK parameters and clinical efficacy.
- To perform the immunophenotyping of peripheral blood, including activated CD4+ and CD8+ T cells, percentage of CD19, CD20, etc.
- To assess MRD status in ALL patients achieving CR/CRi using a multiparameter flow cytometer.

13.3 Regimen design

This study was a clinical phase I, multi-center, open-label, dose escalation design. The study was divided into two stages. The inclusion and sample size of patients in the first stage followed the regimen of Simon R, et al., and the second stage followed the traditional 3+3 dose escalation design.

Stage I: Accelerated titration design

The primary objective of stage I was to determine the level of the priming dose. Other objectives included safety, PK and immunogenicity.

A total of 5 dose groups were set as follows: 0.3 mcg, 0.6 mcg, 1.2 mcg, 2.4 mcg, and 4.8 mcg; one patient per dose group.

The dose-limiting toxicity (DLT) was assessed for 21 days.

In the first dosing cycle, dosing was performed once per week in week 1 and twice per week in weeks 2 and 3, with a DLT observation period of 21 days.

From the second dosing cycle, dosing was performed twice a week with a cycle length of 3 weeks and continued until occurrence of progressive disease (PD) or intolerance.

Accelerated dose escalation was performed for all 5 dose groups; if one DLT or adverse event (AE) of $\geq$ grade 2 occurred during the 21-day period, the dose group was expanded to 3 patients and the dose escalation was changed to a 3+3 design.

If a single cytokine release syndrome (CRS) of $\geq$ grade 3 or two CRSs of grade 2 occurred at a given dose level, a second priming dose, referred to as an "intermediate" priming dose, will be added in the first cycle of each dose level. The intermediate priming dose will be 1 dose level higher than the determined initial priming dose level. The DLT period will be extended to 28 days to include the priming dose regimen (one week for each of the initial priming dose and the intermediate priming dose) and a 2-week escalation dose level. The determination and optimization of the priming dose schedule (initial and intermediate doses) will be based on all available data, including safety, effectiveness, PK, PD (including cytokine data) and other data to be reviewed by the SMC. Pharmacodynamic (PD) analysis to determine the active biological dose will include B cell clearance, and determination of first CRS of grade 2 and clinical response.

If the first patient treated with 0.3 mcg developed cytokine release syndrome (CRS) of $\geq$ grade 3 or neurotoxicity, a 3+3 method would be used to treat a group of patients at 0.15 mcg. If no CRS of $\geq$ grade 3 was seen at 0.15 mcg or the maximum tolerated dose (MTD) was not reached, the dose was escalated to 0.3 mcg.

If no individual case with DLT or AEs of $\geq$ grade 2 was reported (extrinsic causes or disease progression were excluded), patients at the next dose level will be enrolled after day 21 of the previous dose group. If a DLT or AE occurs at any time during the accelerated portion of the study, the study will recover to the 3+3 design.

Stage II: Traditional 3+3 dose escalation design

The primary objectives of stage II were to study safety, PK, immunogenicity and preliminary efficacy, and to determine the recommended phase II dose (RP2D).

A total of 5 dose groups were set as follows: 9.6 mcg, 16 mcg, 24 mcg, 34 mcg, 45 mcg; 3 patients per dose group.

In the first dosing cycle, dosing was performed once per week in week 1 and twice per week in weeks 2 and 3, with a DLT observation period of 21 days.

From the second dosing cycle, dosing was performed twice a week with a cycle length of 3 weeks and continued until occurrence of PD or intolerance.

Patients will be admitted to hospital for observation within the first 72 hours after enrollment to receive initial dose, intermediate dose (if applicable), and escalation dose. The first dose (Cycle 1 Day 1, C1D1) will be slowly infused over 3 hours. If there is no infusion-related reaction (IRR) of $\geq$ grade 2, the next dose (Cycle 1 Day 8, C1D8) can be dosed within 2 hours, and if there is no IRR, the third and subsequent doses can be dosed within 1 hour.

Premedication with antihistamine and acetaminophen 1 hour before the first 3 doses of LMOAME (e.g., before the initial dose of C1D1, before the first 2 BIW doses of C1D8 and C1D11) was required. Dexamethasone may be added for subsequent infusions according to clinical indications. The dose of subsequent premedication with dexamethasone may be reduced or canceled at the discretion of the investigators. Patients will be monitored after infusion for a period equivalent to twice the infusion time. If CRS is present on the first 3 doses, a premedication of 20 mg of dexamethasone, antihistamine, and acetaminophen should be given within 1 hour before subsequent dosing. If two CRS events of $\geq$ grade 2 or one CRS event of $\geq$ grade 3 occur during the study, all patients will be prophylactically treated with 650 mg oral acetaminophen, 50 mg diphenhydramine (through oral administration or intravenous injection), and 8 mg/kg tocilizumab (through intravenous infusion within over 1 hour), followed by giving the first escalation dose.

On Day 8 of Cycle 1, patients will receive the first dose at the escalation dose level according to the BIW schedule for 2 weeks. The DLT period was 3 weeks (1 week for the priming dose, and 2 weeks for the escalation dose). If a single CRS event of ≥ grade 3 or two CRS events of grade 2 occurred at a given dose level, a second intermediate priming dose will be added in the first cycle of each dose level, which was the same as in Part I (see above). The escalation dose was increased after all patients at the previous dose levels had passed the DLT assessment period and the MTD was not exceeded. For Cycle 2 and beyond, patients will be dosed BIW starting on Day 1. The length of these cycles was 3 weeks.

Patients will continue to receive treatment until occurrence of disease progression, DLT, or intolerable toxicity, withdrawal from treatment, or revocation of informed consent, or it can be determined at the discretion of the investigators in the best interests of the patient.

RP2D was determined after reviewing all safety, PK, PD and preliminary efficacy data. The dose level at the putative RP2D was then expanded to 6-10 NHL patients, and 6-10 B-ALL patients.

13.4 Number of people to be included

Stage I

| Dose group | Increment percentage (%) | Administrated dose (mcg) | Number of patients |
|---|---|---|---|
| 1-1 |  | 0.3 | 1-6 |
| 1-2 | 100 | 0.6 | 1-6 |
| 1-3 | 100 | 1.2 | 1-6 |
| 1-4 | 100 | 2.4 | 1-6 |
| 1-5 | 100 | 4.8 | 1-6 |

Stage II

| Dose group | Increment percentage (%) | Administrated dose (mcg) | Number of patients |
|---|---|---|---|
| 2-1 | 100 | 9.6 | 3-6 |
| 2-2 | 67 | 16 | 3-6 |
| 2-3 | 50 | 24 | 3-6 |
| 2-4 | 42 | 34 | 3-6 |
| 2-5 | 32 | 45 | 3-6 |

Once the maximum tolerated dose (MTD) has been determined, additional 6-10 persons will be included in the NHL and B-ALL population for an extension stage study to further determine the safety and effectiveness at this MTD dose.

13.5 Dosing mode

Once reconstituted, the lyophilized powder of LMOAME was administered by intravenous infusion at the dose and frequency described in the section 13.3 above.

13.6 Enrollment criteria

Male or female patients aged 18 years and older who meet the following criteria will be enrolled in the study:

- One disease of the following groups of histologically confirmed B cell malignancies.

Lymphocytes or lymphoma cells must be positive for CD19 antigen expression as determined by immunohistochemistry and/or flow cytometry; if the patient has received the prior treatment targeting CD19, CD19 expression must be determined after the treatment.

Non-Hodgkin Lymphoma (NHL):

Invasive B cell NHL including:

- Diffuse large B-cell lymphoma (DLBCL), NOS
- Primary mediastinal large B cell lymphoma
- High-grade B cell lymphoma with MYC and BCL2 and/or BCL6 rearrangements
- High-grade B cell lymphoma, NOS
- High-grade B cell lymphoma caused by indolent NHL
- Mantle cell lymphoma (which can be manifested as an indolent or aggressive disease)
- Follicular lymphoma, grade 3B

Indolent B cell NHL including:

- Follicular lymphoma (grades 1-3A)
- Splenic marginal zone lymphoma
- Extranodal marginal zone lymphoma of mucosa-associated lymphoid tissue (MALT lymphoma, excluding gastric MALT lymphoma)

Other requirements for enrollment of patients with NHL include:

- Patients with NHL entering the stage II study must have $\geq$ 1 measurable target lesion as defined by the Lugano criteria (SAD for nodular lesions should be $\geq$ 15 mm; LD for extranodal lesions should be $\geq$ 10 mm).
- Patients with indolent lymphoma will be included in the scope of treatment only if they meet the criteria for systemic therapy.
- Patients with rapidly progressing high-grade NHL will be included in the study scope only after the biologically active dose level is reached.

Aggressive NHL: the patients must have received prior treatment with rituximab, cyclophosphamide, doxorubicin, vincristine, and prednisone (R-CHOP, R-EPOCH, or an equivalent anti-CD20 agent-containing therapy), have received $\geq$ 2 lines of systemic therapies, and have received or cannot tolerate all other standard therapies considered to be able to provide clinical benefits.

Indolent NHL: the patients have been refractory or relapsed after having received $\geq$ 2 previous systemic therapies, including the treatment with rituximab or other anti-CD20 monoclonal antibodies alone or in combination with chemotherapy (such as bendamustine, CHOP, and CVP), and have received or cannot tolerate all other standard therapies considered to provide clinical benefit.

B cell acute lymphoblastic leukemia (B-ALL):

- Philadelphia chromosome-positive or -negative B cell leukemia that is refractory or relapsed after the treatments with a first-line therapy (induction/consolidation/maintenance), a salvage therapy (such as blinatumomab, inotuzumabozogamycin, and chemotherapy), and all available tyrosine kinase inhibitors for Bcr-Abl.
- The patients have received or cannot tolerate all other standard therapies known to provide clinical benefits.
- Relapsed or refractory CD19+ B cell ALL, for which all available therapies with known clinical efficacy are ineffective.
- No active acute or chronic graft-versus-host disease for 2 months prior to enrollment and no received immunosuppressive therapy at enrollment. The peripheral blood cell count must be < 10000/mm3 at the first dose. Prednisone and/or hydroxyurea may be used to maintain this value during screening. Patients with B ALL will be included in the dose escalation portion of the study only after the dose level has reached the biologically active dose level. The biologically active dose level is determined based on the dose level when first CRS of $\geq$ grade 2 and peripheral B cell depletion occur.

Both NHL and ALL patients should meet the following criteria:

- ECOG performance status of 0-2, life expectancy greater than 3 months.
- During the screening period, clinical biochemical indices meet the following requirements:

- Total bilirubin must be < 1.5 times the upper limit of the normal range (ULN). If the increase in total bilirubin can be reasonably attributed to the presence of metastatic diseases in the liver, or for patients with documented Gilbert syndrome (unconjugated hyperbilirubinemia), the total bilirubin can be elevated to 3xULN.
- Alanine aminotransferase (ALT) or aspartate aminotransferase (AST) must be $<3 \times$ ULN. If the increase in AST and ALT can reasonably be attributed to the presence of metastatic diseases in the liver, then AST and ALT can be elevated to 5xULN.
- Calculated creatinine clearance > 50 mL/min (Cockcroft-Gault formula)
- Hemoglobin must be $\geq$ 8 g/dL.
- Neutrophil count must be > 1,500/mm$^3$. For ALL, there is no criterion for neutrophil count if the reduction of neutrophils is thought to be due to BM involvement by leukemic cells.
- The platelet count must be greater than 75,000/mm$^3$. For patients with ALL, there is no criterion for platelet count if the reduction of platelet is thought to be due to BM involvement by leukemic cells; platelets may be transfused as needed, at the discretion of the investigators.
- All patients must have a peripheral blood cell count $\leq$ 10,000/mm$^3$ before the first dose of LMOAME. This can be achieved by taking dexamethasone at 20-40 mg/m$^2$ for 4 consecutive days. The dosage of hydroxyurea can also be used to control the white blood cell count. If these measures cannot reduce the white blood cell count to $\leq$ 10,000/mm$^3$, the patient will not be enrolled.
- The prothrombin time-international normalized ratio (PT-INR) must be $\leq$ 1.5.

- If the patient is female, she should have a negative urine pregnancy test, or be postmenopausal for at least 1 year before screening.
- Female patients of childbearing potential or male patients with a partner of childbearing potential must use one or more contraceptive methods starting from screening, and this should be continued during study treatment until 3 months after the last medication. Contraceptive methods include:

   a. Complete abstinence (if consistent with the usual lifestyle preferred by the patient). Periodic abstinence (e.g., the calendar method, ovulation period method, sympathetic method, and post-ovulation method) and withdrawal are unacceptable contraceptive methods.
   b. Female sterilization surgeries (bilateral salpingectomy with or without hysterectomy) or tubal ligation performed at least 12 weeks prior to study treatment.
   c. Male sterilization (for at least 6 months prior to screening). For a female patient participating in this study, whose male partner who underwent vasectomy should be her only partner.
   d. Use of an oral, injectable, or implantable hormonal contraception method; an implantable intrauterine contraceptive device (IUD) or an intrauterine contraceptive system (IUS); use of other hormonal contraceptive methods with comparable efficacy (failure rate <1%), such as a hormonal vaginal ring or transdermal hormonal contraception.
   e. If a female patient is taking oral contraceptives, she should have taken the same drug steadily for at least 3 months before taking the study treatment.

- The patients should be able to understand and willing to provide written informed consent, and follow scheduled visit and study procedures.

### 13.7 Exclusion criteria

- Patients who have received systemic anti-cancer treatment (including I/O therapy) within 4 weeks prior to initiation of LMOAME treatment.
- Patients who have received radiotherapy within 2 weeks prior to study entry.
- Patients who have received CAR-T or transplantation treatment within 3 months prior to study entry.
- Occurrence of severe infection within 14 days prior to study entry which requires treatment with antibiotics.
- Patients who have previously received an anti-CD19 targeting therapy should be enrolled in this study only if their tumor cells are shown to still express CD19 after completion of the CD19-targeted therapy.
- Patients with brain metastases or other major neurological diseases, except for patients with brain metastases who are asymptomatic and radiologically stable within 2 weeks prior to the administration of the first dose of LMOAME and do not require the use of steroids. Patients with ALL must have no obvious CNS disease.
- Treatment with corticosteroids (> 10 mg prednisone or an equivalent per day) or immunosuppressive drugs $\leq$ 7 days prior to the first dose of LMOAME, with the following exceptions:

- Topical, ophthalmic, intra-articular, intranasal, or inhaled corticosteroid drugs
- Use of dexamethasone to reduce peripheral blood cell counts in ALL

- Treatment with any investigational product (including cell or gene therapies) within 5 half-lives of the agent or within 4 weeks before starting to use LMOAME (whichever is shorter).
- Current autoimmune diseases or a history of a autoimmune disease with underlying central nervous system involvement.
- Hypersensitivity to any of the ingredients (including polysorbate 80) included in the formulation of the drug product.
- Received illicit drugs, substance abuse or alcohol abuse or evidence of these.
- Occurrence of any cerebrovascular accident (CVA), transient ischemic attack (TIA), myocardial infarction (MI), unstable angina pectoris or Class III or IV heart failure as defined by New York Heart Association (NYHA) within 6 months prior to enrollment; uncontrolled arrhythmias within < 3 months after enrollment.
- Major surgery within 4 weeks or minor surgery within 2 weeks prior to screening; wound must be completely healed (minor surgery such as catheter placement does not belong to the exclusion criteria).
- A history of grades 3-4 allergic reactions to other monoclonal antibody treatments, or known hypersensitivity to protein drugs or recombinant proteins or excipients in the LMOAME drug preparation.
- Presence of congenital long QT syndrome, QTcF > 480 ms, use of a cardiac pacemaker, left ventricular ejection fraction (LVEF) < 50%, clinically significant arrhythmias requiring intervention, cardiac troponin I or T > 2.0 ULN, poorly controlled diabetes (HbA1c > 9%), or hypertension (systolic blood pressure > 160 mmHg or diastolic blood pressure > 100 mmHg).
- Any other serious underlying disease (such as active gastric ulcer, uncontrolled seizures, cerebrovascular events, gastrointestinal bleeding, severe symptoms and signs of coagulation and coagulation disorders, cardiac conditions), psychiatric, psychological, familial, or geographic conditions that, at the discretion of the investigators, may interfere with planned staging, treatment, and follow-up, affect patient compliance, or place the patient at high risk for treatment-related complications.
- Patients who were infected with COVID-19, have been quarantined and have tested negative for the virus can participate.
- Patients with other concurrent malignancies within 5 years prior to enrollment, except for adequately treated cervical carcinoma in situ, localized cutaneous squamous cell carcinoma, basal cell carcinoma, localized prostate cancer, ductal carcinoma in situ of the breast, or urothelial carcinoma at < T1.
- Previous organ or allogeneic stem cell/bone marrow transplantation. Patients who have received an autologous stem cell transplantation > 6 months prior to study screening are eligible if their hematologic parameters meet the enrollment requirements.
- A history of grades 3-4 immune-related adverse events (irAEs) or irAEs requiring discontinuation of prior treatment, except for grade 3 endocrinopathy managed with hormone replacement therapy.
- Pleural effusion, pericardial effusion or ascites requiring frequent drainage or medical intervention
- Live virus vaccination within 4 weeks prior to study enrollment.
- Active hepatitis B or C; HBV carriers without an active disease (HBV DNA titer < 1000 cps/mL or 200 IU/mL), or patients with cured hepatitis C (negative HCV RNA test) can be included.
- Known HIV infection.

13.8 Endpoints of the study

Safety endpoints of the study

- The proportion of patients with serious adverse reactions (SAEs) and adverse reactions (AEs) at each dose level;
- The correlation of DLT type, frequency and doses;
- Unexpected clinical or laboratory findings.

PK study endpoints:

PK parameters, including AUC, $C_{max}$, $C_{min}$, initial clearance rate, volume of distribution and elimination half-life

PD study endpoints:

B cell clearance, cytokine release (including IL-2, IL-6, IFNg, and TNFa)
Immunogenicity endpoints of the study:
Proportion of patients with anti-drug antibodies (ADA), neutralizing antibodies.

Efficacy endpoint:

Objective response rate (ORR)
Sequence information

Table 6. Linker sequences

| SEQ ID NO: | Designation | Description | Sequence |
|---|---|---|---|
| 1 | Linker 1-1 | The first linker sequence | GGGGSGGGGSGGGGS |
| 2 | Linker 1-2 | The first linker sequence | GGGGS |
| 3 | Linker 1-3 | The first linker sequence | GGSGGSGGSGGSGG |
| 4 | Linker 2-1 | A long linker containing a C→S mutation | ASKSKKEIFRWPESPKAQA**S**SVPTAQPQAEGSLAKA **TT**APA**TT**RNTGRGGEEKKKEKEKEEQEERETKTPESP SHTQPLGV (Boxed portions are glycosylation sites, and the C→S mutation is underlined) |
| 5 | Linker 2-2 | A short linker without glyco-sylation sites | RNTGRGGEEKKKEKEKEEQEERETKTPE_C_PSHTQPLG V (Cysteine C is underlined) |
| 6 | Linker 2-3 | A short linker without glycosylation sites and containing a C→S mutation | RNTGRGGEEKKKEKEKEEQEERETKTPE_S_PSHTQPLG V (the C→S mutation is underlined) |
| 7 | Linker 2-4 | The repeat unit in the sec-ond linker sequence | EAAAK |

(continued)

| SEQ ID NO: | Designation | Description | Sequence |
|---|---|---|---|
| 8 | Linker 2-5 | A long linker containing a C→G mutation | ASKSKKEIFRWPESPKAQA**S**SVPTAQPQAEGSLAKA TTAPATTRNTGRGGEEKKKEKEKEEQEERETKTPE<u>G</u> PSHTQPLGV (boxed portions are glycosylation sites, and the C→G mutation is underlined) |
| 9 | Linker 2-6 | A short linker without glycosylation sites and containing a C→G mutation | RNTGRGGEEKKKEKEKEEQEERETKTPE<u>G</u>PSHTQPL GV (the C→G mutation is underlined) |
| 10 | Linker 2-0 | The linker of the prior application | ASKSKKEIFRWPESPKAQA**S**SVPTAQPQAEGSLAKA TTAPATTRNTGRGGEEKKKEKEKEEQEERETKTPE<u>C</u>P SHTQPLGV |

Table 7. Sequences of the trispecific antibody LMOAME

| SEQ ID NO | Description | Sequence |
|---|---|---|
| **First domain, CD19 binding domain** | | |
| 11 | The amino acid sequence of heavy chain CDR1 | SYWMN |
| 12 | The amino acid sequence of heavy chain CDR2 | QIWPGDGDTNYNGKFKG |
| 13 | The amino acid sequence of heavy chain CDR3 | RETTTVGRYYYAMDY |
| 14 | The amino acid sequence of light chain CDR1 | KASQSVDYDGDSYLN |
| 15 | The amino acid sequence of light chain CDR2 | DASNLVS |
| 16 | The amino acid sequence of light chain CDR3 | QQSTEDPWT |
| 17 | The amino acid sequence of the heavy chain variable region | QVQLQQSGAELVRPGSSVKISCKASGYAFS**SYWMN**WVKQRPGQGLEWIG**QIWPGDGDTNYNGKFKG**KATLTADESS STAYMQLSSLASEDSAVYFCAR**RETTTVGRYYYAMDY**WGQGTTVTVSS |
| 18 | Optimized coding sequence for the heavy chain variable region | caggtgcagctccagcagtctggggctgagctggtgcggcccggcagcagcgtcaagatttcctgtaaagcatctggatatgcctttctagttactgga tgaattgggtgaagcagagaccggccaggagggactggagtggattggacagatttggcctggggatggtgacaccacacaaggcaagttcaaagg caaggctaccctgacagcagagacgaatcaagctccacagcttacatgcagcttgtctagtctggcatcagaggatagcgccgtgtattttgcgctcggag ggaaccacaactgtcggcgcctactattacgctatgggctactgggctactgggcccaggggaccacagtgacagtctcaagc |
| 19 | The amino acid sequence of the light chain variable region | DIQLTQSPASLAVSLGQRATISC**KASQSVDYDGDSYLN**WYQQIPGQPPKLLIY**DASNLVS**GIPPRFSGSGSGTDFTLNIHP VEKVDAATYHC**QQSTEDPWT**FGGGTKLEIK |
| 20 | Optimized coding sequence for the light chain variable region | gacatccagctgacacagtctccagctagtctggcagtgagcctgggacagagagctactatttcctgcaaggcaagcagtccgtggactacgatgg agactcctatctgaactggtatcagcagatcccggccagccccttaaactgctgatttatgatgcttcaaatctggttgagcggcatcccaccccgcttct ctggaagtgggtcaggcaccgatttacactgaacattcaccccgtggagaaggtggacgccgcctaccaccattgccagcagtccactgaggaccct ggaccttcggccggcaccaagctgaaatcaaa |

(continued)

| SEQ ID NO | Description | Sequence |
|---|---|---|
| **First domain, CD19 binding domain** | | |
| 21 | The amino acid sequence of scFv | DIQLTQSPASLAVSLGQRATISC**KASQSVDYDGDSYLN**WYQQIPGQPPKLLIY**DASNLVS**GIPPRFSGSGSGTDFTLNIHP VEKVDAATYHC**QQSTEDPWT**FGGGTKLEIKGGGGSGGGGSGGGGSQVQLQQSGAELVRPGSSVKISCKASGYAFS**SY WMN**WVKQRPGQGLEWIG**QIWPGDGDTNYNGKFKG**KATLTADESSSTAYMQLSSLASEDSAVYFCAR**RETTTVGRY YYAMDY**WGQGTTVTVSS |
| **Second domain, CD3 binding domain** | | |
| 22 | The amino acid sequence of heavy chain CDR1 | RYTMH |
| 23 | The amino acid sequence of heavy chain CDR2 | YINPSRGYTNYNQKFKD |
| 24 | The amino acid sequence of heavy chain CDR3 | YYDDHYCLDY |
| 25 | The amino acid sequence of light chain CDR1 | RASSSVSYM N |
| 26 | The amino acid sequence of light chain CDR2 | DTSKVAS |
| 27 | The amino acid sequence of light chain CDR3 | QQWSSNPLT |
| 28 | The amino acid sequence of the heavy chain variable region | DIKLQQSGAELARPGASVKMSCKTSGYTFT**RYTMH**WVKQRPGQGLEWIG**YINPSRGYTNYNQKFKD**KATLTTDKSSST AYMQLSSLTSEDSAVYYCAR**YYDDHYCLDY**WGQGTTLTVSSVE |
| 29 | Optimized coding sequence for the heavy chain variable region | gacatcaagctccagcagtctggtgccgagctggctcgaccaggagccagtgtgaagatgtcatgtaaaaccagtggctatactttcaccagatacaca atgcactgggtgaaacagcgcccagggcagggtctggaatggatcgggtacattaaccccagccggggctacaccaactacaaccagaagttcaagg ataaagccactctgactaccgacaagtcctctagtaccgcttatatgcagctgtcaagcctgacatccgaggacagcgccgtgtactactgcgccaggt attacgacgatcattattgtctggattactggggccagggaacaactctgaccgtgagcagcgtcgaa |
| 30 | The amino acid sequence of the light chain variable region | VDDIQLTQSPAIMSASPGEKVTMTC**RASSSVSYMN**WYQQKSGTSPKRWIY**DTSKVAS**GVPYRFSGSGSGTSYSLTISSM EAEDAATYYC**QQWSSNPLT**FGAGTKLELK |

42

EP 4 556 486 A1

| Second domain, CD3 binding domain | | |
|---|---|---|
| 31 | Optimized coding sequence for the light chain variable region | gtggacgacatccagctgacccagtctcctgccattatgagcgccagccccggcgagaaggtgacaatgacttgccgagctagttcaagcgtcagctat atgaattggtatcagcagaaatctggcaccagccctaaacgatggatctatgacacctctaaagtggcaagcggagtcccataccggttctctgggagt ggttcaggcactagctattccctgaccatttcctctatggaggcagaagatgcagccacctattactgtcagcagtggagttcaaatcccctgacatttgg agccgggactaagctggagctgaaa |
| 32 | The amino acid sequence of scFv | DIKLQQSGAELARPGASVKMSCKTSGYTFT**RYTMH**WVKQRPGQGLEWIG**YINPSRGYTNYNQKFKD**KATLTTDKSSST AYMQLSSLTSEDSAVYYCAR**YYDDHYCLDY**WGQGTTLTVSSVEGGSGGSGGSGGSGGVDDIQLTQSPAIMSASPGEKV TMTC**RASSSVSYMN**WYQQKSGTSPKRWIY**DTSKVAS**GVPYRFSGSGSGTSYSLTISSMEAEDAATYYC**QQWSSNPLT**F GAGTKLELK |
| The third domain, CD28 binding domain | | |
| 33 | The amino acid sequence of heavy chain CDR1 | SYYIH |
| 34 | The amino acid sequence of heavy chain CDR2 | CIYPGNVNTNYNEKFKD |
| 35 | The amino acid sequence of heavy chain CDR3 | SHYGLDWNFDV |
| 36 | The amino acid sequence of light chain CDR1 | HASQNIYVWLN |
| 37 | The amino acid sequence of light chain CDR2 | KASNLHT |
| 38 | The amino acid sequence of light chain CDR3 | QQGQTYPYT |
| 39 | The amino acid sequence of the heavy chain variable region | QVQLVQSGAEVKKPGASVKVSCKASGYTFT**SYYIH**WVRQAPGQGLEWIG**CIYPGNVNTNYNEKFKD**RATLTVDTSISTA YMELSRLRSDDTAVYFCTR**SHYGLDWNFDV**WGQGTTVTVSS |
| 40 | Optimized coding sequence for the heavy chain variable region | caggtgcagctggtccagtcaggagcagaagtcaaaaaacccggcgcatcagtcaaagtctcttgtaaagcctccggatacacatttacatcctactat atccactgggtcagacaggcacctggccagggactggagtggatcggctgcatttacccaggaaacgtgaacaccaactacaacgaaaagttcaagg accgcgctactctgaccgtcgatacatcaattagcactgcatacatggagctgtctcggctgaggagtgacgatacagctgtgtacttctgtactcgatct cattatggcctggactggaactttgatgtgtgggggcagggcaccacagtgaccgtcagctcc |

| The third domain, CD28 binding domain | | |
|---|---|---|
| 41 | The amino acid sequence of the light chain variable region | DIQMTQSPSSLSASVGDRVTITC**HASQNIYVWLN**WYQQKPGKAPKLLIY**KASNLHT**GVPSRFSGSGSGTDFTLTISSLQP EDFATYYC**QQGQTYPYT**FGGGTKVEIKR |
| 42 | Optimized coding sequence for the light chain variable region | gacatccagatgacacagagcccttcaagcctgagcgcctccgtcggtgacagagtgaccattacctgccacgcctcccagaatatctacgtgtggctg aactggtatcagcagaagccaggcaaagcacccaagctgctgatctacaaagccagcaatctgcacaccggagtcccaagcagattctccggttctgg cagtggaacagactttaccctgacaatcagcagcctccagcctgaggatttcgcaacttactattgccagcagggccagacatacccatatactttggc ggagggaccaaagtggagatcaagcgg |
| 43 | The amino acid sequence of scFv | QVQLVQSGAEVKKPGASVKVSCKASGYTFT**SYYIH**WVRQAPGQGLEWIG**CIYPGNVNTNYNEKFKD**RATLTVDTSISTA YMELSRLRSDDTAVYFCTR**SHYGLDWNFDV**WGQGTTVTVSSGGGGSGGGGSGGGGSDIQMTQSPSSLSASVGDRVT ITC**HASQNIYVWLN**WYQQKPGKAPKLLIY**KASNLHT**GVPSRFSGSGSGTDFTLTISSLQPEDFATYYC**QQGQTYPYT**FG GGTKVEIKR |
| Full-length trispecific antibody | | |
| 44 | Amino acid sequence | DIQLTQSPASLAVSLGQRATISCKASQSVDYDGDSYLNWYQQIPGQPPKLLIYDASNLVSGIPPRFSGSGSGTDFTLNIHPV EKVDAATYHCQQSTEDPWTFGGGTKLEIKGGGGSGGGGSGGGGSQVQLQQSGAELVRPGSSVKISCKASGYAFSSYW MNWVKQRPGQGLEWIGQIWPGDGDTNYNGKFKGKATLTADESSSTAYMQLSSLASEDSAVYFCARRETTTVGRYYYA MDYWGQGTTVTVSSGGGGSDIKLQQSGAELARPGASVKMSCKTSGYTFTRYTMHWVKQRPGQGLEWIGYINPSRGY TNYNQKFKDKATLTTDKSSSTAYMQLSSLTSEDSAVYYCARYYDDHYCLDYWGQGTTLTVSSVEGGSGGGSGGSGGSGG VDDIQLTQSPAIMSASPGEKVTMTCRASSSVSYMNWYQQKSGTSPKRWIYDTSKVASGVPYRFSGSGSGTSYSLTISSM EAEDAATYYCQQWSSNPLTFGAGTKLELKRNTGRGGEEKKKEKEKEEQEERETKTPESPSHTQPLGVQVQLVQSGAEVK KPGASVKVSCKASGYTFTSYYIHWVRQAPGQGLEWIGCIYPGNVNTNYNEKFKDRATLTVDTSISTAYMELSRLRSDDTA VYFCTRSHYGLDWNFDVWGQGTTVTVSSGGGGSGGGGSGGGGSDIQMTQSPSSLSASVGDRVTITCHASQNIYVWL NWYQQKPGKAPKLLIYKASNLHTGVPSRFSGSGSGTDFTLTISSLQPEDFATYYCQQGQTYPYTFGGGTKVEIKR* |

EP 4 556 486 A1

44

(continued)

| Full-length trispecific antibody | | |
|---|---|---|
| 45 | Coding sequence | atggccacccggctgaccgtgctggccctgctggccggcctgctggcctcctccagggccgacatccagctgacacagtctccagctagtctggcagtga gcctgggacagagagctactatttcctgcaaggcaagccagtccgtggactacgatggagactcctatctgaactggtatcagcagatccccggccagc cccctaaactgctgatttatgatgcttcaaatctggtgagcggcatcccaccccgcttctctggaagtgggtcaggcaccgattttacactgaacattcac cccgtggagaaggtggacgccgctacctaccattgccagcagtccactgaggacccctggaccttcggcggcggcaccaagctggaaatcaaaggtg gcggagggtccggtggcggagggtctggtggcggagggagccaggtgcagctccagcagtctggagcagagctggtgcggcccggcagcagcgtca agatttcctgtaaagcatctgggtatgccttttctagttactggatgaattgggtgaagcagagacccggccagggactggagtggattggacagatttg gcctggggatggtgacaccaactacaatggcaagttcaaaggcaaggctaccctgacagcagacgaatcaagctccacagcttacatgcagctgtcta gtctggcatcagaggatagcgccgtgtattttgcgctcggagggaaaccacaactgtcggccgctactattacgctatggactactggggccagggaa ccacagtgacagtctcaagcggtggcggagggtccgacatcaagctccagcagtctggtgccgagctggctcgaccaggagccagtgtgaagatgtca |
| Note: The CDR sequence is shown in **bold with an underline** | | |

Table 8. Sequences of the trispecific antibody MLMOF

| SEQ ID NO: | Description | Sequence |
|---|---|---|
| **First domain, CD19 binding domain** | | |
| 46 | The amino acid sequence of heavy chain CDR1 | SYVMH |
| 47 | The amino acid sequence of heavy chain CDR2 | YINPYNDGTKYNEKFQG |
| 48 | The amino acid sequence of heavy chain CDR3 | GTYYYGTRVFDY |
| 49 | The amino acid sequence of light chain CDR1 | RSSKSLQNVNGNTYLY |
| 50 | The amino acid sequence of light chain CDR2 | RMSNLNS |
| 51 | The amino acid sequence of light chain CDR3 | MQHLEYPIT |
| 52 | The amino acid sequence of the heavy chain variable region | EVQLVESGGGLVKPGGSLKLSCAASGYTFT**SYVMH**WVRQAPGKGLEWIG**YINPYNDGTKYNEKFQG**RVTISSDKSISTA YMELSSLRSEDTAMYYCAR**GTYYYGTRVFDY**WGQGTLVTVSS |
| 53 | Optimized coding sequence for the heavy chain variable region | gaggtgcagctggtggagagcggcggcggactggtgaagcctggcggaagcctgaagctgagctgtgccgcctccggctacacctttacctcctacgt gatgcactgggtgaggcaggcccccggcaagggactggagtggatcggctacatcaatccctacaatgatggcacaaagtacaatgagaagtttcag ggcagggtgaccatcagctccgataagagcatctccaccgcctacatggagctgtccagcctgagatccgaggataccgccatgtactactgtgccagg ggcacctactactacggcacaagggtgttcgactactggggccagggcacactggtgacagtgtcctcc |
| 54 | The amino acid sequence of the light chain variable region | DIVMTQSPATLSLSPGERATLSC**RSSKSLQNVNGNTYLY**WFQQKPGQSPQLLIY**RMSNLNS**GVPDRFSGSGSGTEFTLTI SSLEPEDFAVYYC**MQHLEYPIT**FGAGTKLEIK |
| 55 | Optimized coding sequence for the light chain variable region | gacatcgtgatgacacagagccctgccaccctgagcctgagccccggagagagggccaccctgtcctgtaggtccagcaagagcctgcagaacgtca atggcaatacatacctgtactggttccagcagaagcctggccagtccccccagctgctgatctacaggatgagcaatctgaacagcggcgtgcctgata |

| SEQ ID NO: | Description | Sequence |
|---|---|---|
| **First domain, CD19 binding domain** | | |
| | | ggttcagcggctccggcagcggcaccgagttcaccctgaccatcagcagcctggagcccgaggatttcgccgtgtactactgcatgcagcacctggagt<br><br>accccatcacattcggcgccggcaccaagctggagatcaag |
| 56 | The amino acid sequence of scFv | DIVMTQSPATLSLSPGERATLSCRSSKSLQNVNGNTYLYWFQQKPGQSPQLLIYRMSNLNSGVPDRFSGSGSGTEFTLTIS<br><br>SLEPEDFAVYYCMQHLEYPITFGAGTKLEIKGGGGSGGGGSGGGGSEVQLVESGGGLVKPGGSLKLSCAASGYTFTSYV<br><br>MHWVRQAPGKGLEWIGYINPYNDGTKYNEKFQGRVTISSDKSISTAYMELSSLRSEDTAMYYCARGTYYYGTRVFDYW<br><br>GQGTLVTVSS |
| **Second domain, CD3 binding domain** | | |
| 57 | The amino acid sequence of heavy chain CDR1 | KYAMN |
| 58 | The amino acid sequence of heavy chain CDR2 | RIRSKYNNYATYYADSVKD |
| 59 | The amino acid sequence of heavy chain CDR3 | HGNFGNSYISYWAY |
| 60 | The amino acid sequence of light chain CDR1 | GSSTGAVTSGNYPN |
| 61 | The amino acid sequence of light chain CDR2 | GTKFLAP |
| 62 | The amino acid sequence of light chain CDR3 | VLWYSNRWV |
| 63 | The amino acid sequence of the heavy chain variable region | EVQLVESGGGLVQPGGSLKLSCAASGFTFN**KYAMN**WVRQAPGKGLEWVA**RIRSKYNNYATYYADSVKD**RFTISRDDS<br><br>KNTAYLQMNNLKTEDTAVYYCVR**HGNFGNSYISYWAY**WGQGTLVTVSS |

| Second domain, CD3 binding domain | | |
|---|---|---|
| 64 | Optimized coding sequence for the heavy chain variable region | gaggtgcaactggttgagagcggcggcggtctggtgcagcccggaggatctctgaagctgagctgtgccgccagcggctttacattcaacaagtacgc catgaattgggtgaggcaggcccccggaaagggcctggagtgggtggctaggatcaggtccaagtacaacaattacgccacatactacgccgattccg tgaaggacaggttcacaatcagcagagacgactccaagaataccgcctacctgcaaatgaataacctgaagaccgaggacacagccgtgtactactg cgtgaggcacggcaattttggcaacagctacatcagctactgggcctactggggccagggaacactggtgaccgtgagcagc |
| 65 | The amino acid sequence of the light chain variable region | QTVVTQEPSLTVSPGGTVTLTC**GSSTGAVTSGNYPN**WVQQKPGQAPRGLIG**GTKFLAP**GTPARFSGSLLGGKAALTLSG VQPEDEAEYYC**VLWYSNRWV**FGGGTKLTVL |
| 66 | Optimized coding sequence for the light chain variable region | cagacagtggtgacacaggagccctccctgaccgtgagccccggaggaacagtgaccctgacctgcggcagctccacaggcgccgtgacaagcggca actaccccaattgggtgcagcagaagcccggccaggccccaaggggactgatcggaggcaccaagttcctggccctggcacccctgccagattctcc ggcagcctgctgggcggcaaggccgctctgacactgtccggcgtgcagcccgaggatgaggccgaatactactgcgtcctgtggtacagcaacagatg ggtgttcggcggcggcaccaagctcacagtgctg |
| 67 | The amino acid sequence of scFv | EVQLVESGGGLVQPGGSLKLSCAASGFTFNKYAMNWVRQAPGKGLEWVARIRSKYNNYATYYADSVKDRFTISRDDSK NTAYLQMNNLKTEDTAVYYCVRHGNFGNSYISYWAYWGQGTLVTVSSGGGGSGGGGSGGGGSQTVVTQEPSLTVSP GGTVTLTCGSSTGAVTSGNYPNWVQQKPGQAPRGLIGGTKFLAPGTPARFSGSLLGGKAALTLSGVQPEDEAEYYCVL WYSNRWVFGGGTKLTVL |

EP 4 556 486 A1

| Full-length trispecific antibody | | |
|---|---|---|
| 68 | Amino acid sequence | DIVMTQSPATLSLSPGERATLSCRSSKSLQNVNGNTYLYWFQQKPGQSPQLLIYRMSNLNSGVPDRFSGSGSGTEFTLTIS SLEPEDFAVYYCMQHLEYPITFGAGTKLEIKGGGGSGGGGSGGGGSEVQLVESGGGLVKPGGSLKLSCAASGYTFTSYV MHWVRQAPGKGLEWIGYINPYNDGTKYNEKFQGRVTISSDKSISTAYMELSSLRSEDTAMYYCARGTYYYGTRVFDYW GQGTLVTVSSGGGGSEVQLVESGGGLVQPGGSLKLSCAASGFTFNKYAMNWVRQAPGKGLEWVARIRSKYNNYATYY ADSVKDRFTISRDDSKNTAYLQMNNLKTEDTAVYYCVRHGNFGNSYISYWAYWGQGTLVTVSSGGGGSGGGGSGGG GSQTVVTQEPSLTVSPGGTVTLTCGSSTGAVTSGNYPNWVQQKPGQAPRGLIGGTKFLAPGTPARFSGSLLGGKAALTL SGVQPEDEAEYYCVLWYSNRWVFGGGTKLTVLRNTGRGGEEKKKEKEKEEQEERETKTPESPSHTQPLGVQVQLVQSG AEVKKPGASVKVSCKASGYTFTSYYIHWVRQAPGQGLEWIGCIYPGNVNTNYNEKFKDRATLTVDTSISTAYMELSRLRS DDTAVYFCTRSHYGLDWNFDVWGQGTTVTVSSGGGGSGGGGSGGGGSDIQMTQSPSSLSASVGDRVTITCHASQNIY VWLNWYQQKPGKAPKLLIYKASNLHTGVPSRFSGSGSGTDFTLTISSLQPEDFATYYCQQGQTYPYTFGGGTKVEIKR |

EP 4 556 486 A1

(continued)

| Full-length trispecific antibody | | |
|---|---|---|
| 69 | Coding sequence | atggacatgagggtccccgctcagctcctggggctcctgctactctggctccgaggtgccagatgtgacatcgtgatgacacagagccctgccaccctgagcctgagcccccggagagagggccaccctgtcctgtaggtccagcaagagcctgcagaacgtcaatggcaatacatacctgtactggttccagcagaagcctggccagtcccccagctgctgatctacaggatgagcaatctgaacagcggcgtgcctgataggttcagcggctccggcagcggcaccgagttcaccctgaccatcagcagcctggagcccgaggatttcgccgtgtactactgcatgcagcacctggagtaccccatcacattcggcgccggcaccaagctggagatcaagggcggcggcggcagcggaggaggaggaagcggaggaggcggctccgaggtgcagctggtggagagcggcggcggactggtgaagcctggcggaagcctgaagctgagctgtgccgcctccggctacacctttacctcctacgtgatgcactgggtgaggcaggcccccggcaagggactggagtggatcggctacatcaatccctacaatgatggcacaaagtacaatgagaagtttcagggcagggtgaccatcagctccgataagagcatctccaccgcctacatggagctgtccagcctgagatccgaggataccgccatgtactactgtgccaggggcacctactactacggcacaagggtgttcgactactggggccagggcacactggtgacagtgtcctccggtggcggagggtccgaggtgcaactggttgagagcggcggcggcggtctggtgcagcccggaggatctctgaagctgagctgtgccgccagcggctttacattcaacaagtacgccatgaattgggtgaggcaggcccccggaaagggcctggagtgggtggctaggatcaggtccaagtacaacaattacgccacatactacgccgattccgtgaaggacaggttcacaatcagcagagacgactccaagaataccgcctacctgcaaat |

| Full-length trispecific antibody | | |
|---|---|---|
| | | gaataacctgaagaccgaggacacagccgtgtactactgcgtgaggcacggcaattttggcaacagctacatcagctactgggcctactggggccagg<br><br>gaacactggtgaccgtgagcagcggcggcggcggcagcggaggaggaggcagcggaggcggaggaagccagacagtggtgacacaggagccctc<br><br>cctgaccgtgagccccggaggaacagtgaccctgacctgcggcagctccacaggcgccgtgacaagcggcaactaccccaattgggtgcagcagaag<br><br>cccggccaggccccaagggggactgatcggaggcaccaagttcctggcccctggcacccctgccagattctccggcagcctgctgggcggcaaggccgc<br><br>tctgacactgtccggcgtgcagcccgaggatgaggccgaatactactgcgtcctgtggtacagcaacagatgggtgttcggcggcggcaccaagctca<br><br>cagtgctgagaaataccggcagaggcggagaggagaagaagaaggagaaggagaaggaggagcaggaggagagagagaccaagacacccgagt<br><br>ctcctagccacacacagcctctgggagtgcaggtgcagctggtccagtcaggagcagaagtcaaaaaacccggcgcatcagtcaaagtctcttgtaaa<br><br>gcctccggatacacatttacatcctactatatccactgggtcagacaggcacctggccagggactggagtggatcggctgcatttacccaggaaacgtg<br><br>aacaccaactacaacgaaaagttcaaggaccgcgctactctgaccgtcgatacatcaattagcactgcatacatggagctgtctcggctgaggagtga<br><br>cgatacagctgtgtacttctgtactcgatctcattatggcctggactggaactttgatgtgtgggggcagggcaccacagtgaccgtcagctccggagga<br><br>ggaggctctggaggaggagggagtggcggaggaggatcagacatccagatgacacagagcccttcaagcctgagcgcctccgtcggtgacagagtg<br><br>accattacctgccacgcctcccagaatatctacgtgtggctgaactggtatcagcagaagccaggcaaagcacccaagctgctgatctacaaagccag<br><br>caatctgcacaccggagtcccaagcagattctccggttctggcagtggaacagactttaccctgacaatcagcagcctccagcctgaggatttcgcaact<br><br>tactattgccagcagggccagacatacccatatacttttggcggagggaccaaagtggagatcaagcggtag |
| Note: The CDR sequence is shown in **bold with an underline** | | |

EP 4 556 486 A1

**Claims**

1. A trispecific antibody specifically binding to CD19, CD3, and CD28, wherein the trispecific antibody is a single chain fusion protein and comprises:

   (1) a first binding domain specifically binding to CD19;
   (2) a first linker sequence;
   (3) a second binding domain specifically binding to CD3;
   (4) a second linker sequence, which is an amino acid sequence selected from SEQ ID NO: 4, SEQ ID NO: 5, SEQ ID NO: 6, SEQ ID NO: 8 or SEQ ID NO: 9; and
   (5) a third binding domain specifically binding to CD28.

2. The trispecific antibody of claim 1, wherein the first linker sequence comprises $(G_4S)n$ or $(G_2S)n$, or the first linker sequence is $(G_4S)n$ or $(G_2S)n$, wherein n is an integer of 1-6, preferably 1-3.

3. The trispecific antibody of claim 2, wherein the first linker sequence is selected from an amino acid sequence of SEQ ID NO: 1, SEQ ID NO: 2, or SEQ ID NO: 3.

4. The trispecific antibody of any one of claims 1-3, wherein the first binding domain is a single chain antibody specifically binding to CD19, comprising a heavy chain variable region (VH) and a light chain variable region (VL), wherein

   (a) the VH comprises heavy chain CDR1 as set forth in SEQ ID NO: 11, heavy chain CDR2 as set forth in SEQ ID NO: 12, and heavy chain CDR3 as set forth in SEQ ID NO: 13, and the VL comprises light chain CDR1 as set forth in SEQ ID NO: 14, light chain CDR2 as set forth in SEQ ID NO: 15, and light chain CDR3 as set forth in SEQ ID NO: 16; or
   (b) the VH comprises heavy chain CDR1 as set forth in SEQ ID NO: 46, heavy chain CDR2 as set forth in SEQ ID NO: 47, and heavy chain CDR3 as set forth in SEQ ID NO: 48, and the VL comprises light chain CDR1 as set forth in SEQ ID NO: 49, light chain CDR2 as set forth in SEQ ID NO: 50, and light chain CDR3 as set forth in SEQ ID NO: 51.

5. The trispecific antibody of claim 4, wherein:

   (a) the VH comprises or consists of an amino acid sequence as set forth in SEQ ID NO: 17, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 17, in which all amino acid differences are located in non-CDR regions; and
   the VL comprises or consists of an amino acid sequence as set forth in SEQ ID NO: 19, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 19, in which all amino acid differences are located in non-CDR regions; or
   (b) the VH comprises or consists of an amino acid sequence as set forth in SEQ ID NO: 52, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 52, in which all amino acid differences are located in non-CDR regions; and

   the VL comprises or consists of an amino acid sequence as set forth in SEQ ID NO: 54, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 54, in which all amino acid differences are located in non-CDR regions.

6. The trispecific antibody of claim 5, wherein the first binding domain comprises or consists of an amino acid sequence as set forth in SEQ ID NO: 21 or SEQ ID NO: 56, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 21 or SEQ ID NO: 56, in which all amino acid differences are located in non-CDR regions.

7.  The trispecific antibody of any one of claims 1-6, wherein the second binding domain is a single chain antibody specifically binding to CD3, comprising a heavy chain variable region (VH) and a light chain variable region (VL), wherein:

    (a) the VH comprises heavy chain CDR1 as set forth in SEQ ID NO: 22, heavy chain CDR2 as set forth in SEQ ID NO: 23, and heavy chain CDR3 as set forth in SEQ ID NO: 24, and the VL comprises light chain CDR1 as set forth in SEQ ID NO: 25, light chain CDR2 as set forth in SEQ ID NO: 26, and light chain CDR3 as set forth in SEQ ID NO: 27; or
    (b) the VH comprises heavy chain CDR1 as set forth in SEQ ID NO: 57, heavy chain CDR2 as set forth in SEQ ID NO: 58, and heavy chain CDR3 as set forth in SEQ ID NO: 59, and the VL comprises light chain CDR1 as set forth in SEQ ID NO: 60, light chain CDR2 as set forth in SEQ ID NO: 61, and light chain CDR3 as set forth in SEQ ID NO: 62.

8.  The trispecific antibody of claim 7, wherein

    (a) the VH comprises or consists of an amino acid sequence as set forth in SEQ ID NO: 28, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 28, in which all amino acid differences are located in non-CDR regions; and
    the VL comprises or consists of an amino acid sequence as set forth in SEQ ID NO: 30, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 30, in which all amino acid differences are located in non-CDR regions; or
    (b) the VH comprises or consists of an amino acid sequence as set forth in SEQ ID NO: 63, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 63, in which all amino acid differences are located in non-CDR regions; and

    the VL comprises or consists of an amino acid sequence as set forth in SEQ ID NO: 65, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 65, in which all amino acid differences are located in non-CDR regions.

9.  The trispecific antibody of claim 8, wherein the scFv specifically binding to CD3 comprises or consists of an amino acid sequence as set forth in SEQ ID NO: 32 or SEQ ID NO: 67, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 32 or SEQ ID NO: 67, in which all amino acid differences are located in non-CDR regions.

10. The trispecific antibody of any one of claims 1-9, wherein the third binding domain is a single chain antibody specifically binding to CD28, comprising a heavy chain variable region (VH) and a light chain variable region (VL), wherein the VH comprises heavy chain CDR1 as set forth in SEQ ID NO: 33, heavy chain CDR2 as set forth in SEQ ID NO: 34, and heavy chain CDR3 as set forth in SEQ ID NO: 35, and the VL comprises light chain CDR1 as set forth in SEQ ID NO: 36, light chain CDR2 as set forth in SEQ ID NO: 37, and light chain CDR3 as set forth in SEQ ID NO: 38.

11. The trispecific antibody of claim 10, wherein

    the VH comprises or consists of an amino acid sequence as set forth in SEQ ID NO: 39, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 39, in which all amino acid differences are located in non-CDR regions; and

the VL comprises or consists of an amino acid sequence as set forth in SEQ ID NO: 41, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 41, in which all amino acid differences are located in non-CDR regions.

12. The trispecific antibody of claim 11, wherein the third binding domain comprises or consists of an amino acid sequence as set forth in SEQ ID NO: 43, or an amino acid sequence having at least 80% homology (such as at least 85% homology, preferably at least 90% homology, more preferably at least 95% homology, even more preferably at least 96%, at least 97%, at least 98% or at least 99% homology) with the amino acid sequence as set forth in SEQ ID NO: 43, in which all amino acid differences are located in non-CDR regions.

13. The trispecific antibody of any one of claims 1-12, comprising or consisting of an amino acid sequence as set forth in SEQ ID NO: 44 or SEQ ID NO: 68; or comprising or consisting of an amino acid sequence having at least 80%, at least 90%, at least 95%, at least 96%, at least 97%, at least 98%, or at least 99% homology with the amino acid sequence as set forth in SEQ ID NO: 44 or SEQ ID NO: 68.

14. An isolated nucleic acid molecule encoding the trispecific antibody of any one of claims 1-13.

15. The isolated nucleic acid molecule of claim 14, comprising or consisting of a nucleotide sequence as set forth in SEQ ID NO: 45 or SEQ ID NO: 69.

16. An expression vector comprising the isolated nucleic acid molecule of claim 14 or 15.

17. A host cell comprising the isolated nucleic acid molecule of claim 14 or 15 or the expression vector of claim 16.

18. A method for preparing a trispecific antibody, comprising:

(a) providing the isolated nucleic acid molecule of claim 14 or 15 or the expression vector of claim 16,
(b) introducing the isolated nucleic acid molecule or the expression vector in (a) into a host cell,
(c) culturing the host cell obtained in (b) to express the trispecific antibody, and
(d) recovering the trispecific antibody from the culture of the cell.

19. A method for preparing a trispecific antibody, comprising:

(a) providing the host cell of claim 17,
(b) culturing the host cell to express the trispecific antibody, and
(c) recovering the trispecific antibody from the culture of the cell.

20. Use of the trispecific antibody of any one of claims 1-13 in the preparation of a medicament.

21. The use of claim 20, wherein the medicament is used for treating a B cell-associated tumor, e.g., B cell lymphoma, B cell leukemia, a refractory or relapsed B cell tumor, aggressive or indolent non-Hodgkin lymphoma, B cell acute lymphoblastic leukemia.

22. A pharmaceutical composition comprising the trispecific antibody of any one of claims 1-13 and a pharmaceutically acceptable carrier.

23. A pharmaceutical combination comprising the trispecific antibody of any one of claims 1-13, and an additional therapeutic agent, e.g., a therapeutic agent for the treatment of a B cell-associated tumor.

24. A linker sequence having an amino acid sequence as set forth in SEQ ID NO: 4, SEQ ID NO: 5, SEQ ID NO: 6, SEQ ID NO: 8, or SEQ ID NO: 9.

Figure 1

—— LMOAME: ~84kDa

Lane descriptions
1: Molecular weight marker
2: LMOAME-purification1 (RS2020001, 1ug)
3: LMOAME-purification2 (DS2020001, 1ug)
4: LMOAME-purification3 (DS2021001, 1ug)
5: LMOAME- purification4 (RS2021002, 1ug)
6: LMOAME-purification5 (DS2021002, 1ug)
7: LMOAME-purification1 (RS2020001, 10ng)

Figure 2A

Figure 2B

Figure 3

Figure 4A

Figure 4B

1: Molecular weight marker
2: LMOAME (non-reducing)
3: BLMOAM (non-reducing)
4: Molecular weight marker
5: LMOAME (reducing)
6: BLMOAM (reducing)

Figure 5

Figure 6

rhCD19

Figure 7A

rhCD28

Figure 7B

Figure 8A

Figure 8B

Figure 9

Figure 10

Figure 11

## Body weight

G1: vehicle control group; BIW*3weeks, i.v.
G2: LMOAME 20ug/kg; BIW*3weeks, i.v.
G3: LMOAME 80ug/kg; BIW*3weeks, i.v.
G4: LMOAME 150ug/kg; BIW*week, i.v.

Days after grouping

Figure 12A

## Tomor volume

G1: vehicle control group; BIW*3weeks, i.v.
G2: LMOAME 20ug/kg; BIW*3weeks, i.v.
G3: LMOAME 80ug/kg; BIW*3weeks, i.v.
G4: LMOAME 150ug/kg; BIW*week, i.v.

Days after grouping

$*P < 0.05$, $**P < 0.01$, significantly different from G1 (Dunnett's multi-comparison test)

Figure 12B

Figure 13

Figure 14

G1: vehicle control group, days 1, 4, 8, 11, 15, 18, 22 and 25, i.v.
G2: LMOAME 50ug/kg, days 1, 4, 8, 11, 15, 18, 22 and 25, i.v.
G3: LMOAME 120ug/kg, days 1, 4, 8, 11, 15, 18, 22 and 25, i.v.
G4: LMOAME 300ug/kg, days 1, 4, 8, 11, 15, 18, 22 and 25, i.v.

Days after treatment (d)

Figure 15A

Figure 15B

Figure 16

<div align="center">

**INTERNATIONAL SEARCH REPORT**

</div>

| International application No. |
|---|
| **PCT/CN2023/106817** |

**A. CLASSIFICATION OF SUBJECT MATTER**

C07K 14/46(2006.01)i; C12N 15/13(2006.01)i; C12N 15/85(2006.01)i; A61K 39/395(2006.01)i; C07K 14/435(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

IPC: C07K C12N A61K A61P

Documentation searched other than minimum documentation to the extent that such documents are included in the fields searched

Electronic data base consulted during the international search (name of data base and, where practicable, search terms used)

CNABS, WPABS, WPABSC, DWPI, CNTXT, WOTXT, USTXT, EPTXT, EPTXTC, CNKI, PUBMED, ISI web of science, Genbank, EMBL, STN, 万方数据库 WANFANG DATABASE, 百度学术 BAIDU SCHOLAR, 中国专利生物序列检索系统 China Patents Biological Sequence Search System. 连接, 接头, 半胱氨酸, 糖基化, 突变, 替换, 替代, 取代, CD19, CD3, CD28, IgD, C161?, linker, glycosylation, Cys, mutation, mutate, substitution, replacement, replace, subtitute, subtitutate; 对 SEQ ID NO: 1-17, 19, 21-28, 30, 32-39, 41, 43-44, 46-52, 54, 56-63, 65, 67-68 进行序列检索, sequence search on SEQ ID NO: 1-17, 19, 21-28, 30, 32-39, 41, 43-44, 46-52, 54, 56-63, 65, 67-68.

**C. DOCUMENTS CONSIDERED TO BE RELEVANT**

| Category* | Citation of document, with indication, where appropriate, of the relevant passages | Relevant to claim No. |
|---|---|---|
| X | WO 2021231655 A1 (LYELL IMMUNOPHARMA INC.) 18 November 2021 (2021-11-18) see table 1, SEQ ID NO: 786 | 24 |
| Y | CN 106589129 A (SHANGHAI NOVOPROTEIN BIOTECHNOLOGY CO., LTD.) 26 April 2017 (2017-04-26) see claims 1-16, and embodiments 1-5 | 1-23 |
| Y | US 2007237779 A1 (LEDBETTER J. A.; HAYDEN-LEDBETTER M. S.; THOMPSON P. A.) 11 October 2007 (2007-10-11) see description, paragraphs 112 and 380-388 | 1-23 |
| Y | CN 110520444 A (BIOMUNEX PHARMACEUTICALS) 29 November 2019 (2019-11-29) see description, paragraph 228 | 1-23 |
| Y | WO 2022072538 A1 (BIOTHERYX INC.) 07 April 2022 (2022-04-07) see description, paragraphs 94-102, and SEQ ID NO:11-18 and 39-46 | 4-9, 13 |

☑ Further documents are listed in the continuation of Box C.     ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **29 August 2023** | **29 September 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

| INTERNATIONAL SEARCH REPORT | International application No. |
|---|---|
| | **PCT/CN2023/106817** |

**C.    DOCUMENTS CONSIDERED TO BE RELEVANT**

| Category* | Citation of document, with indication, where appropriate, of the relevant passages | Relevant to claim No. |
|---|---|---|
| Y | CN 114173810 A (NOVARTIS AG) 11 March 2022 (2022-03-11)<br>    see table 12 | 4-9, 13 |
| PX | CN 115724986 A (HUIHE BIOTECHNOLOGY (SHANGHAI) CO., LTD.) 03 March 2023<br>(2023-03-03)<br>    see claims 1-24, and embodiment | 1-24 |
| A | CN 113286821 A (F. HOFFMANN-LA ROCHE AG) 20 August 2021 (2021-08-20)<br>    see claims 1-63, and SEQ ID NO: 20-27 | 1-24 |

Form PCT/ISA/210 (second sheet) (July 2022)

<table>
<tr><td>**INTERNATIONAL SEARCH REPORT**</td><td>International application No.<br>**PCT/CN2023/106817**</td></tr>
</table>

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

       ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

INTERNATIONAL SEARCH REPORT
Information on patent family members

International application No.

**PCT/CN2023/106817**

| Patent document cited in search report | | | Publication date (day/month/year) | Patent family member(s) | | | Publication date (day/month/year) |
|---|---|---|---|---|---|---|---|
| WO | 2021231655 | A1 | 18 November 2021 | EP | 4149968 | A1 | 22 March 2023 |
| | | | | KR | 20230022411 | A | 15 February 2023 |
| | | | | US | 2021380658 | A1 | 09 December 2021 |
| | | | | JP | 2023526234 | A | 21 June 2023 |
| | | | | IL | 298114 | A | 01 January 2023 |
| | | | | AU | 2021270299 | A1 | 15 December 2022 |
| | | | | BR | 112022022953 | A2 | 21 March 2023 |
| | | | | CA | 3157427 | A1 | 18 November 2021 |
| CN | 106589129 | A | 26 April 2017 | None | | | |
| US | 2007237779 | A1 | 11 October 2007 | US | 7754209 | B2 | 13 July 2010 |
| CN | 110520444 | A | 29 November 2019 | SG | 11201906313 | SA | 27 August 2019 |
| | | | | JP | 2020503873 | A | 06 February 2020 |
| | | | | WO | 2018127608 | A1 | 12 July 2018 |
| | | | | US | 2019330377 | A1 | 31 October 2019 |
| | | | | CA | 3052084 | A1 | 12 July 2018 |
| | | | | MX | 2019008241 | A | 09 December 2019 |
| | | | | AU | 2018206229 | A1 | 29 August 2019 |
| | | | | KR | 20190102271 | A | 03 September 2019 |
| | | | | BR | 112019014147 | A2 | 11 February 2020 |
| | | | | ZA | 201905160 | B | 26 May 2021 |
| | | | | RU | 2019125228 | A | 09 February 2021 |
| | | | | RU | 2019125228 | A3 | 22 April 2021 |
| | | | | EP | 3565847 | A1 | 13 November 2019 |
| | | | | IL | 267886 | A | 26 September 2019 |
| | | | | JP | 2023027215 | A | 01 March 2023 |
| WO | 2022072538 | A1 | 07 April 2022 | None | | | |
| CN | 114173810 | A | 11 March 2022 | AU | 2020279224 | A1 | 16 December 2021 |
| | | | | KR | 20220010743 | A | 26 January 2022 |
| | | | | EP | 3972634 | A2 | 30 March 2022 |
| | | | | JP | 2022537887 | A | 31 August 2022 |
| | | | | IL | 287918 | A | 01 January 2022 |
| | | | | US | 2022396631 | A1 | 15 December 2022 |
| | | | | WO | 2020236795 | A2 | 26 November 2020 |
| | | | | CA | 3140142 | A1 | 26 November 2020 |
| CN | 115724986 | A | 03 March 2023 | None | | | |
| CN | 113286821 | A | 20 August 2021 | CA | 3123493 | A1 | 25 June 2020 |
| | | | | EP | 3898682 | A1 | 27 October 2021 |
| | | | | BR | 112021012101 | A2 | 08 September 2021 |
| | | | | SG | 11202105093 | RA | 29 June 2021 |
| | | | | JP | 2022514343 | A | 10 February 2022 |
| | | | | CR | 20210326 | A | 10 September 2021 |
| | | | | PE | 20211696 | A1 | 01 September 2021 |
| | | | | KR | 20210108981 | A | 03 September 2021 |
| | | | | WO | 2020127618 | A1 | 25 June 2020 |
| | | | | MA | 54513 | A | 30 March 2022 |
| | | | | AU | 2019410073 | A1 | 10 June 2021 |
| | | | | MX | 2021007320 | A | 07 July 2021 |
| | | | | US | 2020199234 | A1 | 25 June 2020 |
| | | | | US | 11608376 | B2 | 21 March 2023 |

Form PCT/ISA/210 (patent family annex) (July 2022)

<table>
<tr><td colspan="2" align="center"><strong>INTERNATIONAL SEARCH REPORT</strong><br>Information on patent family members</td><td colspan="2">International application No.<br><strong>PCT/CN2023/106817</strong></td></tr>
<tr><td>Patent document<br>cited in search report</td><td>Publication date<br>(day/month/year)</td><td>Patent family member(s)</td><td>Publication date<br>(day/month/year)</td></tr>
<tr><td></td><td></td><td>TW   202039567   A</td><td>01 November 2020</td></tr>
<tr><td></td><td></td><td>CO   2021008696   A2</td><td>19 July 2021</td></tr>
<tr><td></td><td></td><td>JP   2023021958   A</td><td>14 February 2023</td></tr>
<tr><td></td><td></td><td>IL   283811   A</td><td>29 July 2021</td></tr>
<tr><td></td><td></td><td>CL   2021001603   A1</td><td>11 February 2022</td></tr>
</table>

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210822959

- CN 106589129 A

**Non-patent literature cited in the description**

- **SCHIETINGER A** ; **GREENBERG PD.** Tolerance and exhaustion: defining mechanisms of T cell dysfunction.. *Trends in Immunology.*, 2014, vol. 35, 51-60
- **JEONG S** ; **PARK SH.** Co-stimulatory receptors in cancers and their implications for cancer immunotherapy.. *Immune Netw.*, 2020, vol. 20 (1), e3
- **ESENSTEN JH** ; **HELOU YA** ; **CHOPRA G et al.** CD28 costimulation: from mechanism to therapy.. *Immunity*, 2017, vol. 44, 973-988
- **BOISE LH** ; **MINN AJ** ; **NOEL PJ** ; **JUNE CH** ; **ACCAVITTI MA** ; **LINDSTEN T** ; **THOMPSON CB.** CD28 costimulation can promote T cell survival by enhancing the expression of Bcl-xL.. *Immunity.*, 2010, vol. 3, 87-98
- **WATTS TH.** Staying alive: T cell costimulation, CD28, and Bcl-xL.. *J Immunol.*, 2010, vol. 185, 3785-3787
- **PETTITT D** ; **ARSHAD Z** ; **SMITH J et al.** CAR-T cells: a systematic review and mixed methods analysis of the clinical trial landscape.. *Molecular Therapy*, 2018, vol. 26, 342-353

- **MA S** ; **LI XC** ; **WANG XY et al.** Current Progress in CAR-T cell therapy for solid tumors. *Int J Biol Sci.*, 2019, vol. 15, 2548-2560
- **SAVOLDO B** ; **RAMOS CA** ; **LIU E et al.** CD28 costimulation improves expansion and persistence of chimeric antigen receptor-modified T cells in lymphoma patients.. *Immunology*, 2011, vol. 121, 1822-1826
- **GUEDAN S** ; **POSEY JR., AD** ; **SHAW C et al.** Enhancing CAR T cell persistence through ICOS and 4-1BB costimulation.. *Immunology*, 2018, vol. 3 (1), e96976
- **DICKOPF S** ; **GEORGES GJ** ; **BRINKMANN U**. Format and geometries matter: Structure-based design defines the functionality of bispecific antibodies.. *Computational and Structural Biotechnology Journal*, 2020, vol. 18, 1221-1227
- **ALTSCHUL, S. F. et al.** *J. Mol. Biol.*, 1990, vol. 215, 403-410